# EUROPEAN PATENT APPLICATION

(11) **EP 4 154 904 A1**
(43) Date of publication of application: **29.03.2023**
(21) Application number: 22198839.7
(22) Date of filing: 17.07.2019
(51) Int. Cl.: A61K 38/48, C07K 16/06, C12N 9/24, C12N 9/58, C12N 15/86, A61K 39/12, C12N 9/64

(54) **COMPOSITIONS AND METHODS FOR INCREASING OR ENHANCING TRANSDUCTION OF GENE THERAPY VECTORS AND FOR REMOVING OR REDUCING IMMUNOGLOBULINS**

(30) Priority: 17.07.2018 EP 18305971; 16.11.2018 US 201862768731 P
(62) Divisional of application: 19740384.3
(71) Applicant: INSTITUT NATIONAL DE LA SANTE ET DE LA RECHERCHE MEDICALE - INSERM, 75013 Paris (FR); Genethon, 91000 Evry (FR); Sorbonne Université, 75006 Paris (FR); Université Paris Cité, 75006 Paris (FR); Spark Therapeutics, Inc., Philadelphia, Pennsylvania 19104 (US)
(72) Inventor: LACROIX-DESMAZES, Sébastien, 75006 PARIS (FR); MINGOZZI, Federico, PHILADELPHIA, 19118 (US); DIMITROV, Jordan, 75006 PARIS (FR); LEBORGNE, Christian, 91000 EVRY (FR); ARMOUR, Sean, HOLLAND, 18966 (US)
(74) Representative: Lavoix

(57) **Abstract**

Disclosed herein are methods for treating patients that may develop or already have preexisting gene therapy neutralizing antibodies by administering a protease that cleaves peptide bonds present in immunoglobulins or by administering a glycosidase that cleaves carbohydrate residues present on immunoglobulins, or other similar enzymatic cleavage of immunoglobulins *in vivo.* Also disclosed are methods for utilizing IdeS and other immunoglobulin G-degrading enzyme polypeptides for gene therapy treatment of a disease in a patient in need thereof.

## Description

### Related Applications

This application claims priority to European Patent Application No. EP18305971.6, filed July 17, 2018, and U.S. Provisional Patent Application No. 62/768,731, filed November 16, 2018. The entire contents of the foregoing applications are incorporated herein by reference, including all text, tables, sequence listings and drawings.

### Introduction

Adeno-associated virus (AAV) and other viral vectors as well as lipid-, polymer-, and protein-based nanoparticle gene therapy approaches can be targeted by the adaptive immune system, leading to blunted efficacy and the possibility of a patient becoming completely refractory to therapeutic intervention. The adaptive-immune system relies on development of antigen-specific immunoglobulin (*e.g*., IgG) antibodies which lead to the inhibition or clearance of the target molecule. Since humans are naturally exposed to wild-type AAV, AAV gene therapy can be hampered by the presence of pre-existing anti-AAV antibodies. Additionally, development of anti-AAV antibodies following AAV gene transfer can prevent redosing with the same or cross-reactive vectors.

Exposure to wild-type AAV after birth induces antibodies directed against the virus capsid within the first two years of life (Calcedo et al. (2011) Clin Vaccine Immunol. 18, 1586-8; Erles et al. (1999) J Med Virol. 59, 406-11; Li et al. (2012) Gene Ther. 19, 288-94). Depending on the AAV serotype and the age of the individual, the proportion of subjects positive for anti-AAV antibodies can reach 60%. Some anti-AAV antibodies target viral epitopes critical for cellular entry and neutralize virus infectivity, even when present at relatively low titers. Importantly, anti-AAV antibodies show a high degree of cross-reactivity with the different AAV serotypes (Boutin et al. (2010) Hum Gene Ther. 21, 704-12). As demonstrated in several preclinical and clinical studies, such neutralizing antibodies drastically reduce the transduction efficiency, particularly when the vector is delivered directly into the bloodstream (Manno et al. (2006) Nat Med. 12, 342-7; Masat et al. (2013) Discov Med. 15, 379-89; Arruda et al. (2010) Blood. 115,4678-88; Haurigot et al. (2010) Mol Ther. 18, 1318-29; Jiang et al. (2006) Blood. 108, 3321-8; Scallan et al. (2006) Blood. 107, 1810-7).

The high prevalence of neutralizing anti-AAV antibodies excludes certain subjects from enrollment in gene transfer trials with AAV vectors and will exclude certain patients from receiving approved AAV gene therapies, leaving certain patients without access to potentially life-saving therapies. Furthermore, neutralizing anti-AAV antibodies are induced following AAV gene transfer, which prevents the transduction efficiency in case of redosing of the same individual. For example, Hemophilia B is a rare, X-linked hemorrhagic disorder caused by deficiency or dysfunction in coagulation factor IX (FIX), and, when severe (circulating FIX levels below 1% of normal), results in frequent bleeding episodes, development of arthropathy and risk of early death (Mannucci et al. (2001) N Engl J Med. 344, 1773-9). The current treatment of exogenous FIX administrations to prevent or treat bleeds is not ideal. The short half-life of the molecule dictates frequent intravenous injections, associated with high costs and the risk of developing inhibitory antibodies. The monogenic nature of hemophilia B makes gene therapy an attractive choice to restore continuous endogenous FIX expression, and circumvent the limitations of current therapy. In this regard, AAV-mediated gene transfer for the long-term correction of hemophilia B in severe patients has shown great promise (Nathwani et al. (2014) N Engl J Med. 371, 1994-2004; Nathwani et al. (2011) N Engl J Med. 365, 2357-65; George et al. (2017) N Engl J Med. 377, 2215-2227), however, the presence of pre-existing anti-AAV antibodies presents an obstacle to treating certain hemophilia B patients with AAV-mediated gene therapy. Similarly, AAV-mediated gene transfer has shown great promise for the treatment of hemophilia A, spinal muscular atrophy (Mendell et al., 2017, N. Engl. J. Med., 377:1713-1722), and many other diseases, but the presence of pre-existing of anti-AAV antibodies likewise presents an obstacle to treating certain patients with these diseases and disorders.

Proteolysis of immunoglobulins is a common mechanism used by pathogens in order to circumvent host defense systems (Travis et al. (2000) Biochim Biophys Acta. 1477, 35-50; Travis et al. (1995) Trends Microbiol. 3, 405-7). For example, IdeS is a naturally occurring 35 kDa cysteine protease, specifically an endopeptidase, expressed by the pathogenic bacteria *Streptococcus pyogenes* that exhibits specificity for its target sequence found in human IgG, in addition to several other species. IdeS is capable of cleaving IgG below the hinge region (von Pawel-Rammingen et al. (2003) Curr Opin Microbiol. 6, 50-5), leading to the generation of F(ab')2 and Fc/2 fragments (Ryan et al. (2008) Mol Immunol. 45, 1837-46).

By way of further example, EndoS is a naturally occurring glycosidase, specifically an endoglycosidase, from S. *pyogenes,* that specifically hydrolyzes glycans from human IgG and alters antibody effector functions, including Fc receptor binding.

Described herein are, *inter alia,* methods for treating patients that may develop or already have pre-existing neutralizing antibodies to gene therapy vectors, by administering a protease that cleaves peptide bonds present in immunoglobulins or by administering a glycosidase that cleaves carbohydrate residues present on immunoglobulins, or other similar enzymatic cleavage of immunoglobulins *in vivo.* Also described herein are, *inter alia,* methods for treating patients that may develop or already have pre-existing antibodies that bind to a heterologous polynucleotide or a protein or peptide encoded by the heterologous polynucleotide encapsidated by a gene therapy vector by administering a protease that cleaves peptide bonds present in immunoglobulins or by administering a glycosidase that cleaves carbohydrate residues present on immunoglobulins, or other similar enzymatic cleavage of immunoglobulins *in vivo.*

### Summary

Disclosed herein are methods for utilizing a protease such as IdeS to reduce antibody *(e.g.,* IgG) levels in human plasma. Methods according to the instant invention may be used, *inter alia,* to treat patients with pre-existing neutralizing antibodies to gene therapy vectors and to re-dose patients previously treated with a gene therapy vector.

In certain embodiments, a method of treating a subject in need of treatment for a disease caused by a loss of function or activity of a protein includes: (a) administering to the subject a recombinant viral vector comprising a heterologous polynucleotide that encodes a protein or peptide that provides or supplements a function or activity of the protein; and (b) administering to the subject an amount of a protease or glycosidase effective to degrade or digest and/or inhibit or reduce effector function of antibodies that bind to said recombinant viral vector and/or the protein or peptide encoded by the heterologous polynucleotide.

In certain embodiments,a method of treating a subject in need of treatment for a disease caused by a gain of function activity or expression, of a protein includes: (a) administering to the subject a recombinant viral vector comprising a heterologous polynucleotide that is transcribed into a nucleic acid that inhibits, decreases or reduces expression of the gain of function, activity or expression of said protein; and (b) administering to the subject a protease or glycosidase effective to degrade or digest and/or inhibit or reduce effector function of antibodies that bind to said recombinant viral vector.

In certain embodiments, a method of treating a subject in need of treatment for a disease caused by a loss of function or activity of a protein includes: (a) administering to the subject a protease or glycosidase effective to degrade or digest and/or inhibit or reduce effector function of viral vector binding antibodies; and (b) administering to the subject a recombinant viral vector comprising a heterologous polynucleotide that encodes a protein or peptide that provides or supplements a function or activity of said protein.

In certain embodiments, a method of treating a subject in need of treatment for a disease caused by a gain of function activity or expression, of a protein includes: (a) administering to the subject a protease or glycosidase effective to degrade or digest and/or inhibit or reduce effector function of viral vector binding antibodies; and (b) administering to the subject a recombinant viral vector comprising a heterologous polynucleotide that is transcribed into a nucleic acid that inhibits, decreases or reduces expression of the gain of function, activity or expression of said protein.

In certain embodiments, in methods of treating a subject step (b) is performed within about 90 days after step (a) is performed. In certain embodiments, step (b) is performed within about 60 days before or after step (a). In certain embodiments, step (b) is performed within about 45 days before or after step (a). In certain embodiments, step (b) is performed within about 30 days before or after step (a). In certain embodiments, step (b) is performed within about 21 days before or after step (a). In certain embodiments, step (b) is performed within about 14 days before or after step (a). In certain embodiments, step (b) is performed within about 7 days before or after step (a). In certain embodiments, step (b) is performed within about 72 hours before or after step (a). In certain embodiments, step (b) is performed within about 48 hours before or after step (a). In certain embodiments, step (b) is performed within about 24 hours before or after step (a). In certain embodiments, step (b) is performed within about 12 hours before or after step (a). In certain embodiments, step (b) is performed within about 6 hours before or after step (a).

In certain embodiments, the protease comprises a cysteine protease or a thiol protease. In certain embodiments, the protease comprises a protease from *Streptococcus pyogenes, Streptococcus equi* or *Mycoplasma canis.* In certain embodiments, the protease comprises IdeS or a modified variant thereof set forth in any of SEQ ID NOs:3 - 18 23 or 48.

In certain embodiments, the glycosidase comprises an endoglycosidase. In certain embodiments, the endoglycosidase comprises a sequence set forth in any of SEQ ID NOs:44 - 47.

In certain embodiments, the protease or glycosidase degrades or digests and/or inhibits or reduces effector function of human antibodies.

In certain embodiments, the viral vector comprises a lentiviral vector, an adenoviral vector or an adeno-associated virus (AAV) vector.

In certain embodiments, the lentiviral vector includes envelope proteins to which the antibodies bind.

In certain embodiments, the AAV vector includes capsid proteins to which the antibodies bind.

In certain embodiments, the AAV vector comprises VP1, VP2 and/or VP3 capsid proteins to which the antibodies bind.

In certain embodiments, the AAV vector comprises VP1, VP2 and/or VP3 capsid protein having 60% or more sequence identity to VP1, VP2 and/or VP3 capsid protein selected from the group consisting of AAV1, AAV2, AAV3, AAV4, AAV5, AAV6, AAV7, AAV8, AAV9, AAV10, AAV3B, AAV-2i8, Rh10, Rh74, SEQ ID NO:1 and SEQ ID NO:2 VP1, VP2 and/or VP3 capsid proteins.

In certain embodiments, the AAV vector comprises VP1, VP2 and/or VP3 capsid protein having 100% sequence identity to VP1, VP2 and/or VP3 capsid protein selected from the group consisting of AAV1, AAV2, AAV3, AAV4, AAV5, AAV6, AAV7, AAV8, AAV9, AAV10, AAV3B, AAV-2i8, Rh10, Rh74, SEQ ID NO:1 and SEQ ID NO:2 VP1, VP2 and/or VP3 capsid proteins.

In certain embodiments, the subject has antibodies that bind to said viral vector.

In certain embodiments, antibodies that bind to the viral vector are absent from the subject.

In certain embodiments, the subject has antibodies that bind to the protein or peptide encoded by the heterologous polynucleotide.

In certain embodiments, the antibodies comprise IgG, IgM, IgA, IgD and/or IgE.

In certain embodiments, a method includes determining the presence of, quantifying the amount of or an effector function of viral vector binding antibodies present in said subject before performing step (a), after performing step (a) but before performing step (b) and/or after performing steps (a) and (b).

In certain embodiments, a method includes analyzing a biological sample from said subject for the presence, amount or an effector function of viral vector binding antibodies present in said sample before performing step (a), after performing step (a) but before performing step (b) and/or after performing steps (a) and (b).

In certain embodiments, a biological sample from the subject is a blood product. In certain embodiments, a blood product comprises plasma or serum.

In certain embodiments, a method leads to a reduction of 20-50%, 50-75%, 75-90%, 90-95% or 95% or more of said viral vector binding antibodies.

In certain embodiments, the viral vector binding antibodies present in the biological sample or blood product from the subject is less than about 1:100,000 where 1 part of said biological sample or blood product diluted in 100,000 parts of buffer results in 50% viral vector neutralization.

In certain embodiments, the viral vector binding antibodies present in said biological sample or blood product from said subject is less than about 1:50,000, where 1 part of the biological sample or blood product diluted in 50,000 parts of buffer results in 50% viral vector neutralization.

In certain embodiments, the viral vector binding antibodies present in the biological sample or blood product from the subject is less than about 1:10,000, where 1 part of the biological sample or blood product diluted in 10,000 parts of buffer results in 50% viral vector neutralization.

In certain embodiments, the viral vector binding antibodies present in the biological sample or blood product from the subject is less than about 1:1,000, where 1 part of the biological sample or blood product diluted in 1,000 parts of buffer results in 50% viral vector neutralization.

In certain embodiments, the viral vector binding antibodies present in the biological sample or blood product from the subject is less than about 1:100, where 1 part of the biological sample or blood product diluted in 100 parts of buffer results in 50% viral vector neutralization.

In certain embodiments, the viral vector binding antibodies present in the biological sample or blood product from the subject is less than about 1:10, where 1 part of the biological sample or blood product diluted in 10 parts of buffer results in 50% viral vector neutralization.

In certain embodiments, the viral vector binding antibodies present in the biological sample or blood product is less than about 1:5, where 1 part of the biological sample or blood product diluted in 5 parts of buffer results in 50% viral vector neutralization.

In certain embodiments, the ratio of viral vector binding antibodies present in the biological sample or blood product is less than about 1:4, where 1 part of the biological sample or blood product diluted in 4 parts of buffer results in 50% viral vector neutralization.

In certain embodiments, the ratio of viral vector binding antibodies present in the biological sample or blood product is less than about 1:3, where 1 part of the biological sample or blood product diluted in 3 parts of buffer results in 50% viral vector neutralization.

In certain embodiments, the ratio of viral vector binding antibodies present in the subject, biological sample or blood product is less than about 1:2, where 1 part of the biological sample or blood product diluted in 2 parts of buffer results in 50% viral vector neutralization.

In certain embodiments, the ratio of viral vector binding antibodies present in the subject, biological sample or blood product is less than about 1:1, where 1 part of the biological sample or blood product diluted in 1 part of buffer results in 50% viral vector neutralization.

In certain embodiments, a method includes determining the presence of or quantifying the amount of antibodies that bind to the polypeptide or peptide encoded by the heterologous polynucleotide after performing step (a) but before performing step (b) and/or after performing steps (a) and (b).

In certain embodiments, a method includes determining the presence of or quantifying the amount of antibodies that bind to the nucleic acid after performing step (a) but before performing step (b) and/or after performing steps (a) and (b).

In certain embodiments, a subject has a lung disease (*e.g*., cystic fibrosis), a bleeding disorder (*e.g*., hemophilia A or hemophilia B with or without inhibitors), thalassemia, a blood disorder (e.g., anemia), Alzheimer's disease, Parkinson's disease, Huntington's disease, amyotrophic lateral sclerosis (ALS), epilepsy, a lysosomal storage disease (*e.g*., aspartylglucosaminuria, Batten disease, late infantile neuronal ceroid lipofuscinosis type 2 (CLN2), cystinosis, Fabry disease, Gaucher disease types I, II, and III, glycogen storage disease II (Pompe disease), GM2-gangliosidosis type I (Tay Sachs disease), GM2-gangliosidosis type II (Sandhoff disease), mucolipidosis types I (sialidosis type I and II), II (I-cell disease), III (pseudo-Hurler disease) and IV, mucopolysaccharide storage diseases (Hurler disease and variants, Hunter, Sanfilippo Types A,B,C,D, Morquio Types A and B, Maroteaux-Lamy and Sly diseases), Niemann-Pick disease types A/B, C1 and C2, and Schindler disease types I and II), hereditary angioedema (HAE), a copper or iron accumulation disorder (*e.g.*, Wilson's or Menkes disease), lysosomal acid lipase deficiency, a neurological or neurodegenerative disorder, cancer, type 1 or type 2 diabetes, adenosine deaminase deficiency, a metabolic defect (*e.g.,* glycogen storage diseases), a disease of solid organs (*e.g.,* brain, liver, kidney, heart), or an infectious viral (*e.g.,* hepatitis B and C, HIV, etc.), bacterial or fungal disease. In certain embodiments, a subject has a blood clotting disorder. In certain embodiments, a subject has hemophilia A, hemophilia A with inhibitory antibodies, hemophilia B, hemophilia B with inhibitory antibodies, a deficiency in any coagulation Factor: VII, VIII, IX, X, XI, V, XII, II, von Willebrand factor, or a combined FV/FVIII deficiency, thalassemia, vitamin K epoxide reductase C1 deficiency or gamma-carboxylase deficiency.

In certain embodiments, a subject has anemia, bleeding associated with trauma, injury, thrombosis, thrombocytopenia, stroke, coagulopathy, disseminated intravascular coagulation (DIC); over-anticoagulation associated with heparin, low molecular weight heparin, pentasaccharide, warfarin, small molecule antithrombotics (*i.e.,* FXa inhibitors), or a platelet disorder such as, Bernard Soulier syndrome, Glanzmann thrombasthenia, or storage pool deficiency.

In certain embodiments, a subject has a disease that affects or originates in the central nervous system (CNS). In certain embodiments, the disease is a neurodegenerative disease. In certain embodiments, the CNS or neurodegenerative disease is Alzheimer's disease, Huntington's disease, ALS, hereditary spastic hemiplegia, primary lateral sclerosis, spinal muscular atrophy, Kennedy's disease, a polyglutamine repeat disease, or Parkinson's disease. In certain embodiments, the CNS or neurodegenerative disease is a polyglutamine repeat disease. In certain embodiments, the polyglutamine repeat disease is a spinocerebellar ataxia (SCA1, SCA2, SCA3, SCA6, SCA7, or SCA17).

In certain embodiments, the heterologous polynucleotide encodes a protein selected from the group consisting of insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor α (TGFα), platelet-derived growth factor (PDGF), insulin growth factors I and II (IGF-I and IGF-II), TGFβ, activins, inhibins, bone morphogenic protein (BMP), nerve growth factor (NGF), brain-derived neurotrophic factor (BDNF), neurotrophins NT-3 and NT4/5, ciliary neurotrophic factor (CNTF), glial cell line derived neurotrophic factor (GDNF), neurturin, agrin, netrin-1 and netrin-2, hepatocyte growth factor (HGF), ephrins, noggin, sonic hedgehog and tyrosine hydroxylase.

In certain embodiments, the heterologous polynucleotide encodes a protein selected from the group consisting of thrombopoietin (TPO), interleukins (IL1 through IL-36), monocyte chemoattractant protein, leukemia inhibitory factor, granulocyte-macrophage colony stimulating factor, Fas ligand, tumor necrosis factors α and β, interferons α, β, and γ, stem cell factor, flk-2/flt3 ligand, IgG, IgM, IgA, IgD and IgE, chimeric immunoglobulins, humanized antibodies, single chain antibodies, T cell receptors, chimeric T cell receptors, single chain T cell receptors, class I and class II MHC molecules.

In certain embodiments, the heterologous polynucleotide encodes CFTR (cystic fibrosis transmembrane regulator protein), a blood coagulation (clotting) factor (Factor XIII, Factor IX, Factor VIII, Factor X, Factor VII, Factor VIIa, protein C, etc.), a gain of function blood coagulation factor, an antibody, retinal pigment epithelium-specific 65 kDa protein (RPE65), erythropoietin, LDL receptor, lipoprotein lipase, ornithine transcarbamylase, β-globin, α-globin, spectrin, α-antitrypsin, adenosine deaminase (ADA), a metal transporter (ATP7A or ATP7), sulfamidase, an enzyme involved in lysosomal storage disease (ARSA), hypoxanthine guanine phosphoribosyl transferase, β-25 glucocerebrosidase, sphingomyelinase, lysosomal hexosaminidase, branched-chain keto acid dehydrogenase, a hormone, a growth factor, insulin-like growth factor 1 or 2, platelet derived growth factor, epidermal growth factor, nerve growth factor, neurotrophic factor-3 and -4, brain-derived neurotrophic factor, glial derived growth factor, transforming growth factor α and β, a cytokine, α-interferon, β-interferon, interferon-y, interleukin-2, interleukin-4, interleukin 12, granulocyte-macrophage colony stimulating factor, lymphotoxin, a suicide gene product, herpes simplex virus thymidine kinase, cytosine deaminase, diphtheria toxin, cytochrome P450, deoxycytidine kinase, tumor necrosis factor, a drug resistance protein, a tumor suppressor protein (*e.g.,* p53, Rb, Wt-1, NF1, Von Hippel-Lindau (VHL), adenomatous polyposis coli (APC)), a peptide with immunomodulatory properties, a tolerogenic or immunogenic peptide or protein Tregitope or hCDR1, insulin, glucokinase, guanylate cyclase 2D (LCA-GUCY2D), Rab escort protein 1 (Choroideremia), LCA 5 (LCA-Lebercilin), ornithine ketoacid aminotransferase (Gyrate Atrophy), Retinoschisin 1 (X-linked Retinoschisis), USH1C (Usher's Syndrome 1C), X-linked retinitis pigmentosa GTPase (XLRP), MERTK (AR forms of RP: retinitis pigmentosa), DFNB1 (Connexin 26 deafness), ACHM 2, 3 and 4 (Achromatopsia), PKD-1 or PKD-2 (Polycystic kidney disease), TPP1, CLN2, a sulfatase, N-acetylglucosamine-1-phosphate transferase, cathepsin A, GM2-AP, NPC1, VPC2, a sphingolipid activator protein, one or more zinc finger nuclease for genome editing, and one or more donor sequence used as repair templates for genome editing.

In certain embodiments, the heterologous polynucleotide encodes an inhibitory nucleic acid. In certain embodiments, the inhibitory nucleic acid is selected from the group consisting of a siRNA, an antisense molecule, miRNA, RNAi, a ribozyme and a shRNA. In certain embodiments, the inhibitory nucleic acid binds to a gene, a transcript of a gene, or a transcript of a gene associated with a polynucleotide repeat disease selected from the group consisting of a huntingtin (HTT) gene, a gene associated with dentatorubropallidoluysian atrophy (atrophin 1, ATN1), androgen receptor on the X chromosome in spinobulbar muscular atrophy, human Ataxin-1, -2, -3, and -7, Caᵥ2.1 P/Q voltage-dependent calcium channel (CACNA1A), TATA-binding protein, Ataxin 8 opposite strand (ATXN8OS), Serine/threonine-protein phosphatase 2A 55 kDa regulatory subunit B beta isoform in spinocerebellar ataxia (type 1, 2, 3, 6, 7, 8, 12 17), *FMR1* (fragile X mental retardation 1) in fragile X syndrome, *FMR1* (fragile X mental retardation 1) in fragile X-associated tremor/ataxia syndrome, *FMR1* (fragile X mental retardation 2) or AF4/FMR2 family member 2 in fragile XE mental retardation; Myotonin-protein kinase (MT-PK) in myotonic dystrophy; Frataxin in Friedreich's ataxia; a mutant of superoxide dismutase 1 (SOD1) gene in amyotrophic lateral sclerosis; a gene involved in pathogenesis of Parkinson's disease and/or Alzheimer's disease; apolipoprotein B (APOB) and proprotein convertase subtilisin/kexin type 9 (PCSK9), hypercholesterolemia; HIV Tat, human immunodeficiency virus transactivator of transcription gene, in HIV infection; HIV TAR, HIV TAR, human immunodeficiency virus transactivator response element gene, in HIV infection; C-C chemokine receptor (CCR5) in HIV infection; Rous sarcoma virus (RSV) nucleocapsid protein in RSV infection, liver-specific microRNA (miR-122) in hepatitis C virus infection; p53, acute kidney injury or delayed graft function kidney transplant or kidney injury acute renal failure; protein kinase N3 (PKN3) in advance recurrent or metastatic solid malignancies; LMP2, LMP2 also known as proteasome subunit beta-type 9 (PSMB 9), metastatic melanoma; LMP7,also known as proteasome subunit beta-type 8 (PSMB 8), metastatic melanoma; MECL1 also known as proteasome subunit beta-type 10 (PSMB 10), metastatic melanoma; vascular endothelial growth factor (VEGF) in solid tumors; kinesin spindle protein in solid tumors, apoptosis suppressor B-cell CLL/lymphoma (BCL-2) in chronic myeloid leukemia; ribonucleotide reductase M2 (RRM2) in solid tumors; Furin in solid tumors; polo-like kinase 1 (PLK1) in liver tumors, diacylglycerol acyltransferase 1 (DGAT1) in hepatitis C infection, beta-catenin in familial adenomatous polyposis; beta2 adrenergic receptor, glaucoma; RTP801/Redd1 also known as DNA damage-inducible transcript 4 protein, in diabetic macular edema (DME) or age-related macular degeneration; vascular endothelial growth factor receptor I (VEGFR1) in age-related macular degeneration or choroidal neovascularization, caspase 2 in non-arteritic ischaemic optic neuropathy; Keratin 6A N17K mutant protein in pachyonychia congenital; influenza A virus genome/gene sequences in influenza infection; severe acute respiratory syndrome (SARS) coronavirus genome/gene sequences in SARS infection; respiratory syncytial virus genome/gene sequences in respiratory syncytial virus infection; Ebola filovirus genome/gene sequence in Ebola infection; hepatitis B and C virus genome/gene sequences in hepatitis B and C infection; herpes simplex virus (HSV) genome/gene sequences in HSV infection, coxsackievirus B3 genome/gene sequences in coxsackievirus B3 infection; silencing of a pathogenic allele of a gene (allele-specific silencing) like torsin A (TOR1A) in primary dystonia, pan-class I and HLA-allele specific in transplant; and mutant rhodopsin gene (RHO) in autosomal dominantly inherited retinitis pigmentosa (adRP).

In certain embodiments, the protein encoded by the heterologous polynucleotide comprises a gene editing nuclease. In certain embodiments, the gene editing nuclease comprises a zinc finger nuclease (ZFN) or a transcription activator-like effector nuclease (TALEN). In certain embodiments, the gene editing nuclease comprises a functional Type II CRISPR-Cas9.

In certain embodiments, step (a) and/or step (b) of a method according to the instant invention are performed two or more times.

In certain embodiments, the subject is a mammal. In certain embodiments, the subject is a human.

Also disclosed herein are compositions, for example and without limitation, packages and kits, having components that may be used to practice methods according to the instant invention.

In certain embodiments, a package or kit has disposed therein: (a) a recombinant viral vector comprising a heterologous polynucleotide that encodes a protein or peptide; (b) a protease or glycosidase that degrades or digests antibodies; and (c) a label with instructions for performing a method as disclosed herein. In certain embodiments, (a) and (b) are in separate or the same container.

In certain embodiments, a package or kit has disposed therein: (a) a recombinant viral vector comprising a heterologous polynucleotide that is transcribed into a nucleic acid that inhibits, decreases or reduces expression of a protein; (b) a protease or glycosidase that degrades or digests antibodies; and (c) a label with instructions for performing a method as disclosed herein. In certain embodiments, (a) and (b) are in separate or the same container.

In certain embodiments, a method for treating a disease treated by gene therapy using a vector comprising administering to a subject in need thereof a therapeutically effective amount of an immunoglobulin G-degrading enzyme polypetide.

### Description of Drawings

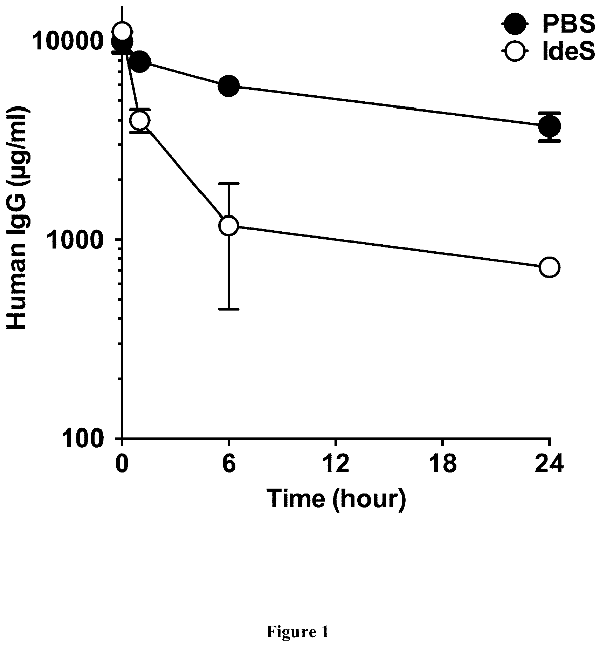

**Figure 1** is a graph showing hydrolysis of human IgG in mice reconstituted with IVIg. Wild-type C57BL/6 mice were reconstituted with human IgG (IVIg, 9 mg/mouse) and injected 30 min later with IdeS (250 IU/mouse, 3 mice) or PBS (2 mice). Blood was collected before the injection of IdeS and 1, 6 and 10 hours later. Intact human IgG was measured in mouse serum by ELISA and is expressed as µg/mL using IVIg as a standard.

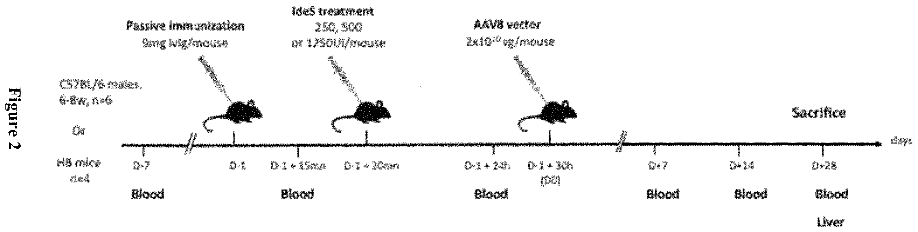

**Figure 2** is a scheme of the experimental protocol for the in vivo neutralization of anti-AAV IgG. Wild-type C57BL/6 mice (6 mice per group) or Hemophilia B (HB) mice (4 mice per group) were reconstituted with human IgG (IVIg, 9 mg/mouse, day (D) minus 1 (D-1)) and injected 30 minutes (min, mn) later with IdeS (250, 500 or 1250 IU/mouse, D-1 + 30 min). Thirty hours (hr, h) later (D0), the mice were injected with AAV8 vector (2×1010 vg/mouse). Blood was collected 15 min after IVIg injection, 24 hours after IdeS injection, as well as at D-7, D7, D14 and D28. At euthanasia (D28), livers were collected for a vector genome copy number (VGCN) assessment. Control mice received IVIg alone, IdeS alone or PBS only prior to AAV vectors injection.

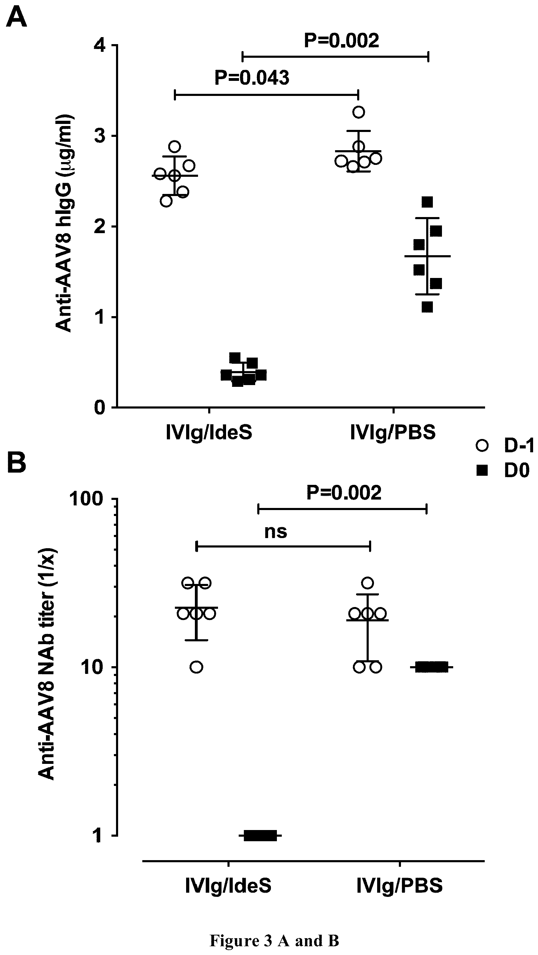

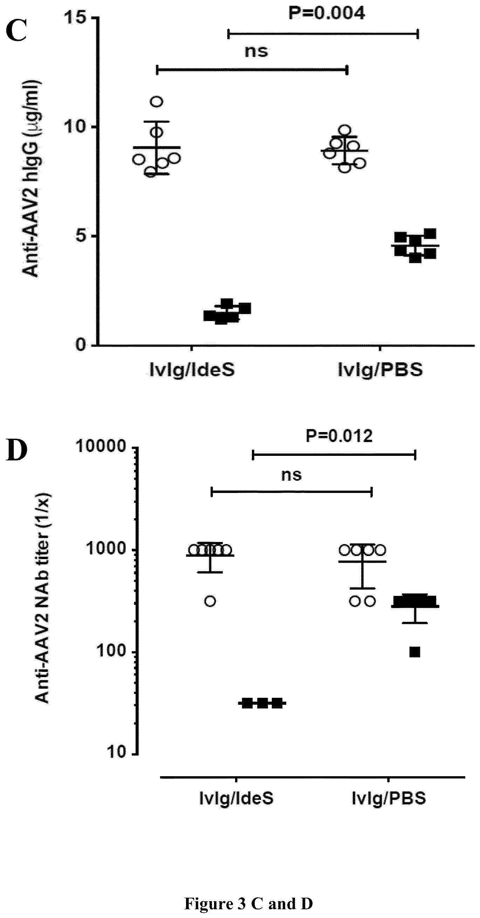

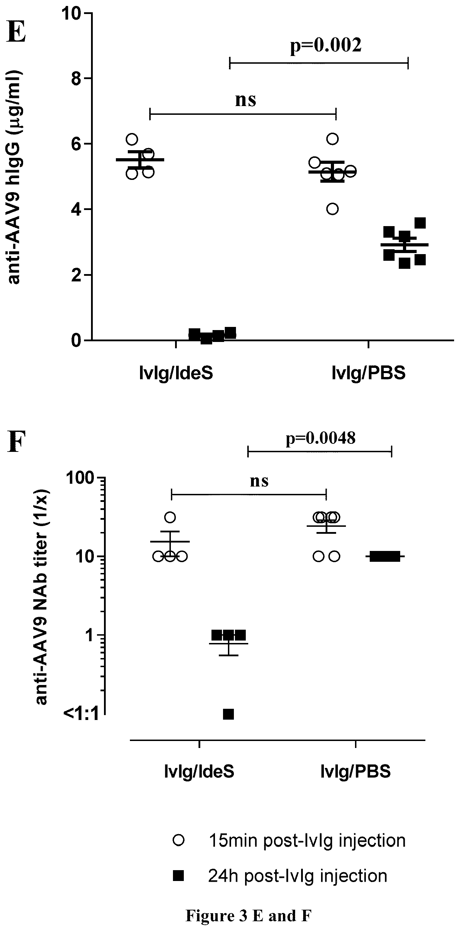

**Figures 3A, 3B****,** **3C, 3D****,** **3E, and 3F** are graphs showing the effect of IdeS on human anti-AAV8 IgG, anti-AAV8 neutralizing antibody (NAb), anti-AAV2 IgG, anti-AAV2 NAb, anti-AAV9 IgG, and anti-AAV9 NAb, respectively, in passively immunized mice. Blood was collected from mice (6 mice/group) injected with IVIg and IdeS (IVIg/IdeS) or with IVIg alone (IVIg/PBS), at D-1+15min and D-1+24 (see scheme in Figure 2). The dose of IdeS was 250 IU/mouse in Figs.
3A, 3B, 3C and 3D, and 500 IU/mouse in Figs. 3E and 3F. The levels of anti-AAV IgG were measured by ELISA and the levels of AAV NAbs were measured by a neutralization assay. Levels of anti-AAV8, anti-AAV2 and anti-AAV9 IgG, respectively in Figs. 3A, 3C and 3E, are expressed in µg/mL. Levels of anti-AAV8, anti-AAV2 and anti-AAV9 NAb, respectively in Figs. 3B, 3D and 3F, are expressed in titer (1/x). Data are represented as mean ± SD. Statistical differences were assessed using the non-parametric two-sided Mann-Whitney test (ns: not significant).

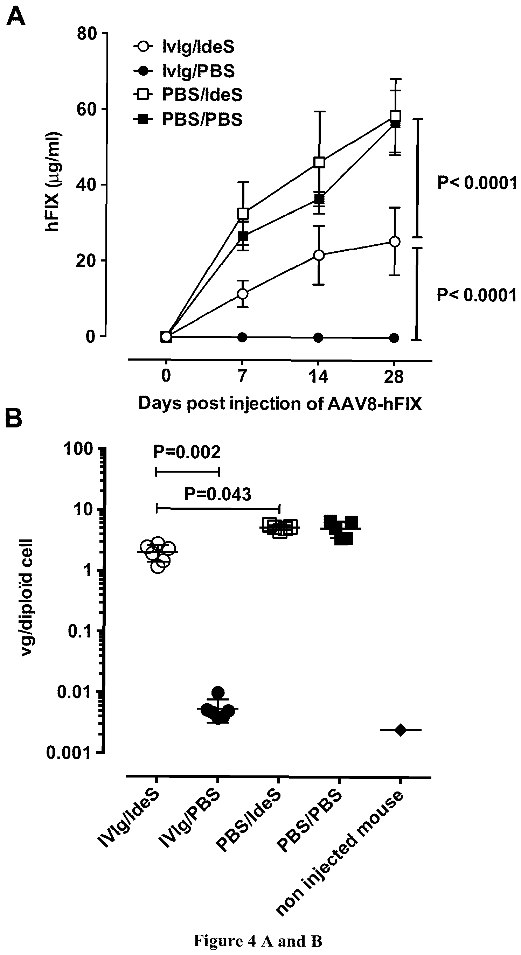

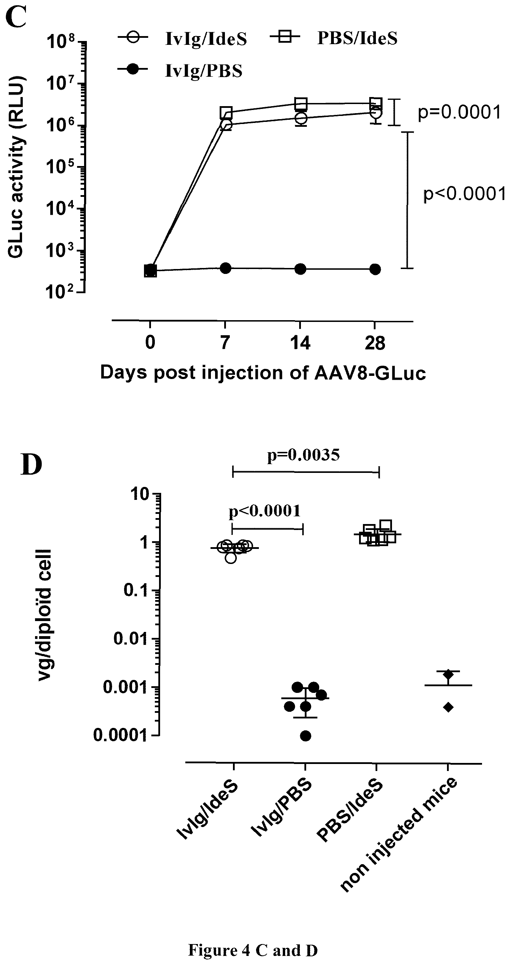

**Figures 4A, 4B****,** **4C and 4D** are graphs showing that IdeS treatment rescues transgene expression with AAV8 vectors in passively immunized mice. Mice (6 mice/group) passively immunized with IVIg or PBS and injected with IdeS (250 IU/mouse (Figs. 4A and 4B) or 1250 IU/mouse (Figs. 4C and 4D)) or PBS were treated with 2×1010 vg/mouse AAV8 vector encoding human Factor IX (hFIX) (Figs. 4A and 4B) or Gaussia luciferase (GLuc) (Figs. 4C and 4D). Fig. 4A: the hFIX plasma levels were measured using a specific anti-hFIX ELISA. hFIX levels are expressed as µg/mL using purified hFIX as a standard. Fig. 4C: the Gaussia luciferase activity in plasma was measured using a luminescence assay. GLuc activity is expressed as RLU (Relative Light Units). Figs. 4B and 4D: vector genome copy number per diploid genome in the liver of mice by qPCR at the time of sacrifice. Data are represented as mean ± SD. Statistical analyses were performed by two-way ANOVA with Dunnett test.

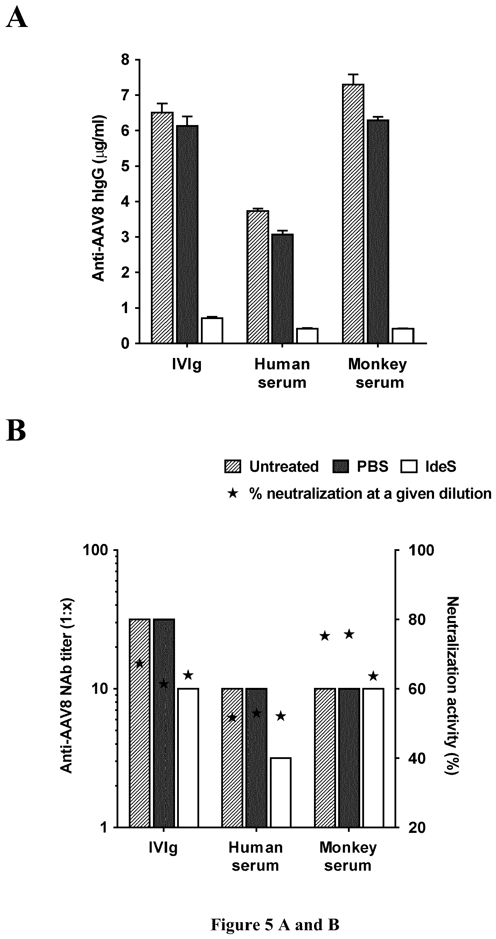

**Figures 5A and 5B** are graphs showing the assay of in vitro digestion of human and non-human primate anti-AAV8 IgG by IdeS. IVIg (200 µg), serum from an AAV8 seropositive human, and serum from an AAV8 seropositive monkey were incubated with IdeS (100 IU) or PBS for 22 hr at 37 °C. Fig. 5A depicts the levels of anti-AAV8 IgG measured by ELISA. Fig. 5B depicts results of an AAV neutralization assay (left Y axis) and the % of neutralizing activity observed at the dilution (right Y axis, black stars). Data are represented as mean ± SD.

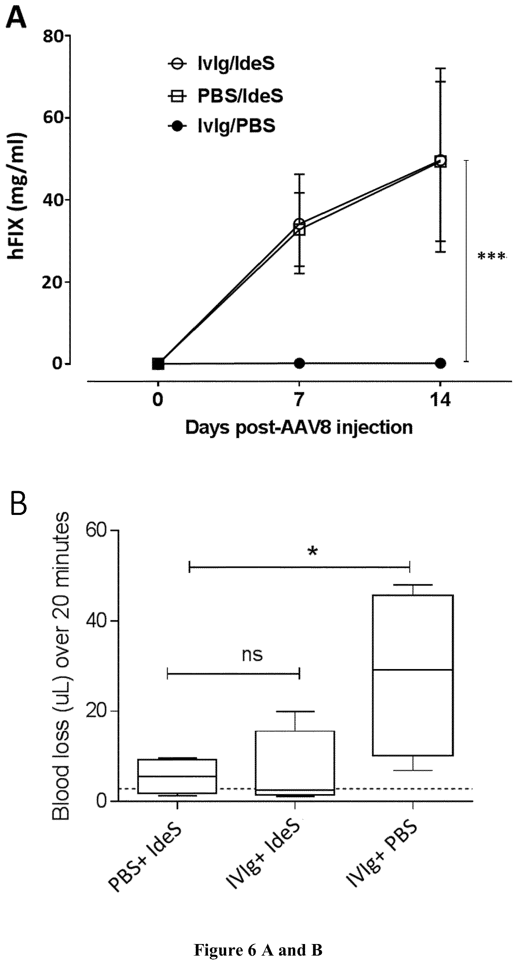

**Figures 6A and 6B** are graphs showing that IdeS treatment allows efficient gene therapy with AAV8 vectors in passively immunized hemophilia B (HB) mice. HB mice (4 mice per group) were reconstituted with human IgG (IVIg, 9 mg/mouse, D-1) and injected 30 min later with IdeS (1250 IU/mouse, D-1 + 30 min). 24 hours later (D0), mice were injected with an AAV8 vector carrying a hFIX transgene (2×1010 vg/mouse). Blood was collected 24 hours after IdeS injection, as well as at D7 and D14. Levels of hFIX were measured in plasma using a dedicated ELISA (Fig.
6A). At D21, the efficacy of gene therapy in HB mice was assessed using a Tail clip bleeding assay, where blood loss over 20 minutes was estimated (Fig. 6B). Data are represented as mean ± SD. Statistical analyses were performed by two-way ANOVA with Dunnett test for hFIX ELISA and one-way ANOVA with Dunnett test for tail clip assay (^{∗}: p<0.05; ^{∗∗∗}: p<0.001)

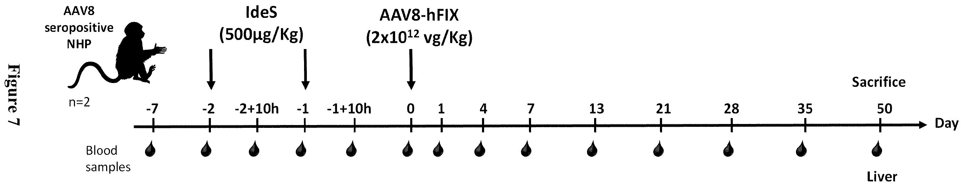

**Figure 7** is a scheme of the experimental protocol in NHPs. Two male cynomolgus monkeys, seropositive for AAV8 (NAb titer 1:17.2), were infused with 2×1012 vg/kg of AAV8-hFIX vector (D0). Before vector injection, NHP2 received double injections (i.v.) of IdeS (500 µg/kg) at days D-2 and D-1 (NHP treated). NHP1 received no prior treatment (control NHP). Blood samples were collected before and after IdeS injections, as well as at D0, D1, D4, D7, D13, D21, D28, D35 and D50. At euthanasia (D50), liver biopsies were collected from 4 lobes for a VGCN assessment.

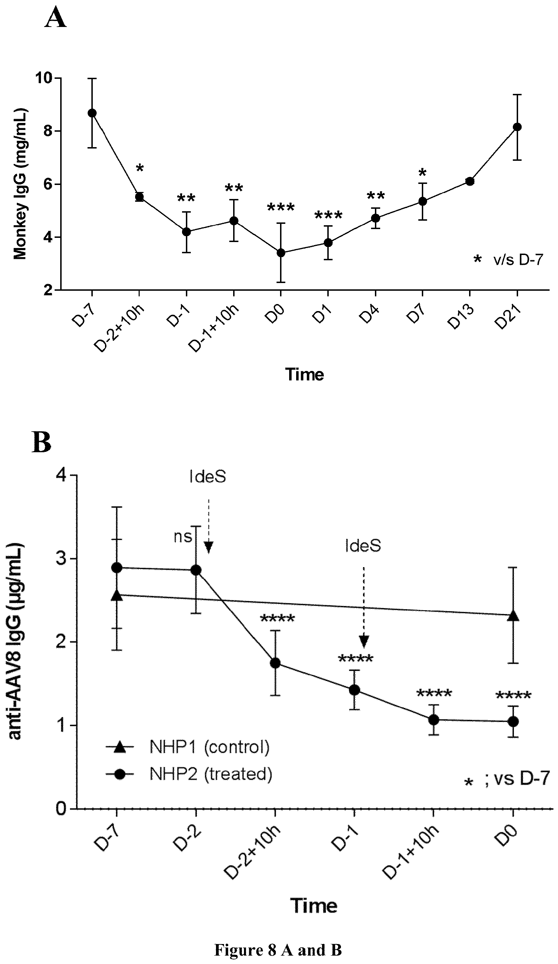

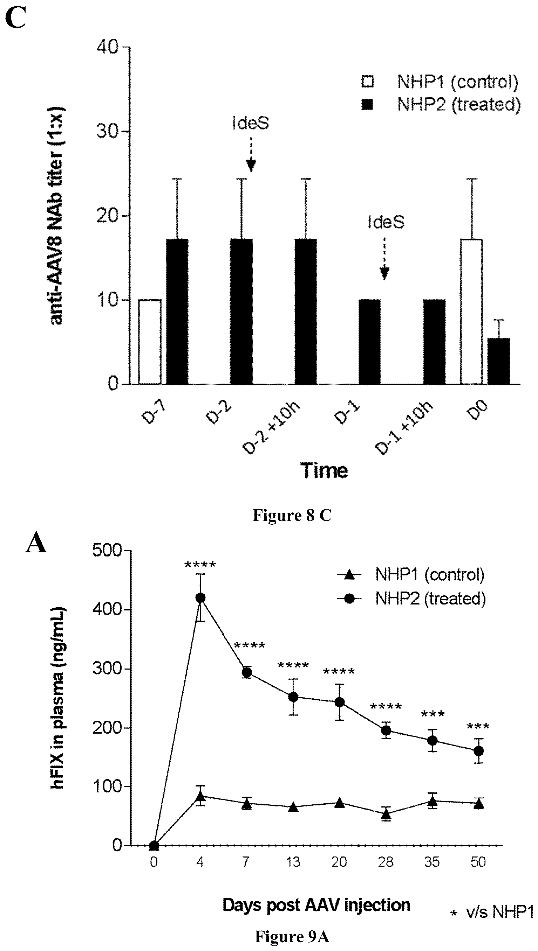

**Figures 8A, 8B** **and** **8C** are graphs showing the effect of IdeS treatment on IgG in a AAV8-seropositive monkey. Fig. 8A: Intact IgG level in serum was measured by ELISA and is expressed as mg/mL, using purified monkey IgG as a standard. Fig. 8B: Anti-AAV8 IgG level in serum was measured before and after IdeS treatment by ELISA. In the treated monkey (NHP2), the anti-AAV8 IgG level was 2.7-fold reduced after the injection of IdeS (from 2.86 to 1.05 µg/mL). Fig. 8C: Anti-AAV8 NAb level was measured before and after IdeS treatment using a neutralization assay. In the treated monkey (NHP2), the anti-AAV8 NAb titer was 3-fold reduced (titer from 17.2 to 5.4). Data are represented as mean ± SD. Statistical analyses were performed by two-way ANOVA with Tukey test (ns: not significant; ^{∗}: p<0.05; ^{∗∗}: p<0.01; ^{∗∗∗}: p<0.001; ^{∗∗∗∗}: p<0.0001).

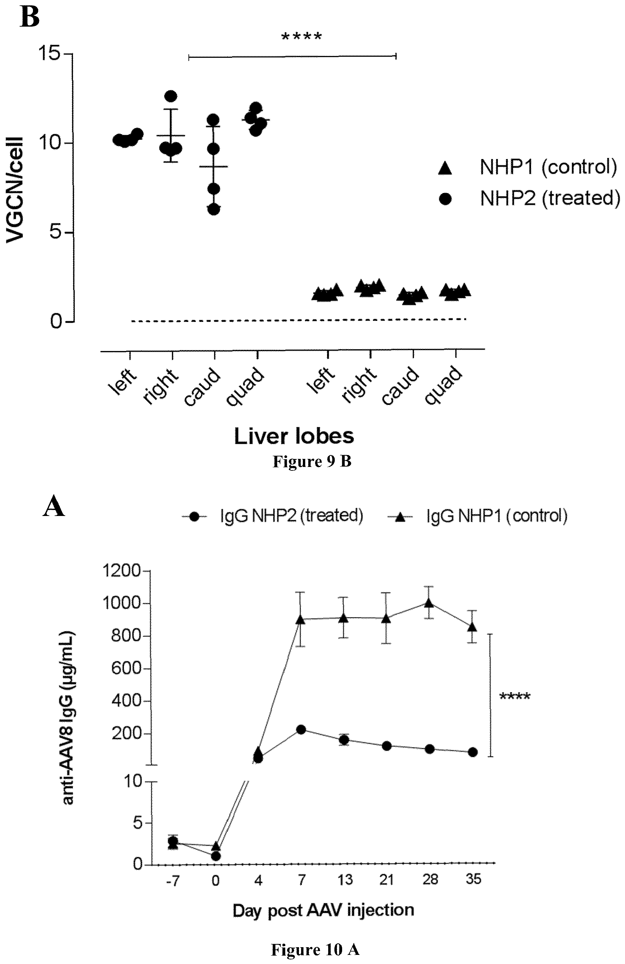

**Figures 9A** **and** **9B** are graphs showing higher transgene expression in an AAV8-seropositive monkey treated with IdeS. Fig. 9A: hFIX plasma levels were measured using a specific anti-hFIX ELISA. hFIX levels are expressed as ng/mL using purified hFIX as a standard. Fig. 9B: vector genome copy number (VGCN) per diploid genome in the liver by qPCR at the time of sacrifice. The VGCN assessment was done from 4 wedge biopsies, taken at different sites of the 4 lobes (right, left, caudate (caud), and quadrate (quad)). Data are represented as mean ± SD. Statistical analyses were performed by two-way ANOVA with Sidak's multiple comparison test (^{∗∗∗}: p<0.001; ^{∗∗∗∗}: p<0.0001). Compared to the control monkey (NHP1), the monkey that received IdeS treatment (NHP2) showed higher levels of hFIX in plasma and more efficient AAV liver transduction.

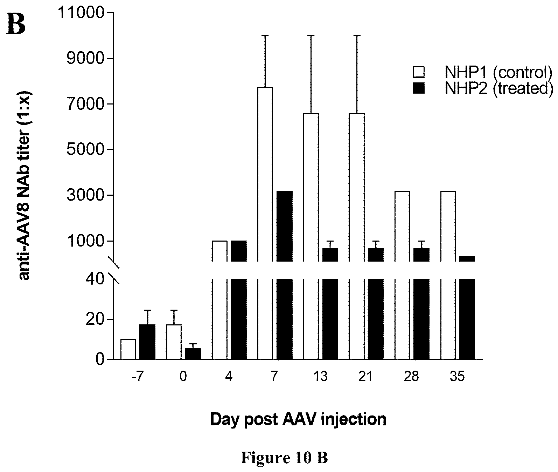

**Figures 10A** **and** **10B** are graphs shows levels of anti-AAV8 IgG and anti-AAV8 NAb, respectively, in an AAV8-seropositive monkey treated with IdeS. The levels of anti-AAV8 IgG were measured by ELISA and are expressed in µg/mL (Figure 10A). Anti-AAV8 NAb were measured in neutralization assays and are expressed in titer (1:x) (Figure 10B). Data are represented as mean ± SD. Statistical analyses were performed by two-way ANOVA with Tukey's multiple comparison test (^{∗∗∗∗}: p<0.0001).

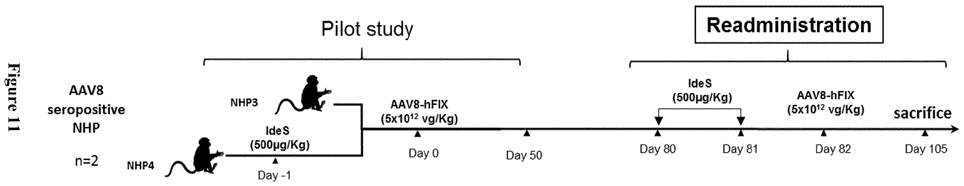

**Figure 11** is a scheme of the experimental protocol for AAV8 re-administration in NHPs. Two males cynomolgus monkeys, seropositive for AAV8 (NAb titer 1:31.6), were infused with 5×1012 vg/kg of AAV8-hFIX vector (D0). Before vector injection, NHP4 received a single intravenous (IV) injection of IdeS (500 µg/kg) at day D-1 (NHP treated). NHP3 received no prior treatment (control NHP). Subsequently, both monkeys received double IV injections of IdeS (500 µg/kg) at days D80 and D81, and 5×1012 vg/kg of AAV8-hFIX vector at D82. Blood samples were collected at different time points during the experiment. At euthanasia (D105), liver biopsies were collected from 4 lobes for a VGCN assessment.

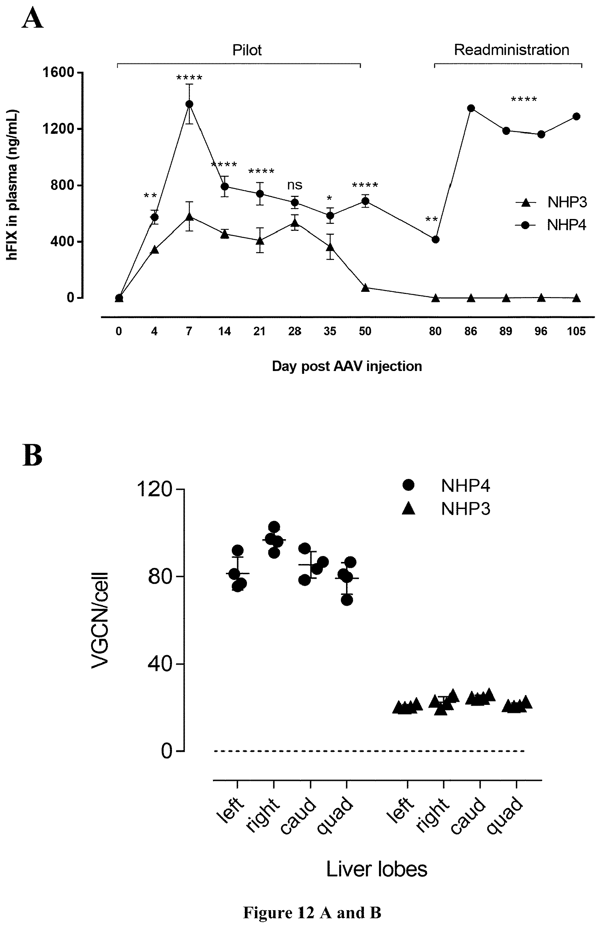

**Figures 12A and 12B** are graphs showing IdeS treatment allows efficient AAV vector re-dosing in an AAV8-seropositive monkey, leading to an increase of transgene expression. Fig. 12A: hFIX plasma levels were measured using a specific anti-hFIX ELISA. hFIX levels are expressed as ng/mL using purified hFIX as a standard. Fig. 12B: vector genome copy number (VGCN) per diploid genome in the liver was determined by qPCR at the time of sacrifice. The VGCN assessment was done from 4 wedge biopsies, taken at different sites of the 4 lobes (left, right, caudate (caud), and quadrate (quad)). Data are represented as mean ± SD. Statistical analyses were performed by two-way ANOVA with Sidak's multiple comparison test (^{∗}: p<0.05; ^{∗∗}: p<0.01; ^{∗∗∗∗}: p<0.0001).

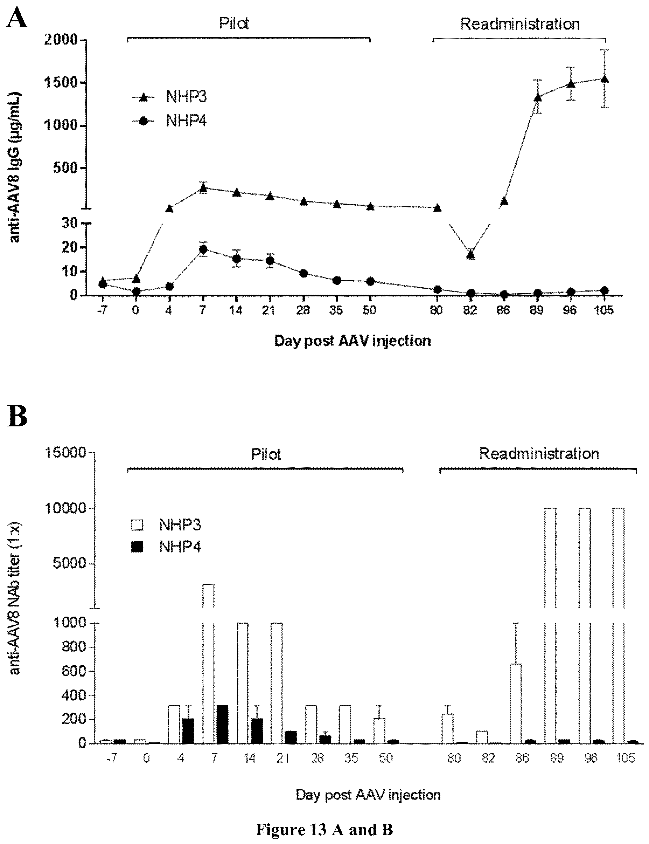

**Figures 13A and 13B** are graphs showing levels of anti-AAV8 IgG and anti-AAV8 NAb, respectively, in AAV8-seropositive monkey treated with IdeS upon re-administration of AAV8 vector. Fig. 13A: Anti-AAV8 IgG levels were measured by ELISA. Fig. 13B: anti-AAV8 NAb titers were measured using a neutralization assay. Data are represented as mean ± SD.

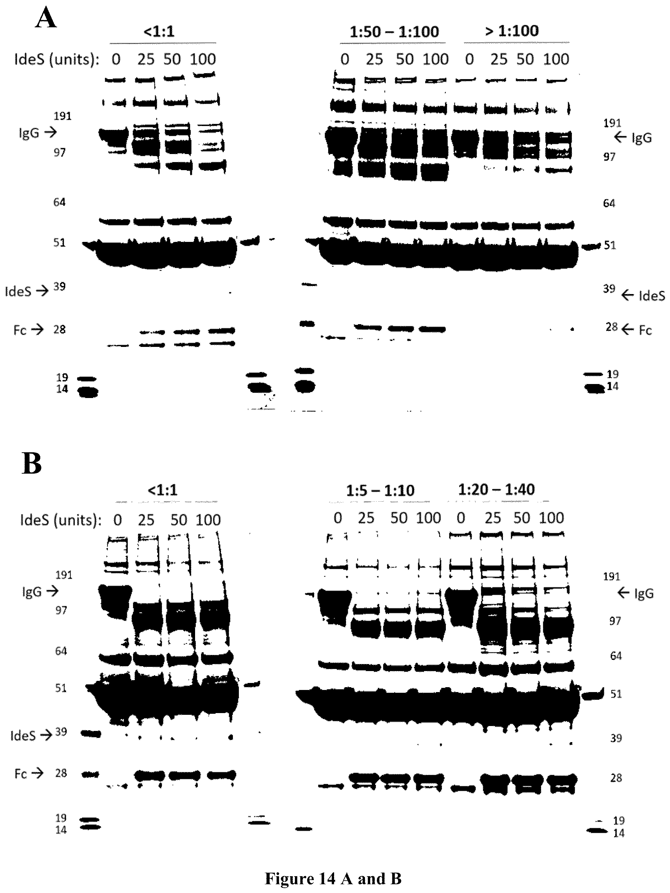

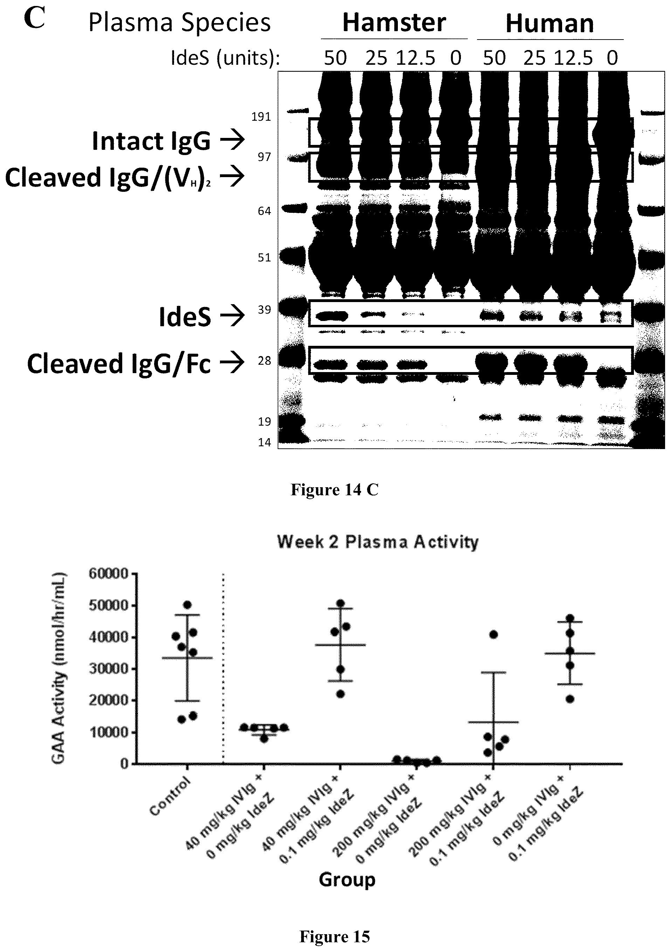

**Figures 14A, 14B** **and** **14C** show SDS-PAGE analyses of cleavage of IgG by IdeS in samples of human patient sera (Fig. 14A), non-human primate (rhesus macaque) plasma (Fig. 14B) and hamster plasma (Fig. 14C), incubated with increasing amounts of IdeS. Samples were incubated without IdeS or with increasing concentrations of IdeS for 1 hr at 37 °C. The reactions were stopped by addition of sample buffer. Samples were analyzed by non-reducing SDS-PAGE and Coomassie stain.
**Figure 15** is a graph showing GAA activity levels in murine plasma after infusion of AAV-Spk1-GAA vector in animals immunized with varying amounts of IVIg that was pre-treated with or without IdeZ. AAV-Spk1-GAA vector was infused one day after IVIg immunization. Transgene activity was assessed by GAA Activity Assay at 2 weeks post vector administration. GAA activity in nmol/hr/mL is plotted for each mouse in each group. Control mice were administered only vector in the absence of IVIg.

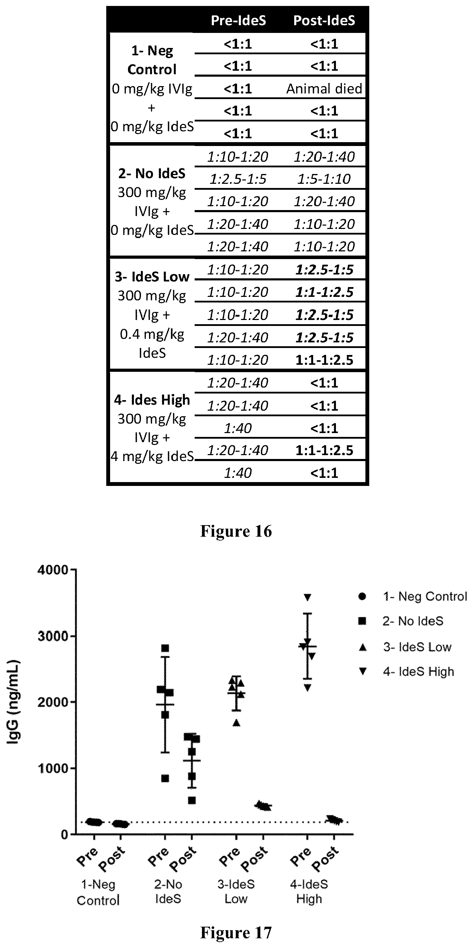

**Figure 16** shows anti-Spkl neutralizing antibody (NAb) titer levels in murine plasma pre- and post-IdeS infusion. Relative NAb titer levels in this study are designated as low titer (<1:1, 1:1-1:2.5) (bold), mid-range (1:2.5-1:5) (bold italics), and high (>1:5-1:10) (italics).
**Figure 17** shows anti-Spkl IgG NAb levels (ng/mL) in murine plasma pre- and post-IdeS infusion. Negative control animals were not treated with either IVIg or IdeS. IdeS Low refers to 0.4 mg/kg IdeS used in the study, and IdeS High refers to 4 mg/kg IdeS.

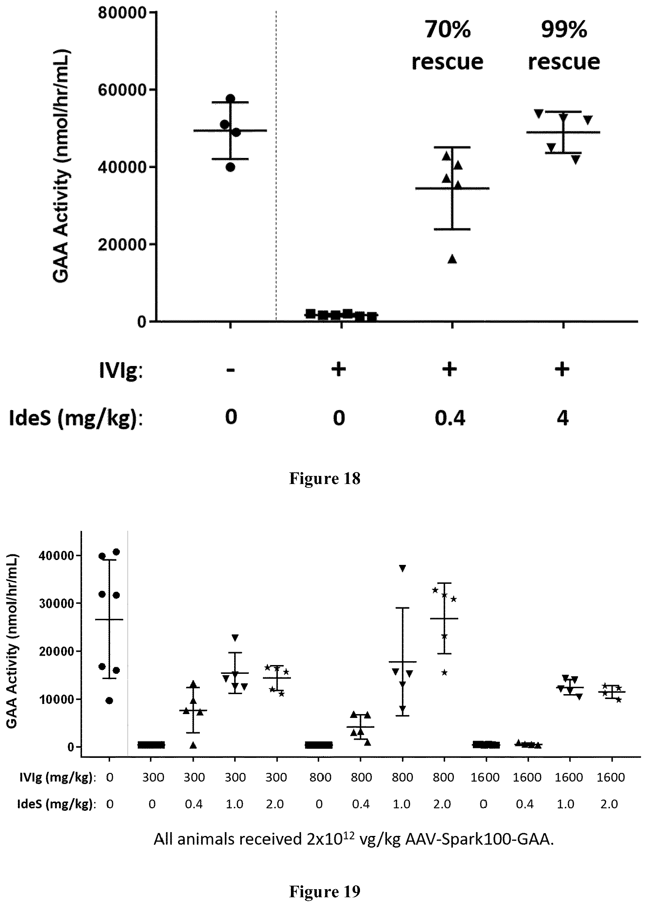

**Figure 18** shows GAA activity levels (nmol/hr/mL) in murine plasma after infusion of AAV-Spk1-GAA vector in animals immunized with IVIg then treated with IdeS. All animals received 2×10¹² vg/kg AAV-Spk1-GAA vector. Transgene activity was measured in plasma samples from mice immunized with IVIg, then treated with or without IdeS, and finally administered with vector. Transgene activity was assessed by GAA Activity Assay at 1 week post vector administration. GAA activity in nmol/hr/mL is plotted for each mouse in each group.
**Figure 19** shows GAA activity levels (nmol/hr/mL) two weeks after infusion of AAV-Spk1-GAA vector (2 × 10¹² vg/kg) in animals previously infused with IVIg (0, 300, 800, or 1600 mg/kg) and treated with IdeS (0, 0.4, 1.0 or 2.0 mg/kg). GAA activity is plotted for each mouse in each group. Mice in IVIg administered groups that did not develop a corresponding anti-Spkl NAb titer *(i.e.,* having NAb titer <1:1 or 1:1-1:2.5 pre-IdeS treatment) were excluded.

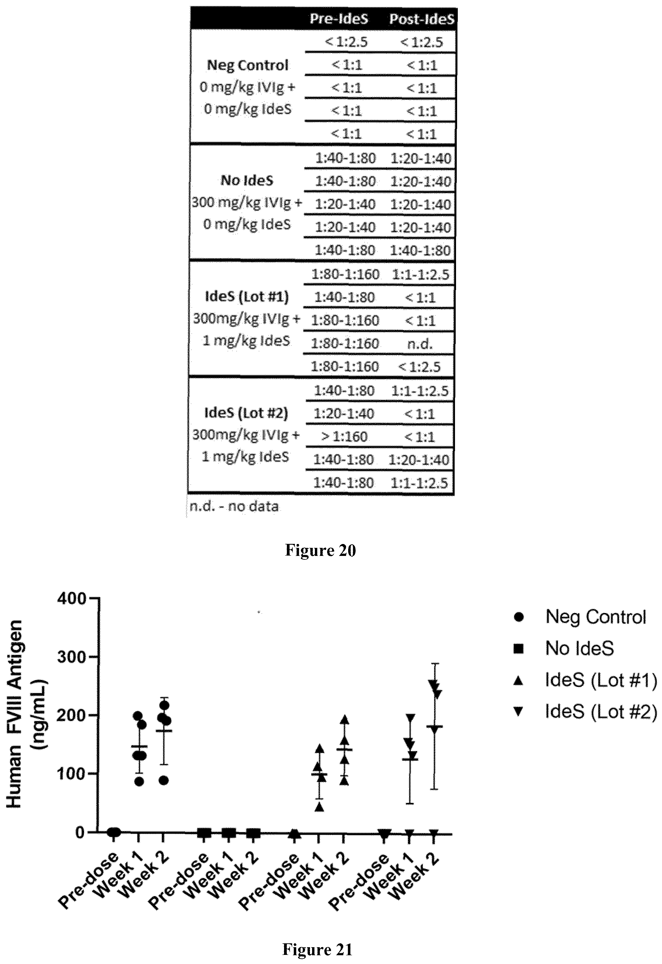

**Figure 20** shows anti-Spkl NAb titer levels in the plasma of C57BL/6 mice, having an artificial titer of human anti-capsid neutralizing IgG, measured pre- and post-infusion with different preparations of IdeS (Lot #1 and Lot #2).
**Figure 21** is a graph showing levels of human Factor VIII in plasma from C57BL/6 mice, having an artificial titer of human anti-capsid neutralizing IgG, pre-dose and at 1 and 2 weeks after dosing with AAV-Spk1-hFVIII.

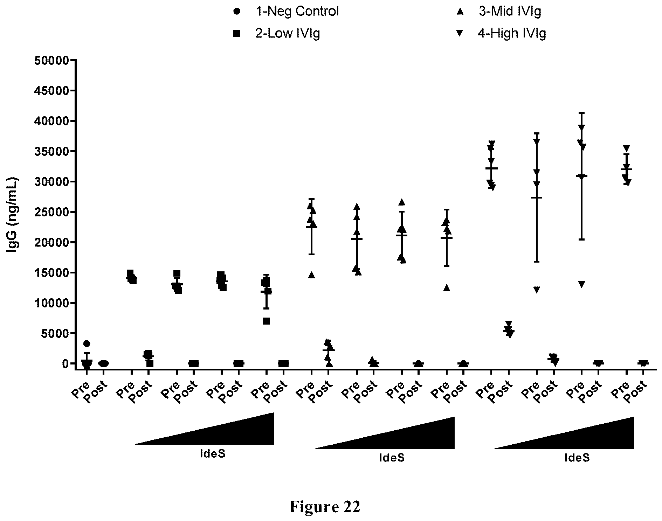

**Figure 22** is a graph showing anti-Spkl capsid IgG levels (ng/mL) in mouse plasma pre- and post-IdeS infusion. C57BL/6 mice were given IVIg to induce an artificial titer of human anti-capsid neutralizing IgG. Negative control animals were not treated with either IVIg or IdeS. Low IVIg refers to 300 mg/kg IVIg used in the study, Mid IVIg refers to 800 mg/kg, and High IVIg refers to 1600 mg/kg. Within each IVIg group, animals were treated with increasing doses of IdeS (0, 0.4, 1.0, 2.0 mg/kg IdeS). Animals treated with IVIg but demonstrating no anti-capsid IgG response were excluded from the graph.

### Detailed Description

Provided herein are methods to improve the benefit or effectiveness of gene therapy comprising administration of an agent that degrades or digests antibodies and/or inhibits or reduces effector function of antibodies. Also provided herein are methods to degrade or digest antibodies that bind to a viral vector, such as a recombinant viral vector, and/or degrade or digest antibodies that bind to a nucleic acid or a protein or peptide encoded by a heterologous polynucleotide encapsidated by a recombinant viral vector and/or inhibit or reduce effector function of antibodies that bind to a recombinant viral vector, and/or a nucleic acid or a protein or peptide encoded by a heterologous polynucleotide encapsidated by the recombinant viral vector. Also provided herein are methods to re-dose or re-administer a gene therapy vector to a subject to whom a gene therapy vector was previously administered, and wherein the subject has developed antibodies that bind and/or neutralize the gene therapy vector.

In certain embodiments, a method comprises administering to a subject an amount of a protease effective to degrade or digest antibodies or a glycosidase effective to inhibit or reduce effector function of antibodies that bind to a recombinant viral vector, and/or a nucleic acid, and/or a protein or peptide encoded by a heterologous polynucleotide. In certain embodiments, a method comprises administering to a subject an amount of an endopeptidase effective to degrade or digest antibodies or an endoglycosidase effective to inhibit or reduce effector function of antibodies that bind to a recombinant viral vector, and/or a nucleic acid, and/or a protein or peptide encoded by a heterologous polynucleotide.

In certain embodiments, a method comprises administering to a subject an amount of a glycosidase effective to reduce Fc receptor binding of antibodies that bind to a recombinant viral vector, and/or a nucleic acid, and/or a protein or peptide encoded by a heterologous polynucleotide. In certain embodiments, a method comprises administering to a subject an amount of an endoglycosidase effective to reduce Fc receptor binding of antibodies that bind to a recombinant viral vector, and/or a nucleic acid, and/or a protein or peptide encoded by a heterologous polynucleotide.

In certain embodiments, the instant invention relates to an immunoglobulin G-degrading enzyme poleptide for use in the treatment of a disease treated by gene therapy using a vector in a patient in need thereof.

In certain embodiments, the instant invention relates to an i) immunoglobulin G-degrading enzyme polypeptide, and ii) a vector, as a combined preparation for simultaneous, separate or sequential use in the treatment of a disease treated by gene therapy using said vector in a patient in need thereof.

In certain embodiments, the instant invention relates to a combination of an immunoglobulin G-degrading enzyme polypeptide and a vector, for use in the treatment of a disease treated by gene therapy using said vector in a patient in need thereof.

In certain embodiments, the immunoglobulin G-degrading enzyme polypeptide is administered before, simultaneously (concomitantly) with, or after said vector.

As used herein the term "a disease treated by gene therapy using a vector" denotes a disease wherein a polynucleotide (encoding at least one polypeptide or inhibitory nucleic acid) is delivered into the cell(s) of a patient as a drug to treat said disease (gene therapy).

In certain embodiments, the vector encodes at least one specific polypeptide or inhibitory nucleic acid useful to treat a disease. In certain embodiments, the vector encodes/comprises a therapeutic polynucleotide appropriate for treating a disease.

In certain embodiments, the vector encodes/comprises a therapeutic polynucleotide appropriate for treating a disease using gene therapy.

In certain embodiments, the patient is a vector-seropositive patient.

In certain embodiments, the patient is a vector-seronegative patient.

As used herein, the term "immunoglobulin G-degrading enzyme polypeptide" denotes a polypeptide which is a protease which degrades immunoglobulin G. In certain embodiments, the immunoglobulin G-degrading enzyme polypeptide is papain, pepsin or the immunoglobulin G-degrading enzyme polypeptide of *S. pyogenes* (IdeS), or variants from other bacterial or microbial organisms, or engineered versions thereof. In certain embodiments, the immunoglobulin G-degrading enzyme polypeptide specifically degrades immunoglobulin G. In certain embodiments, the immunoglobulin G-degrading enzyme polypeptide comprises a sequence selected from any of SEQ ID NOs:3-43 or 48.

As used herein, the terms "patient" and "subject" interchangeably refer to an animal, typically a mammal, such as a rodent, a feline, a canine, and a primate. Particularly, a subject or patient according to the instant invention is a human.

As used herein, the term "treatment" or "treat" refers to both prophylactic or preventive treatment like gene therapy as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment.

Further provided herein is a composition provided, for example, as a package or kit, having (a) a recombinant viral vector comprising a heterologous polynucleotide that encodes a protein or peptide or a heterologous polynucleotide; (b) a protease or glycosidase that degrades or digests antibodies; and (c) a label with instructions for performing the method described herein, wherein (a) and (b) are in separate or the same container. In certain embodiments, viral vectors that may be used in the instant include, for example and without limitation, adeno-associated viral (AAV) vectors. In certain embodiments, viral vectors that may be used in the instant invention include, for example and without limitation, retroviral, adenoviral, helper-dependent adenoviral, hybrid adenoviral, herpes simplex virus, lentiviral, poxvirus, Epstein-Barr virus, vaccinia virus, and human cytomegalovirus vectors, including recombinant versions thereof.

As used herein without limitation, the term "recombinant," as a modifier of a viral vector, such as a recombinant AAV (rAAV) vector, as well as a modifier of sequences such as recombinant polynucleotides and polypeptides, means that compositions have been manipulated *(i.e.,* engineered) in a fashion that generally does not occur in nature. A particular example of a recombinant AAV vector would be where a nucleic acid that is not normally present in a wild-type AAV genome (heterologous polynucleotide) is inserted within a viral genome. An example of which would be where a nucleic acid *(e.g.,* gene) encoding a therapeutic protein or polynucleotide sequence is cloned into a vector, with or without 5', 3' and/or intron regions that the gene is normally associated within the AAV genome. Although the term "recombinant" is not always used herein in reference to an AAV vector, as well as sequences such as polynucleotides, recombinant forms including AAV vectors, polynucleotides, etc., are expressly included in spite of any such omission.

A "rAAV vector," for example, is derived from a wild type genome of AAV by using molecular methods to remove all or a part of a wild type AAV genome, and replacing with a non-native (heterologous) nucleic acid, such as a nucleic acid encoding a therapeutic protein or polynucleotide sequence. Typically, for a rAAV vector one or both inverted terminal repeat (ITR) sequences of AAV genome are retained. A rAAV is distinguished from an AAV genome since all or a part of an AAV genome has been replaced with a non-native sequence with respect to the AAV genomic nucleic acid, such as with a heterologous nucleic acid encoding a therapeutic protein or polynucleotide sequence. Incorporation of a non-native (heterologous) sequence therefore defines an AAV as a "recombinant" AAV vector, which can be referred to as a "rAAV vector."

A recombinant AAV vector sequence can be packaged- referred to herein as a "particle" for subsequent infection (transduction) of a cell, *ex vivo, in vitro* or *in vivo.* Where a recombinant vector sequence is encapsidated or packaged into an AAV particle, the particle can also be referred to as a "rAAV", "rAAV particle" and/or "rAAV virion." Such rAAV, rAAV particles and rAAV virions include proteins that encapsidate or package a vector genome. Particular examples include in the case of AAV, capsid proteins.

A "vector genome", which may be abbreviated as "vg", refers to the portion of the recombinant plasmid sequence that is ultimately packaged or encapsidated to form a rAAV particle. In cases where recombinant plasmids are used to construct or manufacture recombinant AAV vectors, the AAV vector genome does not include the portion of the "plasmid" that does not correspond to the vector genome sequence of the recombinant plasmid. This non-vector genome portion of the recombinant plasmid is referred to as the "plasmid backbone," which is important for cloning and amplification of the plasmid, a process that is needed for propagation and recombinant AAV vector production, but is not itself packaged or encapsidated into rAAV particles. Thus, a "vector genome" refers to the nucleic acid that is packaged or encapsidated by rAAV.

As used herein, the term "serotype" in reference to an AAV vector means a capsid that is serologically distinct from other AAV serotypes. Serologic distinctiveness is determined on the basis of lack of cross-reactivity between antibodies to one AAV as compared to another AAV. Cross-reactivity differences are usually due to differences in capsid protein sequences/antigenic determinants (*e.g.,* due to VP1, VP2, and/or VP3 sequence differences of AAV serotypes). An antibody to one AAV may cross-react with one or more other AAV serotypes due to homology of capsid protein sequence.

Under the traditional definition, a serotype means that the virus of interest has been tested against serum specific for all existing and characterized serotypes for neutralizing activity and no antibodies have been found that neutralize the virus of interest. As more naturally occurring virus isolates are discovered and/or capsid mutants generated, there may or may not be serological differences with any of the currently existing serotypes. Thus, in cases where the new virus (*e.g.,* AAV) has no serological difference, this new virus (*e.g.,* AAV) would be a subgroup or variant of the corresponding serotype. In many cases, serology testing for neutralizing activity has yet to be performed on mutant viruses with capsid sequence modifications to determine if they are of another serotype according to the traditional definition of serotype. Accordingly, for the sake of convenience and to avoid repetition, the term "serotype" broadly refers to both serologically distinct viruses *(e.g.,* AAV) as well as viruses *(e.g.,* AAV) that are not serologically distinct that may be within a subgroup or a variant of a given serotype.

rAAV vectors include any viral strain or serotype. For example and without limitation, a rAAV vector genome or particle (capsid, such as VP1, VP2 and/or VP3) can be based upon any AAV serotype, such as AAV-1, -2, -3, -4, -5, -6, -7, -8, -9, -10, -11, -12, -rh74, -rh10 or AAV-2i8, for example. Such vectors can be based on the same strain or serotype (or subgroup or variant), or be different from each other. For example and without limitation, a rAAV plasmid or vector genome or particle (capsid) based upon one serotype genome can be identical to one or more of the capsid proteins that package the vector. In addition, a rAAV plasmid or vector genome can be based upon an AAV serotype genome distinct from one or more of the capsid proteins that package the vector genome, in which case at least one of the three capsid proteins could be a different AAV serotype, *e.g*., AAV1, AAV2, AAV3, AAV4, AAV5, AAV6, AAV7, AAV8, AAV9, AAV10, AAV11, AAV12, -rh74, -rh10, AAV-2i8, SPK1 (SEQ ID NO:1), SPK2 (SEQ ID NO:2), or variant thereof, for example. More specifically, a rAAV2 vector genome can comprise AAV2 ITRs but capsids from a different serotype, such as AAV1, AAV3, AAV4, AAV5, AAV6, AAV7, AAV8, AAV9, AAV10, AAV11, AAV12, -rh74, -rh10, AAV-2i8, SPK1 (SEQ ID NO:1), SPK2 (SEQ ID NO:2), or variant thereof, for example. Accordingly, rAAV vectors include gene/protein sequences identical to gene/protein sequences characteristic for a particular serotype, as well as "mixed" serotypes, which also can be referred to as "pseudotypes."

In certain embodiments, the vector is an AAV vector, a lentiviral vector or adenovirus vector.

In certain embodiments, the term vector also includes a recombinant vector which induces neutralizing immunoglobulin G after its first administration.

In certain embodiments, the vector is an AAV vector. In certain embodiments, the vector is an AAV8 vector encoding human FIX (comprising a therapeutic polynucleotide encoding human FIX). In certain embodiments, the vector is an AAV8 vector encoding human FIX, useful for the treatment of hemophilia.

In certain embodiments, the rAAV plasmid or vector genome or particle is based upon reptile or invertebrate AAV variants, such as snake and lizard parvovirus (Pénzes et al., 2015, J. Gen. Virol., 96:2769-2779) or insect and shrimp parvovirus (Roekring et al., 2002, Virus Res., 87:79-87).

In certain embodiments, the recombinant plasmid or vector genome or particle is based upon a bocavirus variant. Human bocavirus variants are described, for example, in Guido et al., 2016, World J. Gastroenterol., 22: 8684-8697.

In certain embodiments, a rAAV vector includes or consists of a capsid sequence at least 70% or more *(e.g.,* 75%, 80%, 85%, 90%, 95%, 96%, 97%, 98%, 99%, 99.5%, etc.) identical to one or more AAV1, AAV2, AAV3, AAV4, AAV5, AAV6, AAV7, AAV8, AAV9, AAV10, AAV11, AAV12, -rh74, -rh10 AAV-2i8, SPK1 (SEQ ID NO:1), SPK2 (SEQ ID NO:2) capsid proteins (VP1, VP2, and/or VP3 sequences). In certain embodiments, a rAAV vector includes or consists of a sequence at least 70% or more (*e.g.,* 75%, 80%, 85%, 90%, 95%, 96%, 97%, 98%, 99%, 99.5%, etc.) identical to one or more AAV1, AAV2, AAV3, AAV4, AAV5, AAV6, AAV7, AAV8, AAV9, AAV10, AAV11, AAV12, -rh74, -rh10 or AAV-2i8, ITR(s).

In certain embodiments, rAAV vectors include AAV1, AAV2, AAV3, AAV4, AAV5, AAV6, AAV7, AAV8, AAV9, AAV10, AAV11, AAV12, Rh10, Rh74 and AAV-2i8 variants *(e.g.,* ITR and capsid variants, such as amino acid insertions, additions, substitutions and deletions) thereof, for example, as set forth in WO 2013/158879 (International Application PCT/US2013/037170), WO 2015/013313 (International Application PCT/US2014/047670) and US 2013/0059732 (U.S. Patent Application No. 13/594,773).

rAAV, such as AAV1, AAV2, AAV3, AAV4, AAV5, AAV6, AAV7, AAV8, AAV9, AAV10, AAV11, AAV12, -rh74, -rh10, AAV-2i8, SPK1 (SEQ ID NO:1), SPK2 (SEQ ID NO:2) and variants, hybrids and chimeric sequences, can be constructed using recombinant techniques that are known to a skilled artisan, to include one or more heterologous polynucleotide sequences (transgenes) flanked with one or more functional AAV ITR sequences. Such AAV vectors typically retain at least one functional flanking ITR sequence(s), as necessary for the rescue, replication, and packaging of the recombinant vector into a rAAV vector particle. A rAAV vector genome would therefore include sequences required in cis for replication and packaging (*e.g.,* functional ITR sequences).In certain embodiments, a lentivirus used in the instant invention may be a human immunodeficiency-1 (HIV-1), human immunodeficiency-2 (HIV-2), simian immunodeficiency virus (SIV), feline immunodeficiency virus (FIV), bovine immunodeficiency virus (BIV), Jembrana Disease Virus (JDV), equine infectious anemia virus (EIAV), or caprine arthritis encephalitis virus (CAEV). Lentiviral vectors are capable of providing efficient delivery, integration and long-term expression of heterologous polynucleotide sequences into non-dividing cells both *in vitro* and *in vivo.* A variety of lentiviral vectors are known in the art, see Naldini et al. (Proc. Natl. Acad. Sci. USA, 93:11382-11388 (1996); Science, 272: 263-267 (1996)), Zufferey et al., (Nat. Biotechnol., 15:871-875, 1997), Dull et al., (J Virol. 1998 Nov;72(11):8463-71, 1998), U.S. Pat. Nos. 6,013,516 and 5,994,136, any of which may be a suitable viral vector for use in the instant invention. An immune response, such as humoral immunity, can develop against a wildtype virus in a subject exposed to the wildtype virus. Such exposure can lead to pre-existing antibodies in the subject that bind to a viral vector based upon the wildtype virus even prior to treatment with a gene therapy method employing the viral vector.

As used herein, the term "AAV vector" has its general meaning in the art and denotes a vector derived from an adeno-associated virus serotype, including without limitation AAV1, AAV2, variants AAV2, AAV3, variant AAV3, AAV3B, variant AAV3B, AAV4, AAV5, AAV6, variant AAV6, AAV7, AAV8, AAV9, AAV10 such as AAVcy10 and AAVrh10, AAVrh74, AAVDJ, AAV-Anc80 ; AAV-LK03, AAV2i8 and porcine AAV, such as AAVpo4 and AAVpo6 capsid or with a chimeric capsid or any other naturally occurring serotypes of AAV that can infect humans, monkeys or other species, and all other serotypes engineered by shuffling, peptide insertion, error-prone PCR, rational design, or using machine learning and other techniques like AAVLK03, AAVDJ, Anc80, AAV2i8, AAV-PHP-B. AAV vectors can have one or more of the AAV wild-type genes deleted in whole or part, preferably the *rep* and/or *cap* genes, but retain functional flanking inverted terminal repeat (ITR) sequences. Functional ITR sequences are necessary for the rescue, replication and packaging of the AAV virion. Thus, an AAV vector is defined herein to include at least those sequences required in cis for replication and packaging (*e.g.,* functional ITRs) of the virus. The ITRs need not be the wild-type nucleotide sequences, and may be altered, *e.g.,* by the insertion, deletion or substitution of nucleotides, so long as the sequences provide for functional rescue, replication and packaging. AAV expression vectors are constructed using known techniques to at least provide as operatively linked components in the direction of transcription, control elements including a transcriptional initiation region, the nucleic acid molecule of the instant invention and a transcriptional termination region. The control elements are selected to be functional in a mammalian cell. The resulting construct which contains the operatively linked components is bounded (5' and 3') with functional AAV ITR sequences.

By "adeno-associated virus inverted terminal repeats " or "AAV ITRs" is meant the art-recognized regions found at each end of the AAV genome which function together in cis as origins of DNA replication and as packaging signals for the virus. AAV ITRs, together with the AAV rep coding region, provide for the efficient excision and rescue from, and integration of a nucleotide sequence interposed between two flanking ITRs into a mammalian cell genome. The nucleotide sequences of AAV ITR regions are known. See, *e.g.,* Kotin, 1994; Berns, KI "Parvoviridae and their Replication" in Fundamental Virology, 2nd Edition, (B. N. Fields and D. M. Knipe, eds.) for the AAV-2 sequence. As used herein, an "AAV ITR" does not necessarily comprise the wild-type nucleotide sequence, but may be altered, *e.g.,* by the insertion, deletion or substitution of nucleotides. Additionally, the AAV ITR may be derived from any of several AAV serotypes, including without limitation, AAV-1, AAV-2, AAV-3, AAV-4, AAV-5, AAV-6, etc. Furthermore, 5' and 3' ITRs which flank a selected nucleotide sequence in an AAV vector need not necessarily be identical or derived from the same AAV serotype or isolate, so long as they function as intended, *i.e.,* to allow for excision and rescue of the sequence of interest from a host cell genome or vector, and to allow integration of the heterologous sequence into the recipient cell genome when AAV Rep gene products are present in the cell. In certain embodiments, the AAV vector of the instant invention is selected from vectors derived from AAV serotypes having tropism for and high transduction efficiencies in cells of the mammalian central and peripheral nervous system, particularly neurons, neuronal progenitors, astrocytes, oligodendrocytes and glial cells. In certain embodiments, the AAV vector is an AAV4, AAV9 or an AAVrh10 that have been described to well transduce brain cells especially neurons. In certain embodiments, the AAV vector of the instant invention is a double-stranded, self-complementary AAV (scAAV) vector. As an alternative to the use of single-stranded AAV vector, self-complementary vectors can be used. The efficiency of an AAV vector in terms of the number of genome-containing particles required for transduction, is hindered by the need to convert the single-stranded DNA (ssDNA) genome into double-stranded DNA (dsDNA) prior to expression. This step can be circumvented through the use of self-complementary vectors, which package an inverted repeat genome that can fold into dsDNA without the requirement for DNA synthesis or base-pairing between multiple vector genomes. Resulting self-complementary AAV (scAAV) vectors have increased resulting expression of the transgene. For an overview of AAV biology, ITR function, and scAAV constructs, see McCarty D M. Self-complementary AAV vectors; advances and applications. Mol. Ther. 2008 October; 16 (10): at pages 1648-51, first full paragraph, incorporated herein by reference for disclosure of AAV and scAAV constructs, ITR function, and role of ΔTRS ITR in scAAV constructs. A rAAV vector comprising a ΔTRS ITR cannot correctly be nicked during the replication cycle and, accordingly, produces a self-complementary, double-stranded AAV (scAAV) genome, which can efficiently be packaged into infectious AAV particles. Various rAAV, ssAAV, and scAAV vectors, as well as the advantages and drawbacks of each class of vector for specific applications and methods of using such vectors in gene transfer applications are well known to those of skill in the art (see, for example, Choi et al., 2005, J. Virol., 79(11):6801-7; McCarty et al., 2004, Annu Rev Genet., 38:819-45; McCarty et al., 2001, Gene Ther., 8(16):1248-54; and McCarty 2008, Mol. Ther., 16(10):1648-56; all references cited are incorporated herein by reference for disclosure of AAV, rAAV, and scAAV vectors).

An AAV vector of the instant invention can be constructed by directly inserting the selected sequence (s) into an AAV genome which has had the major AAV open reading frames ("ORFs") excised therefrom. Other portions of the AAV genome can also be deleted, so long as a sufficient portion of the ITRs remain to allow for replication and packaging functions. Such constructs can be designed using techniques well known in the art. See, *e.g.,* U.S. Patents Nos. 5,173, 414 and 5,139, 941; International Publications Nos. WO 92/01070 and WO 93/03769. Alternatively, AAV ITRs can be excised from the viral genome or from an AAV vector containing the same and fused 5' and 3' of a selected nucleic acid construct that is present in another vector using standard ligation techniques. AAV vectors which contain ITRs have been described in, *e.g.,* U.S. Patent No. 5,139, 941. In particular, several AAV vectors are described therein which are available from the American Type Culture Collection ("ATCC") under Accession Numbers 53222, 53223, 53224, 53225 and 53226. Additionally, chimeric genes can be produced synthetically to include AAV ITR sequences arranged 5' and 3' of one or more selected nucleic acid sequences. Preferred codons for expression of the chimeric gene sequence in mammalian CNS and PNS cells can be used. The complete chimeric sequence is assembled from overlapping oligonucleotides prepared by standard methods. In order to produce AAV virions, an AAV expression vector is introduced into a suitable host cell using known techniques, such as by transfection. A number of transfection techniques are generally known in the art. See, *e.g.* , Graham *et al.,* 1973; Sambrook et al. (1989) Molecular Cloning, a laboratory manual, Cold Spring Harbor Laboratories, New York, Davis et al. (1986) Basic Methods in Molecular Biology, Elsevier, and Chu et al., 1981. Particularly suitable transfection methods include calcium phosphate co-precipitation (Graham *et al.,* 1973), direct microinjection into cultured cells (Capecchi, 1980), electroporation (Shigekawa *et al.,* 1988), liposome mediated gene transfer (Mannino *et al.,* 1988), lipid-mediated transduction (Feigner *et al.,* 1987), and nucleic acid delivery using high-velocity microprojectiles (Klein *et al.,* 1987).

Typically, a vector of the instant invention comprises an expression cassette. The term "expression cassette", as used herein, refers to a nucleic acid construct comprising nucleic acid elements sufficient for the expression of the nucleic acid molecule of the instant invention. Typically, an expression cassette comprises the nucleic acid molecule of the instant invention operatively linked to a promoter sequence. The term "operatively linked" refers to the association of two or more nucleic acid fragments on a single nucleic acid fragment so that the function of one is affected by the other. For example, a promoter is operatively linked with a coding sequence when it is capable of affecting the expression of that coding sequence (*e.g.,* the coding sequence is under the transcriptional control of the promoter). Encoding sequences can be operatively linked to regulatory sequences in sense or antisense orientation. In certain embodiments, the promoter is a heterologous promoter. The term "heterologous promoter", as used herein, refers to a promoter that is not found to be operatively linked to a given encoding sequence in nature. In certain embodiments, an expression cassette may comprise additional elements, for example, an intron, an enhancer, a polyadenylation site, a woodchuck response element (WRE), and/or other elements known to affect expression levels of the encoding sequence. As used herein, the term "promoter" refers to a nucleotide sequence capable of controlling the expression of a coding sequence or functional RNA. In general, the nucleic acid molecule of the instant invention is located 3' of a promoter sequence. In certain embodiments, a promoter sequence consists of proximal and more distal upstream elements and can comprise an enhancer element. An "enhancer" is a nucleotide sequence that can stimulate promoter activity and may be an innate element of the promoter or a heterologous element inserted to enhance the level or tissue-specificity of a promoter. In certain embodiments, the promoter is derived in its entirety from a native gene. In certain embodiments, the promoter is composed of different elements derived from different naturally occurring promoters. In certain embodiments, the promoter comprises a synthetic nucleotide sequence. It will be understood by those skilled in the art that different promoters will direct the expression of a gene in different tissues or cell types, or at different stages of development, or in response to different environmental conditions or to the presence or the absence of a drug or transcriptional co-factor. Ubiquitous, cell-type-specific, tissue-specific, developmental stage-specific, and conditional promoters, for example, drug-responsive promoters (*e.g.,* tetracycline-responsive promoters) are well known to those of skill in the art. Examples of promoter include, but are not limited to, the phosphoglycerate kinase (PKG) promoter, CAG (composite of the CMV enhancer the chicken beta actin promoter (CBA) and the rabbit beta globin intron.), NSE (neuronal specific enolase), synapsin or NeuN promoters, the SV40 early promoter, mouse mammary tumor virus LTR promoter; adenovirus major late promoter (Ad MLP); a herpes simplex virus (HSV) promoter, a cytomegalovirus (CMV) promoter such as the CMV immediate early promoter region (CMVIE), SFFV promoter, rous sarcoma virus (RSV) promoter, synthetic promoters, hybrid promoters, and the like. Other promoters can be of human origin or from other species, including from mice. Common promoters include, *e.g.,* the human cytomegalovirus (CMV) immediate early gene promoter, the SV40 early promoter, the Rous sarcoma virus long terminal repeat, [beta]-actin, rat insulin promoter, the phosphoglycerate kinase promoter, the human alpha-1 antitrypsin (hAAT) promoter, the transthyretin promoter, the TBG promoter and other liver-specific promoters, the desmin promoter and similar muscle-specific promoters, the EF1-alpha promoter, the CAG promoter and other constitutive promoters, hybrid promoters with multi-tissue specificity, promoters specific for neurons like synapsin and glyceraldehyde-3-phosphate dehydrogenase promoter, all of which are promoters well known and readily available to those of skill in the art, can be used to obtain high-level expression of the coding sequence of interest. In addition, sequences derived from non-viral genes, such as the murine metallothionein gene, will also find use herein. Such promoter sequences are commercially available from, *e.g*., Stratagene (San Diego, CA).

In certain embodiments, the expression cassette comprises an appropriate secretory signal sequence that will allow the secretion of the polypeptide encoded by the nucleic acid molecule of the instant invention. As used herein, the term "secretory signal sequence" or variations thereof are intended to refer to amino acid sequences that function to enhance (as defined above) secretion of an operably linked polypeptide from the cell as compared with the level of secretion seen with the native polypeptide. As defined above, by "enhanced" secretion, it is meant that the relative proportion of the polypeptide synthesized by the cell that is secreted from the cell is increased; it is not necessary that the absolute amount of secreted protein is also increased. In certain embodiments, essentially all *(i.e.,* at least 95%, 97%, 98%, 99% or more) of the polypeptide is secreted. It is not necessary, however, that essentially all or even most of the polypeptide is secreted, as long as the level of secretion is enhanced as compared with the native polypeptide. Generally, secretory signal sequences are cleaved within the endoplasmic reticulum and, in certain embodiments, the secretory signal sequence is cleaved prior to secretion. It is not necessary, however, that the secretory signal sequence is cleaved as long as secretion of the polypeptide from the cell is enhanced and the polypeptide is functional. Thus, in certain embodiments, the secretory signal sequence is partially or entirely retained. The secretory signal sequence can be derived in whole or in part from the secretory signal of a secreted polypeptide *(i.e.,* from the precursor) and/or can be in whole or in part synthetic. The length of the secretory signal sequence is not critical; generally, known secretory signal sequences are from about 10-15 to 50-60 amino acids in length. Further, known secretory signals from secreted polypeptides can be altered or modified (*e.g.,* by substitution, deletion, truncation or insertion of amino acids) as long as the resulting secretory signal sequence functions to enhance secretion of an operably linked polypeptide. The secretory signal sequences of the instant invention can comprise, consist essentially of or consist of a naturally occurring secretory signal sequence or a modification thereof (as described above). Numerous secreted proteins and sequences that direct secretion from the cell are known in the art. The secretory signal sequence of the instant invention can further be in whole or in part synthetic or artificial. Synthetic or artificial secretory signal peptides are known in the art, see *e.g*., Barash et al., Biochem. Biophys. Res. Comm. 294:835-42 (2002).

In certain embodiments, the instant invention includes a method of treating a disease by gene therapy using a vector, comprising administering to a subject in need thereof a therapeutically effective amount of an immunoglobulin G-degrading enzyme polypeptide.

### Nucleic acids, vectors, recombinant host cells and uses thereof

In certain embodiments, the instant invention relates to a nucleic acid sequence encoding an immunoglobulin G-degrading enzyme polypeptide for use in the treatment of a disease treated by gene therapy using an vector in a patient in need thereof.

In certain embodiments, the instant invention includes i) a nucleic acid sequence encoding an immunoglobulin G-degrading enzyme polypeptide, and ii) a vector, as a combined preparation for simultaneous, separate or sequential use in the treatment of a disease treated by gene therapy using said vector in a patient in need thereof.

In certain embodiments, the instant invention includes a combination of a nucleic acid sequence encoding an immunoglobulin G-degrading enzyme polypeptide and an vector for use the treatment of a disease treated by gene therapy using said vector in a patient in need thereof.

In certain embodiments, the instant invention includes a nucleic acid sequence encoding an IdeS polypeptide of SEQ I D NO:4 or a function-conservative variant thereof as described herein.

In certain embodiments, the nucleic acid sequence encoding an IdeS polypeptide of SEQ ID NO:4 comprises or consists of SEQ ID NO:52.

In certain embodiments, the nucleic acid sequence comprises at least 60%, 65%, 70%, 75%, 80%, 85%, 90%, 95%, 96%, 97%, 98%, 99%, 99.5% or 99.9% identity to the sequence of SEQ ID NO:52.

In certain embodiments, the mRNA of the nucleic acids of the instant invention can be used.

Nucleic acids of the instant invention may be produced by any technique known per se in the art, such as, without limitation, any chemical, biological, genetic or enzymatic technique, either alone or in combination(s).

In certain embodiments, the instant invention includes an expression vector comprising a nucleic acid sequence encoding an immunoglobulin G-degrading enzyme polypeptide for use in the treatment of a disease treated by gene therapy using an vector in a patient in need thereof.

In certain embodiments, the expression vector comprises a nucleic acid sequence encoding an amino sequence comprising SEQ ID NO:52 or a function-conservative variant thereof as described here above.

In certain embodiments, the vector which comprises SEQ ID NO:52 or a function-conservative variant thereof is not an AAV vector.

Expression vectors which may be used in the instant invention may comprise at least one expression control element operationally linked to the nucleic acid sequence. The expression control elements are inserted in the vector to control and regulate the expression of the nucleic acid sequence. Examples of expression control elements include, but are not limited to, lac system, operator and promoter regions of phage lambda, yeast promoters and promoters derived from polyomavirus, adenovirus, retrovirus, lentivirus or SV40. Additional preferred or required operational elements include, but are not limited to, leader sequence, termination codons, polyadenylation signals and any other sequences necessary or preferred for the appropriate transcription and subsequent translation of the nucleic acid sequence in the host system. It will be understood by one skilled in the art that the correct combination of required or preferred expression control elements will depend on the host system chosen. It will further be understood that the expression vector should contain additional elements necessary for the transfer and subsequent replication of the expression vector containing the nucleic acid sequence in the host system. Examples of such elements include, but are not limited to, origins of replication and selectable markers. It will further be understood by one skilled in the art that such vectors are easily constructed using conventional methods or commercially available.

In certain embodiments, the instant invention includes a host cell comprising an expression vector as described here above.

In certain embodiments, host cells may be, for example and without limitation, eukaryote cells, such as animal, plant, insect and yeast cells and prokaryotes cells, such as E. coli. The means by which the vector carrying the gene may be introduced into the cells include, but are not limited to, microinjection, electroporation, transduction, or transfection using DEAE-dextran, lipofection, calcium phosphate or other procedures known to one skilled in the art.

In certain embodiments, eukaryotic expression vectors that function in eukaryotic cells are used. Examples of such vectors include, but are not limited to, viral vectors such as retrovirus, adenovirus, adeno-associated virus, herpes virus, vaccinia virus, poxvirus, poliovirus; lentivirus, bacterial expression vectors, plasmids, such as pcDNA3 or the baculovirus transfer vectors. Preferred eukaryotic cell lines include, but are not limited to, COS cells, CHO cells, HeLa cells, NIH/3T3 cells, 293 cells (ATCC# CRL1573), T2 cells, dendritic cells, or monocytes.

An immune response, such as humoral immunity can also develop against a recombinant viral vector, and/or a heterologous polynucleotide or a protein or peptide encoded by a heterologous polynucleotide encapsidated by the viral vector, resulting in inhibition or reduction in viral vector cell transduction, heterologous polynucleotide expression or function, or function or activity of the protein or peptide encoded by a heterologous polynucleotide in a subject to which the viral vector is administered.

Antibodies that bind to a viral vector used in the instant invention, such as a recombinant viral vector, which can be referred to as "neutralizing" antibodies, can reduce or inhibit cell transduction of viral vectors useful for gene therapy. As a result, while not being bound by theory, cell transduction is reduced or inhibited thereby reducing introduction of the viral packaged heterologous polynucleotide into cells and subsequent expression and, as appropriate, subsequent translation into a protein or peptide. Additionally, antibodies that bind to a heterologous polynucleotide or a protein or peptide encoded by a heterologous polynucleotide encapsidated by the viral vector can inhibit expression of a heterologous polynucleotide, function or activity of a heterologous polynucleotide or function or activity of a protein or peptide encoded by a heterologous polynucleotide.

Accordingly, antibodies can be present that bind to a recombinant viral vector (*e.g.,* AAV) and/or antibodies can be present that bind to a protein or peptide encoded by a heterologous polynucleotide in a subject. In addition, antibodies can be present that bind to a heterologous polynucleotide encapsidated by the recombinant viral vector.

Antibodies that bind to a recombinant viral vector (*e.g.,* AAV) or that bind to a protein or peptide encoded by a heterologous polynucleotide, should they be produced, can be degraded or digested by a protease as set forth herein. Antibodies that bind to a recombinant viral vector (*e.g.,* AAV) or that bind to a protein or peptide encoded by a heterologous polynucleotide, should they be produced, can also have their effector function reduced or inhibited as set forth herein.

As used herein, "effector function" in reference to an antibody means normal functional attributes of an antibody. Nonlimiting examples of antibody functional attributes include, for example, binding to an antigen; activation of the complement cascade (referred to as complement dependent cytotoxicity); binding to Fc receptor on effector cells, such as macrophages, monocytes, natural killer cells and eosinophils, to engage antibody - dependent cellular cytotoxicity (ADCC); and as a signal for ingestion of bound antigen/pathogen by immune cells such as phagocytes and dendritic cells. A reduction or inhibition of antibody effector function can therefore refer to any one or more of the foregoing nonlimiting functional attributes. Effector function assays are known in the art as well as described in WO2016012285, for example.

An "Fc receptor" refers to any Fc receptor. Particular nonlimiting examples of Fc receptors include Fc gamma immunoglobulin receptors (FcyRs) which are present on cells. In humans, FcyR refers to one, some, or all of the family of Fc receptors comprising FcγRI (CD64), FcγRIIA (CD32A), FcγRIIB (CD32B), FcγRIIIA (CD16a) and FcγRIIIB (CD16b). FcγR includes naturally occurring polymorphisms of FcyRI (CD64), FcyRIIA (CD32A), FcyRIIB (CD32B), FcγRIIIA (CD16a) and FcγRIIIB (CD16b).

In certain embodiments, antibody binding to a viral vector is reduced or inhibited by way of a protease.

In certain embodiments, antibody binding to Fc receptor on effector cells, such as macrophages, monocytes, natural killer cells or eosinophils, is reduced or inhibited by way of a glycosidase. In certain embodiments, an endoglycosidase hydrolyzes a glycan structure on the Fc interacting domain of an antibody. In certain embodiments, an endoglycosidase hydrolyzes a glycan structure on the Fc interacting domain of an IgG, such as the N-linked bi-antennary glycan at position Asn-297 (Kabat numbering).

In certain embodiments, antibody activation of complement cascade is reduced or inhibited by way of a protease.

In a certain embodiments, antibody stimulation or reduction of ingestion by immune cells such as phagocytes or dendritic cells, is reduced or inhibited by way of a protease or glycosidase.

In certain embodiments, a protease and/or glycosidase is administered to a subject before administration of a recombinant viral (*e.g.,* AAV) vector. In certain embodiments, a protease and/or glycosidase is administered to a subject after administration of a recombinant viral (*e.g.,* AAV) vector. In certain embodiments, a recombinant viral (*e.g.,* AAV) vector and a protease and/or glycosidase are administered substantially contemporaneously, or at about the same time.

Antibodies comprise any of IgG, IgM, IgA, IgD and/or IgE. Accordingly, the instant invention is directed to digesting, degrading or reducing or inhibiting effector function of any of one of these five classes of antibodies, any two of these five classes of antibodies, any three of these five classes of antibodies, any four of these five classes of antibodies or all five of these five classes of antibodies.

Levels of antibodies in a subject can be analyzed, measured or determined before and/or after administration of a recombinant viral vector. Levels of antibodies in a subject can also be analyzed, measured or determined before and/or after the administration of a protease or glycosidase. Levels of antibodies in a subject may also be analyzed or measured multiple times, before and/or after administration of a recombinant viral vector as well as before and/or after administration of a protease or glycosidase.

Effector function of antibodies in a subject can be analyzed, measured or determined before and/or after administration of a recombinant viral vector. Effector function of antibodies in a subject can also be analyzed, measured or determined before and/or after the administration of a protease or glycosidase. Effector function of antibodies in a subject may also be analyzed or measured multiple times, before and/or after administration of a recombinant viral vector as well as before and/or after administration of a protease or glycosidase.

An increase in the equilibrium binding constant corresponds to a decrease in the binding between IgG and an Fc receptor. Accordingly, a reduction in Fc receptor binding of an antibody as a consequence of glycosidase activity may result in an increase in the equilibrium binding constant for the IgG:FcR interaction. A reduction in Fc receptor binding of an antibody may result in an increase in the equilibrium binding constant for the IgG:FcR interaction by a factor of at least 1, at least 2, at least 3, or at least 4, or at least 5, or at least 6, or at least 7 or at least 8.

In certain embodiments, an immune response (*e.g.,* a humoral immune response) in a subject caused by exposure to wild-type virus is treated by administering an amount of a protease and/or glycosidase effective to degrade or digest antibodies that bind to a recombinant viral vector based upon the wildtype virus prior to administration of the recombinant viral vector to the subject.

In certain embodiments, an immune response (*e.g.,* a humoral immune response) caused by administration of a recombinant viral vector such as AAV is treated by administering an amount of a protease and/or glycosidase effective to degrade or digest antibodies that bind to the recombinant viral vector, or antibodies that bind to the heterologous polynucleotide or a protein or peptide encoded by the heterologous polynucleotide encapsidated by the viral vector.

In certain embodiments, administration of a recombinant viral vector to a subject is preceded by administration of a protease and/or glycosidase to inhibit or prevent an immune response (*e.g.,* a humoral immune response) against the recombinant viral vector or antibodies that bind to the heterologous polynucleotide or a protein or peptide encoded by the heterologous polynucleotide encapsidated by the viral vector.

In certain embodiments, a protease and/or glycosidase is administered to a subject before an immune response (*e.g.,* a humoral immune response), such as before development of neutralizing antibodies or development of antibodies that bind to the heterologous polynucleotide or a protein or peptide encoded by the heterologous polynucleotide encapsidated by the viral vector.

Proteases are enzymes that degrade or digest proteins. As used herein, proteases are also designated peptidases, proteinases, peptide hydrolases, or proteolytic enzymes.

Proteases that may be used in the instant invention can be subdivided into two broad groups based on their substrate-specificity. Proteases may be of the exo-type that hydrolyzes peptide bonds located towards the N-terminal end or the C-terminal end (exoprotease or exopeptidase). Nonlimiting examples of exoproteases or exopeptidases, include, for example, Flavozyme (Novozymes), ProteaAX (Amano), and Pancreatin from porcine pancreas.

Proteases that may be used in the instant invention may be of the endo-type that hydrolyzes peptide bonds internally in polypeptide chains (endoprotease or endopeptidase). Examples of endoproteases include, for example, IdeS, IdeZ, IgdE, IdeMC, trypsin, chymotrypsin, papain and pepsin.

Examples of proteases that may be used in the instant invention include, for example and without limitation, cysteine proteases from *Streptococcus pyogenes, Streptococcus equi, Mycoplasma canis, S. agalactiae,S. pseudoporcinus* or *Pseudomonas putida.* In certain embodiments, a protease includes endopeptidase IdeS from *Streptococcus pyogenes* or a modified variant thereof set forth in any of SEQ ID NO:3-18. In certain embodiments, the protease includes protease set forth in SEQ ID NO:19 or SEQ ID NO:20, or a modified variant thereof. In certain embodiments, the protease includes endopeptidase IdeZ from *Streptococcus equi,* or a modified variant thereof set forth in any of SEQ ID NOs:21-43.

Other proteases that may be used in the instant invention include, for example and without limitation, IgdE enzymes from S. *suis, S. porcinus, S. equi,* described in WO 2017/134274. Other proteases that may be used in the instant invention include, for example and without limitation, IdeMC and homologs described in WO 2018/093868. Other endopeptidases that may be used in the instant invention include, for example and without limitation, IdeZ with and without the N-terminal methionine and signal peptide and IdeS/IdeZ hybrid proteins described in WO 2016/128559. Other proteases that may be used in the instant invention include, for example and without limitation, proteases described in Jordan et al. (N Engl. J Med. 377;5, 2017), Lannergard and Guss (FEMS Microbiol Lett., 262(2006); 230-235) and Hulting et al., (FEMS Microbiol Lett., 298(2009), 44-50), for example.

Glycosidases are enzymes that hydrolyzes glycosidic bonds in complex sugars. There are generally two broad groups, namely exoglycosidases and endoglycosidases. Glycosidases cleave and thereby releases glycans/oligosaccharides from glycoproteins such as antibodies.

Exoglycosidases that may be used in the instant invention include, for example and without limitation, N-acetylglucosaminidase, fucosidase, galactosidase, glucosidase, mannosidase, neuraminidase, and xylosidase. Endoglycosidases that may be used in the instant invention include, for example and without limitation, EndoS, Endo D, endoglycosidase-H, Endo F1, Endo F2 and Endo F3.

Accordingly, in certain embodiments, a glycosidase comprises an endoglycosidase. In certain embodiments, a glycosidase comprises an exoglycosidase.

An example of an endoglycosidase includes, for example and without limitation, EndoS. In certain embodiments, an endoglycosidase comprises a sequence set forth in any one of SEQ ID NOs:44-47, or a modified variant thereof.

In certain embodiments, an EndoS polypeptide includes an EndoS polypeptide, a fragment of an EndoS polypeptide, a variant of an EndoS polypeptide, or a variant of a fragment of an EndoS polypeptide, provided that said polypeptide, fragment, variant or variant of fragment has immunoglobulin (Ig) endoglycosidase activity.

In certain embodiments, an EndoS polypeptide is *S. pyogenes* EndoS. A variant of an EndoS polypeptide may be an EndoS polypeptide from another organism, such as another bacterium. In certain embodiments, a bacterium is a Streptococcus, such as *Streptococcus equi, Streptococcus zooepidemicus* or *Streptococcus pyogenes.* Alternatively, the variant may be from *Corynebacterium pseudotuberculosis,* for example the CP40 protein; *Enterococcus faecalis,* for example the EndoE protein; or *Elizabethkingia meningoseptica* (formerly *Flavobacterium meningosepticum*)*,* for example the EndoF2 protein.

An EndoS polypeptide may comprise or consist of (a) the amino acid sequence of any one of SEQ ID NOs:44-47; or (b) a fragment of (a) having Ig endoglycosidase activity; or (c) a variant of (a) having at least 50% identity to the amino acid sequence of any one of SEQ ID NOs:44-47 and having Ig endoglycosidase activity; or (d) a variant of (b) having at least 50% identity to the corresponding portion of the amino acid sequence of any one of SEQ ID NOs:44-47 and having Ig endoglycosidase activity.

In certain embodiments, the variant polypeptide has at least about 60% or more identity (*e.g.,* 60-70%, 70-80% or 80-90% identity) to the amino acid sequence of any one of SEQ ID NOs:44-47, or a fragment thereof having Ig endoglycosidase activity. In certain embodiments, the variant polypeptide has 90-100% identity to the amino acid sequence of any one of SEQ ID NOs:44-47, or a fragment thereof having Ig endoglycosidase activity.

A protease may comprise or consist of (a) the amino acid sequence of any one of SEQ ID NOs:3-43 or 48; or (b) a fragment of (a) having protease activity; or (c) a variant of (a) having at least 50% identity to the amino acid sequence of any one of SEQ ID NOs:3-43 or 48 and having protease activity; or (d) a variant of (b) having at least 50% identity to the corresponding portion of the amino acid sequence of any one of SEQ ID NOs:3-43 or 48 and having protease activity. In certain embodiments, the variant polypeptide has at least about 60% or more identity *(e.g.,* 60-70%, 70-80% or 80-90% identity) to the amino acid sequence of any one of SEQ ID NOs:3-43 or 48, or a fragment thereof having protease activity. In certain embodiments, the variant polypeptide has 90-100% identity to the amino acid sequence of any one of SEQ ID NOs:3-43 or 48, or a fragment thereof having protease activity.

In certain embodiments, the protease or glycosidase is the devoid of its native signal sequence and has an additional N terminal methionine, such as SEQ ID NO:48 which is the mature form of IdeS of *S. pyogenes* (without the signal sequence, but having an added N terminal methionine). Any protease or glycosidase used in the methods of the invention can include an added N terminal methionine in place of the native signal sequence.

In certain embodiments, the protease or glycosidase has a non-native N terminal signal or leader peptide sequence in place of its native signal sequence. Any protease or glycosidase used in the methods of the invention can include a non-native N terminal signal sequence in place of the native signal sequence. In certain embodiments, the non-native N terminal signal sequence is selected from SEQ ID NOs:49-51, or function-conservative variants thereof.

In certain embodiments, the immunoglobulin G-degrading enzyme polypeptide is capable of degrading adenovirus-neutralizing antibodies present in a patient.

In certain embodiments, the immunoglobulin G-degrading enzyme polypeptide is capable of degrading AAV-neutralizing antibodies present in a patient.

In certain embodiments, the immunoglobulin G-degrading enzyme polypeptide is the immunoglobulin G-degrading enzyme of *S. pyogenes* (IdeS) polypeptide.

In certain embodiments, the immunoglobulin G-degrading enzyme polypeptide is the immunoglobulin G-degrading enzyme of *S. pyogenes* (IdeS) polypeptide, which is administered to degrade adenovirus-neutralizing antibodies present in a patient.

In certain embodiments, the immunoglobulin G-degrading enzyme polypeptide is the immunoglobulin G-degrading enzyme of *S. pyogenes* (IdeS) polypeptide, which is administered to degrade AAV-neutralizing antibodies present in a patient.

Thus, in certain embodiments the instant invention relates to the use of an immunoglobulin G-degrading enzyme of *S. pyogenes* (IdeS) polypeptide in the treatment of a disease treated by administering a gene therapy vector to a patient in need thereof.

Thus, in certain embodiments, the instant invention relates to the use of an immunoglobulin G-degrading enzyme of *S. pyogenes* (IdeS) polypeptide in the treatment of a disease treated by administering a gene therapy vector to a patient in need thereof, wherein the IdeS is administered to the patient in order to degrade neutralizing anti-AAV antibodies present in the patient.

In certain embodiments, the patient is a vector-seropositive patient, and the instant invention relates to an immunoglobulin G-degrading enzyme of *S. pyogenes* (IdeS) polypeptide for use in the treatment of AAV-seropositive patients.

In certain embodiments, the patient is a vector-seronegative patient, and the instant invention relates to an immunoglobulin G-degrading enzyme of *S. pyogenes* (IdeS) polypeptide for use in the treatment of AAV-seronegative patients.

In certain embodiments, the IdeS polypeptide according to the instant invention is *S. pyogenes* IdeS, or a variant or fragment of *S. pyogenes* IdeS which retains cysteine protease activity. The variant may be an IdeS polypeptide from another organism, such as another bacterium. The bacterium is preferably a Streptococcus. The Streptococcus is preferably a group A Streptococcus, a group C Streptococcus or a group G Streptococcus. In particular, the variant may be an IdeS polypeptide from a group C Streptococcus such as *S. equi* or *S. zooepidemicus.* Alternatively, the variant may be from *Pseudomonas putida* or recombinant variant from other bacterial strains. In certain embodiments, the IdeS polypeptide according to the instant invention comprises the sequence of any of SEQ ID NOs:3-43 or 48.

In certain embodiments, the cysteine protease activity of the polypeptide has the ability to cleave all four human subclasses of IgG at glycine residue 237 in the lower hinge region of the IgG heavy chains without cleaving the IgA, IgM, IgD and IgE isotypes.

In certain embodiments, the IdeS polypeptide comprises or consists of the amino acid sequence of any of SEQ ID NOs:3 - 18, 23 or 48 or a function-conservative variant thereof.

In certain embodiments, the polypeptide according to the instant invention may differ by 1, 2, 3, 4 or 5 amino acids from the sequence of any of SEQ ID NOs:3 - 18, 23 or 48.

In certain embodiments, the instant invention includes polypeptides that are function-conservative variants of the polypeptide of any of SEQ ID NOs:3 - 18, 23 or 48.

In certain embodiments, the polypeptide of the instant invention comprises at least 60%, 65%, 70%, 75%, 80%, at least 85%, at least 90%, at least 95%, at least 97% or 100% of identity to the sequence of any of SEQ ID NOs:3 - 18, 23 or 48, and is still able to cleave all four human subclasses of IgG at glycine residue 237 in the lower hinge region of the IgG heavy chains without cleaving the IgA, IgM, IgD and IgE isotypes.

In certain embodiments, the polypeptide of the instant invention consists of the amino acid sequence as set forth in any of SEQ ID NOs:3 - 18, 23 or 48 or a variant thereof comprising at least 60%, 65%, 70%, 75%, 80%, 85%, 90%, 95%, 96%, 97%, 98%, 99%, 99.5% or 99.9% identity with the sequence of any of SEQ ID NOs:3 - 18, 23 or 48 and is still able to cleave all four human subclasses of IgG at glycine residue 237 in the lower hinge region of the IgG heavy chains without cleaving the IgA, IgM, IgD and IgE isotypes.

In certain embodiments, the polypeptide of the instant invention is a fragment of the polypeptide of any of SEQ ID NOs:3 - 18, 23 or 48 which is still able to cleave all four human subclasses of IgG at glycine residue 237 in the lower hinge region of the IgG heavy chains without cleaving the IgA, IgM, IgD and IgE isotypes.

In certain embodiments, the polypeptide of the instant invention contains residues Lys-55 and/or Cys-65 and/or His-233 and/or Asp-255 and/or Asp-257 corresponding to the amino acid residues of any of SEQ ID NOs:3 - 18, 23 or 48. In certain embodiments, the polypeptide of the instant invention contains each of residues corresponding to Lys-55, Cys-65, His-233, Asp-255 and Asp-257 of SEQ ID NO:4. In certain embodiments, the polypeptide of the instant invention contains at least one of, at least two of, at least three of, at least 4 of, or all 5 of the residues corresponding to Lys-55, Cys-65, His-233, Asp-255 and Asp-257 of SEQ ID NO:4.

To verify whether polypeptides or fragments of the instant invention are still able to cleave the four human subclasses of IgG, a test may be performed with each polypeptide or fragment. For example, after incubation of the tested polypeptides or fragments with human IgG, the study of the digestion fragments will be done by Western blot. Lack of detection of intact IgG and detection of digestion fragments of human IgG will indicate that the tested polypeptides or fragments have the capacity to cleave all four human subclasses of IgG.

As used herein, the term "function-conservative variant(s)" refer to those in which a given amino acid residue in a protein or enzyme has been changed (inserted, deleted or substituted) without altering the overall conformation and function of the peptide. Such variants include protein having amino acid alterations such as deletions, insertions and/or substitutions. A "deletion" refers to the absence of one or more amino acids in the protein. An "insertion" refers to the addition of one or more of amino acids in the protein. A "substitution" refers to the replacement of one or more amino acids by another amino acid residue in the protein. Typically, a given amino acid is replaced by an amino acid having similar properties (such as, for example, polarity, hydrogen bonding potential, acidic, basic, hydrophobic, aromatic, and the like). This given amino acid can be a natural amino acid or a non-natural amino acid. Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared. Two amino acid sequences are "substantially homologous" or "substantially similar" when greater than 80 %, preferably greater than 85 %, preferably greater than 90 % of the amino acids are identical, or greater than about 90 %, preferably greater than 95 %, are similar (functionally identical) over the whole length of the shorter sequence. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

In certain embodiments, the polypeptide of the instant invention comprises the sequence of SEQ ID NO:4 or SEQ ID NO:48 in which one or more residues have been conservatively substituted with a functionally similar residue and which displays the functional aspects of the polypeptide of SEQ ID NO:4 or SEQ ID NO:48 as described herein.

In certain embodiments, the polypeptide of the instant invention comprises the sequence of any of SEQ ID NOs:3-18, 23 or 48 in which one or more residues have been conservatively substituted with a functionally similar residue and which displays the functional aspects of the polypeptide of SEQ ID NOs:3-18, 23 or 48 as described herein.

Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid or another.

The term "conservative substitution" also includes the use of a chemically derivatized residue in place of a non-derivatized residue. "Chemical derivative" refers to a subject peptide having one or more residues chemically derivatized by reaction of a functional side group. Examples of such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Chemical derivatives also include peptides that contain one or more naturally-occurring amino acid derivatives of the twenty standard amino acids. For examples: 4-hydroxyproline may be substituted for proline; 5-hydroxylysine may be substituted for lysine; 3-methylhistidine may be substituted for histidine; homoserine may be substituted for serine; and ornithine may be substituted for lysine. The term "conservative substitution" also includes the use of non-natural amino acids aimed to control and stabilize peptides or proteins secondary structures. These non-natural amino acids are chemically modified amino acids such as prolinoamino acids, beta-amino acids, N-methylamino acids, cyclopropylamino acids, alpha, alpha-substituted amino acids as describe here below. These non-natural amino acids may include also fluorinated, chlorinated, brominated or iodinated modified amino acids.

In certain embodiments, the polypeptide of the instant invention differs by at least 1, 2, 5, 10, 20, 30, 50 or more mutations (which may be substitutions, deletions or insertions of amino acids) of SEQ ID NO:4 or of any of the sequences of SEQ ID NOs:3-18, 23 or 48. For example, from 1 to 50, 2 to 30, 3 to 20 or 5 to 10 amino acid substitutions, deletions or insertions may be made. The modified polypeptide generally retains activity as an IgG-specific cysteine protease. In particular embodiments, the amino acid substitutions are conservative substitutions.

In certain embodiments, the polypeptide of the instant invention may additionally include a signal sequence.

In certain embodiments, the signal sequence comprises or consists of the amino acid sequences: SEQ ID NO:49, SEQ ID NO:50 or SEQ ID NO:51 or function-conservative variants thereof.

In certain embodiments, polypeptides of the instant invention may comprise a tag. A tag is a tag-containing sequence which can be useful for the purification of the peptides. It is attached by a variety of techniques such as affinity chromatography, for the localization of said peptide or polypeptide within a cell or a tissue sample using immunolabeling techniques, the detection of said peptide or polypeptide by immunoblotting etc. Examples of tags commonly employed in the art are the GST (glutathion-S-transferase)-tag, the FLAG^{™}-tag, the Strep-tag^{™}, V5 tag, myc tag, His tag etc.

In certain embodiments, polypeptides of the instant invention may be labelled by a fluorescent dye. Dye-labelled fluorescent peptides are important tools in cellular studies. Peptides can be labelled on the N-terminal side or on the C-terminal side.

N-Terminal Peptide Labeling Using Amine-Reactive Fluorescent Dyes: Amine-reactive fluorescent probes are widely used to modify peptides at the N-terminal or lysine residue. A number of fluorescent amino-reactive dyes have been developed to label various peptides, and the resultant conjugates are widely used in biological applications. Three major classes of amine-reactive fluorescent reagents are currently used to label peptides: succinimidyl esters (SE), isothiocyanates and sulfonyl chlorides.

C-Terminal Labeling Using Amine-Containing Fluorescent Dyes: Amine-containing dyes are used to modify peptides using water-soluble carbodiimides (such as EDC) to convert the carboxy groups of the peptides into amide groups. Either NHS or NHSS may be used to improve the coupling efficiency of EDC-mediated protein-carboxylic acid conjugations.

In certain embodiments, polypeptides used in the therapeutic methods according to the instant invention may be modified in order to improve their therapeutic efficacy. Such modification of therapeutic compounds may be used to decrease toxicity, increase circulatory time, modify biodistribution or reduce immunogenicity. For example, the toxicity of potentially important therapeutic compounds can be decreased significantly by combination with a variety of drug carrier vehicles that modify biodistribution.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and trifunctional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomerular filtration (*e.g.,* less than 45 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes (see *e.g*., technologies of established by VectraMed, Plainsboro, N.J.). Such linkers may be used in modifying the peptides-derived described herein for therapeutic delivery.

According to the instant invention, polypeptides may be produced by conventional automated peptide synthesis methods or by recombinant expression. General principles for designing and making proteins are well known to those of skill in the art.

Polypeptides of the instant invention may be synthesized in solution or on a solid support in accordance with conventional techniques. Various automatic synthesizers are commercially available and can be used in accordance with known protocols as described in Stewart and Young; Tam *et al.,* 1983; Merrifield, 1986 and Barany and Merrifield, Gross and Meienhofer, 1979. Peptides of the instant invention may also be synthesized by solid-phase technology employing an exemplary peptide synthesizer such as a Model 433A from Applied Biosystems Inc. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors (Giga-Hama *et al.,* 1999); insect cell systems infected with virus expression vectors *(e.g.,* baculovirus, see Ghosh *et al.,* 2002); plant cell systems transfected with virus expression vectors (e.g., cauliflower mosaic virus, CaMV; tobacco mosaic virus, TMV) or transformed with bacterial expression vectors (*e.g.,* Ti or pBR322 plasmid; see *e.g.,* Babe *et al.,* 2000); or animal cell systems. Those of skill in the art are aware of various techniques for optimizing mammalian expression of proteins, see *e.g*., Kaufman, 2000; Colosimo *et al.,* 2000. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the peptide substrates or fusion polypeptides in bacteria, yeast and other invertebrates are known to those of skill in the art and a briefly described herein below. U.S. Pat. No. 6,569,645; U.S. Pat. No. 6,043,344; U.S. Pat. No. 6,074,849; and U.S. Pat. No. 6,579,520 provide specific examples for the recombinant production of peptides and these patents are expressly incorporated herein by reference for those teachings. Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

In the recombinant production of the polypeptides-derived of the instant invention, it would be necessary to employ vectors comprising polynucleotide molecules for encoding the peptides-derived. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding sites, and appropriate sequences which control transcription and translation.

The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the peptides or polypeptides of the instant invention will of course depend upon the specific host cell to be used, and is within the skill of the ordinary artisan. Methods for the construction of mammalian expression vectors are described, for example, in Okayama *et al.,* 1983; Cosman *et al.,* 1986; Cosman *et al.,* 1984; EP-A-0367566; and WO 91/18982. Other considerations for producing expression vectors are detailed in *e.g.,* Makrides *et al.,* 1999; Kost *et al.,* 1999. Wurm *et al.,* 1999 is incorporated herein as teaching factors for consideration in the large-scale transient expression in mammalian cells for recombinant protein production.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" includes a DNA sequence recognized by the synthetic machinery of the cell, or introduced synthetic machinery, required to initiate the specific transcription of a gene. Nucleotide sequences are operably linked when the regulatory sequence functionally relates to the DNA encoding the peptide of interest *(i.e.,* 4N1K, a variant and the like). Thus, a promoter nucleotide sequence is operably linked to a given DNA sequence if the promoter nucleotide sequence directs the transcription of the sequence.

Similarly, the phrase "under transcriptional control" means that the promoter is in the correct location and orientation in relation to the nucleic acid to control RNA polymerase initiation and expression of the gene. Any promoter that will drive the expression of the nucleic acid may be used. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter. Common promoters include, *e.g.,* the human cytomegalovirus (CMV) immediate early gene promoter, the SV40 early promoter, the Rous sarcoma virus long terminal repeat, [beta]-actin, rat insulin promoter, the phosphoglycerol kinase promoter, the human alpha-1 antitrypsin promoter, the transthyretin promoter, the TBG promoter and other liver-specific promoters, the desmin promoter and similar muscle-specific promoters, the EF1-alpha promoter, the CAG promoter and other constitutive promoters, hybrid promoters with multi-tissue specificity, promoters specific for neurons like synapsin and glyceraldehyde-3-phosphate dehydrogenase promoter, all of which are promoters well known and readily available to those of skill in the art, can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient to produce a recoverable yield of protein of interest. By employing a promoter with well-known properties, the level and pattern of expression of the protein of interest following transfection or transformation can be optimized. Inducible promoters also may be used.

Another regulatory element that is used in protein expression is an enhancer. These are genetic elements that increase transcription from a promoter located at a distant position on the same molecule of DNA. Where an expression construct employs a cDNA insert, one will typically desire to include a polyadenylation signal sequence to effect proper polyadenylation of the gene transcript. Any polyadenylation signal sequence recognized by cells of the selected transgenic animal species is suitable for use in the instant invention, such as human or bovine growth hormone and SV40 polyadenylation signals.

As explained above, the use of an immunoglobulin G-degrading enzyme polypeptide like the Ides can be suitable for the treatment of a disease treated by gene therapy using an AAV vector.

### Therapeutic composition

In certain embodiments, the instant invention relates to a therapeutic composition comprising an immunoglobulin G-degrading enzyme polypeptide or nucleic acids encoding an immunoglobulin G-degrading enzyme polypeptide or an expression vector comprising a nucleic acid encoding an immunoglobulin G-degrading enzyme polypeptide according to the instant invention for use in the treatment of a disease treated by gene therapy using a vector in a patient in need thereof.

Any therapeutic agent of the instant invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

Pharmaceutical compositions of the instant invention may be formulated for topical, oral, intranasal, parenteral, intraocular, intravenous, intramuscular or subcutaneous administration and the like.

Pharmaceutical compositions of the instant invention may contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The doses used for the administration can be adapted as a function of various parameters, and in particular as a function of the mode of administration used, of the relevant pathology, or alternatively of the desired duration of treatment.

In addition, other pharmaceutically acceptable forms include, *e.g*., tablets or other solids for oral administration; time release capsules; and any other form currently can be used.

Pharmaceutical compositions of the instant invention may comprise a further therapeutically active agent.

A protease or glycosidase can be administered to a subject at any suitable dose. For example, a suitable dosage may be from about 0.05 mg/kg to about 5 mg/kg body weight of a subject, or from about 0.1 mg/kg to about 4 mg/kg body weight of a subject.

In certain embodiments, IdeZ is administered at a dosage of about 0.01 mg/kg to about 10 mg/kg body weight of a subject. For example, a suitable dosage may be from about 0.05 mg/kg to about 5 mg/kg body weight of a subject, or from about 0.1 mg/kg to about 4 mg/kg body weight of a subject.

In certain embodiments, IdeS is administered at a dosage of about 0.01 mg/kg to about 10 mg/kg body weight of a subject. For example, a suitable dosage may be from about 0.05 mg/kg to about 5 mg/kg body weight of a subject, or from about 0.1 mg/kg to about 4mg/kg body weight of a subject.

In certain embodiments, EndoS is administered at a dosage of about 0.01 mg/kg to about 10 mg/kg body weight of a subject. For example, a suitable dosage may be from about 0.05 mg/kg to about 5 mg/kg body weight of a subject, or from about 0.1 mg/kg to about 4mg/kg body weight of a subject.

Methods according to the instant invention can be performed in any suitable order unless otherwise indicated herein. In certain embodiments, a method may comprise first (a) administering to a subject a protease and/or glycosidase effective to degrade or digest neutralizing antibodies; and then (b) administering to the subject a recombinant viral vector. In certain embodiments, (b) administering to a subject a recombinant viral vector, is performed between about 1 minute to about 90 days after (a) administering to a subject a protease and/or glycosidase effective to degrade or digest neutralizing antibodies. In certain embodiments, (a) administering to a subject a protease and/or glycosidase effective to degrade or digest neutralizing antibodies, and (b) administering to the subject a recombinant viral vector, are performed at about the same time.

In certain embodiments, a method may comprise first (a) administering to a subject a recombinant viral vector bearing a heterologous polynucleotide and then (b) administering to the subject an amount of a protease and/or glycosidase effective to degrade or digest antibodies that bind to a recombinant viral vector and/or heterologous polynucleotide or protein or peptide encoded by the heterologous polynucleotide. In certain embodiments, (b) administering to the subject an amount of a protease and/or glycosidase effective to degrade or digest antibodies that bind to a recombinant viral vector and/or heterologous polynucleotide or protein or peptide encoded by the heterologous polynucleotide, is performed between about 1 minute to about 90 days after (a) administering to a subject a recombinant viral vector bearing the heterologous polynucleotide. In certain embodiments, (a) administering to a subject a recombinant viral vector bearing a heterologous polynucleotide, and (b) administering to the subject an amount of a protease and/or glycosidase effective to degrade or digest antibodies that bind to a recombinant viral vector and/or heterologous polynucleotide or protein or peptide encoded by the heterologous polynucleotide, are performed at about the same time.

Antibodies, such as neutralizing antibodies, may be preexisting and may be present in a subject, even before administration of a viral vector, at levels that inhibit or reduce recombinant viral vector cell transduction. Alternatively, antibodies may develop in a subject after exposure to a virus upon which the recombinant viral vector is based. Still further, antibodies, such as neutralizing antibodies, or antibodies that bind to the heterologous polynucleotide or a protein or peptide encoded by the heterologous polynucleotide encapsidated by the viral vector may develop in a subject after administration of a recombinant viral vector.

Accordingly, the methods herein are applicable to subjects with pre-existing antibodies and subjects without pre-existing antibodies. As set forth herein, such subjects include subjects that have been exposed to wildtype virus and develop pre-existing antibodies against the viral vector based upon the wildtype virus, as well as subjects that have received a viral vector gene therapy treatment and have developed antibodies and may be subsequently treated with one or more additional doses of the same viral vector gene therapy (referred to as redosing) or be treated with a different gene therapy treatment (e.g., a different heterologous polynucleotide) using the same viral vector to deliver the gene therapy treatment.

A subject may be tested for antibodies prior to viral vector administration and/or prior to administration of a protease glycosidase. Nonlimiting examples of antibodies to test for include neutralizing antibodies, antibodies that bind to a protease or glycosidase as set forth herein, antibodies that bind to the heterologous polynucleotide and antibodies that bind to the protein or peptide encoded by the heterologous polynucleotide. Subjects can therefore be screened for neutralizing antibodies, antibodies that bind to a protease or glycosidase as set forth herein, antibodies that bind to a heterologous polynucleotide or antibodies that bind to a protein or peptide encoded by the heterologous polynucleotide, prior to administration of a recombinant viral vector and/or prior to administration of a protease glycosidase.

Subjects that have pre-existing antibodies (e.g., IgG) that bind to a protease or glycosidase as set forth herein, can optionally be excluded from initial treatment by a method according to the instant invention. However, not all subjects that have or develop antibodies that bind to a protease or glycosidase need be excluded from treatment methods according to the instant invention. For example, a subject with detectable anti-IdeS IgG having a titer of less than 15 mg/per liter can still be treated using methods according to the instant invention.

Subjects also can be screened for neutralizing antibodies, antibodies that bind to a heterologous polynucleotide or antibodies that bind to a protein or peptide encoded by the heterologous polynucleotide after administration of a recombinant viral vector. Such subjects can optionally be monitored for a period of time after administration of the recombinant viral vector in order to determine if such antibodies develop or are prevented from developing in a subject in which pre-existing antibodies have not been detected, or in the case of a subject with pre-existing antibodies whether protease and/or glycosidase decreases or eliminates such pre-existing antibodies.

In certain embodiments, a protease and/or glycosidase is administered to a subject after testing positive for the presence of neutralizing antibodies, antibodies that bind to a protease or glycosidase as set forth herein, antibodies that bind to a heterologous polynucleotide or antibodies that bind to a protein or peptide encoded by the heterologous polynucleotide. In certain embodiments, a protease and/or glycosidase is administered to a subject before testing positive for the presence of neutralizing antibodies, antibodies that bind to a protease or glycosidase as set forth herein, antibodies that bind to a heterologous polynucleotide or antibodies that bind to a protein or peptide encoded by the heterologous polynucleotide.

In certain embodiments, subjects are not tested for antibodies prior to or after administration of a protease and/or glycosidase. Accordingly, testing for neutralizing antibodies, antibodies that bind to a protease or glycosidase as set forth herein, antibodies that bind to a heterologous polynucleotide or antibodies that bind to a protein or peptide encoded by the heterologous polynucleotide after administration of a protease and/or glycosidase or administration of a recombinant viral vector is optional in treatment methods according to the instant invention.

A protease or glycosidase can be administered to a subject any number of times. For example, a protease and/or glycosidase can be administered 2 to 5 times, 2 to 10 times, 2 to 15 times to a subject.

A protease or glycosidase can be administered to a subject for any duration of time on a regular basis, such as consecutive days, or alternating days, or an irregular basis. In certain embodiments, a protease and/or glycosidase is administered from about 1 to 12 weeks, or from about 1 to 10 weeks, or from about 1 to 8 weeks, or from about 1 to 6 weeks, or from about 1 to 4 weeks, or from about 1 to 2 weeks, or about 2 weeks after administration of a recombinant viral vector.

In certain embodiments, a recombinant viral vector is administered before or after a protease or glycosidase is administered to a subject. In certain embodiments, a recombinant viral vector is administered to a subject *e.g.,* 1-12, 12-24 or 24-48 hours, or 2-4, 4-6, 6-8, 8-10, 10-14, 14-20, 20-25, 25-30, 30-50, or more than 50 days following administering a protease or glycosidase to the subject. In certain embodiments, a protease or glycosyl is administered to a subject *e.g.,* 1-12, 12-24 or 24-48 hours, or 2-4, 4-6, 6-8, 8-10, 10-14, 14-20, 20-25, 25-30, 30-50, or more than 50 days following administering a recombinant viral vector to the subject.

The recombinant viral vector, protease and/or glycosidase may be administered alone or in a combination. In certain embodiments, a recombinant viral vector is administered to a subject separately from a protease and/or glycosidase. In certain embodiments, a recombinant viral vector is administered to a subject in combination with a protease and/or glycosidase.

In certain embodiments, a mixture of a protease and glycosidase is administered to a subject, one or more times. In certain embodiments, two or more proteases or glycosidases are administered to a subject, one or more times.

In certain embodiments, at least one immunosuppressive agent is administered to a subject prior to, substantially contemporaneously with or after administration of a recombinant viral vector, protease or glycosidase to the subject. In certain embodiments, an immunosuppressive agent is an anti-inflammatory agent such as a steroid. In certain embodiments, an immunosuppressive agent is prednisone, cyclosporine (e.g., cyclosporine A), mycophenolate, rituximab, rapamycin or a derivative thereof.

Additional strategies to reduce humoral immunity include methods to remove, deplete, capture, and/or inactivate antibodies, commonly referred to as apheresis and more particularly, plasmapheresis where blood products are involved. Apheresis or plasmapheresis, is a process in which a human subject's plasma is circulated *ex vivo* (extracorporal) through a device that modifies the plasma through addition, removal and/or replacement of components before its return to the patient. Plasmapheresis can be used to remove human immunoglobulins (e.g., IgG, IgE, IgA, IgD) from a blood product (e.g., plasma). This procedure depletes, captures, inactivates, reduces or removes immunoglobulins (antibodies) that bind a recombinant viral vector, bind to a heterologous polynucleotide, bind to a protein or peptide encoded by the heterologous polynucleotide, bind to a protease and/or bind to a glycosidase thereby reducing the titer of antibodies in the treated subject that may contribute, for example, to viral vector neutralization. An example is a device composed of an AAV capsid affinity matrix column. Passing blood product (e.g., plasma) through such an AAV capsid affinity matrix would result in binding only of AAV antibodies, and of all isotypes (including IgG, IgM, etc.). Immunoadsorption (U.S. Patent Application publication US 2018/0169273 A1) can also be used to deplete immunoglobulins, and more particularly anti-AAV antibodies. Affinity ligands (international patent application publication WO/2018/158397) can also be used to deplete immunoglobulins, and more particularly anti-AAV antibodies. Any of the aforementioned strategies can be used prior to, substantially contemporaneously with or after administration of a recombinant viral vector, protease or glycosidase to the subject.

Additional strategies to reduce (overcome) or avoid humoral immunity to AAV in systemic gene transfer include use of AAV empty capsid particles and/or capsid proteins as decoys to adsorb anti-AAV antibodies. Still further strategies are described in Mingozzi et al., 2013, Blood, 122:23-36.

In accordance with the instant invention, a protease and/or glycosidase may be encapsulated or complexed with liposomes, nanoparticles, lipid nanoparticles, polymers, microparticles, microcapsules, micelles, or extracellular vesicles.

In accordance with the instant invention, viral particles may be encapsulated or complexed with liposomes, nanoparticles, lipid nanoparticles, polymers, microparticles, microcapsules, micelles, or extracellular vesicles.

In certain embodiments, liposomes, nanoparticles, lipid nanoparticles, polymers, microparticles, microcapsules, micelles, or extracellular vesicles can be used in the instant invention for non-viral delivery of gene therapy nucleic acids.

A "lipid nanoparticle" or "LNP" refers to a lipid-based vesicle useful for delivery of recombinant viral vector and or protease and/or glycosidase and having dimensions on the nanoscale, *i.e.,* from about 10 nm to about 1000 nm, or from about 50 to about 500 nm, or from about 75 to about 127 nm. Without being bound by theory, LNP is believed to provide the protease, glycosidase, or recombinant viral vector with partial or complete shielding from the immune system. Shielding allows delivery of the protease, glycosidase, or recombinant viral vector to a tissue or cell while avoiding inducing a substantial immune response against the protease, glycosidase, or recombinant viral vector *in vivo.* Shielding may also allow repeated administration without inducing a substantial immune response against the protease, glycosidase, or recombinant viral vector *in vivo* (*e.g.,* in a subject such as a human). Shielding may also improve or increase delivery efficiency, duration of therapeutic effect and/or therapeutic efficacy *in vivo.*

The pI (isoelectric point) of AAV is in a range from about 6 to about 6.5. Thus, the AAV surface carries a slight negative charge. As such it may be beneficial for the LNP to comprise a cationic lipid such as, for example, an amino lipid. Exemplary amino lipids have been described in U.S. Patent Nos. 9,352,042, 9,220,683, 9,186,325, 9,139,554, 9,126,966 9,018,187, 8,999,351, 8,722,082, 8,642,076, 8,569,256, 8,466,122, and 7,745,651 and U.S. Patent Publication Nos. 2016/0213785, 2016/0199485, 2015/0265708, 2014/0288146, 2013/0123338, 2013/0116307, 2013/0064894, 2012/0172411, and 2010/0117125.

The terms "cationic lipid" and "amino lipid" are used interchangeably herein to include those lipids and salts thereof having one, two, three, or more fatty acid or fatty alkyl chains and a pH-titratable amino group *(e.g.,* an alkylamino or dialkylamino group). The cationic lipid is typically protonated *(i.e.,* positively charged) at a pH below the pKa of the cationic lipid and is substantially neutral at a pH above the pKa. The cationic lipids may also be titratable cationic lipids. In certain embodiments, the cationic lipids comprise: a protonatable tertiary amine (e.g., pH-titratable) group; C18 alkyl chains, wherein each alkyl chain independently has 0 to 3 *(e.g.,* 0, 1, 2, or 3) double bonds; and ether, ester, or ketal linkages between the head group and alkyl chains.

Cationic lipids may include, without limitation, 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane (y-DLenDMA), 2,2-dilinoleyl-4-(2-dimethylaminoethyl)-[1,3]-dioxolane (DLin-K-C2-DMA, also known as DLin-C2K-DMA, XTC2, and C2K), 2,2-dilinoleyl-4-dimethylaminomethyl-[1,3]-dioxolane (DLin-K-DMA), dilinoleylmethyl-3-dimethylaminopropionate (DLin-M-C2-DMA, also known as MC2), (6Z,9Z,28Z,31 Z)-heptatriaconta-6,9,28,31-tetraen-19-yl 4-(dimethylamino)butanoate (DLin-M-C3-DMA, also known as MC3), salts thereof, and mixtures thereof. Other cationic lipids also include, but are not limited to, 1,2-distearyloxy-N,N-dimethyl-3-aminopropane (DSDMA), 1,2-dioleyloxy-N,N-dimethyl-3-aminopropane (DODMA), 2,2-dilinoleyl-4-(3-dimethylaminopropyl)-[1,3]-dioxolane (DLin-K-C3-DMA), 2,2-dilinoleyl-4-(3-dimethylaminobutyl)-[1,3]-dioxolane (DLin-K-C4-DMA), DLen-C2K-DMA, γ-DLen-C2K-DMA, and (DLin-MP-DMA) (also known as 1-B11).

Still further cationic lipids may include, without limitation, 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), N,N-dioleyl-N,N-dimethylammonium chloride (DODAC), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethyl-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), dexamethasone-sperimine (DS) and disubstituted spermine (D2S) or mixtures thereof.

A number of commercial preparations of cationic lipids can be used, such as, LIPOFECTIN^{®} (including DOTMA and DOPE, available from GIBCO/BRL), and LIPOFECTAMINE^{®} (comprising DOSPA and DOPE, available from GIBCO/BRL).

In certain embodiments, cationic lipid may be present in an amount from about 10% by weight of the LNP to about 85% by weight of the lipid nanoparticle, or from about 50 % by weight of the LNP to about 75% by weight of the LNP.

Sterols may confer fluidity to the LNP. As used herein, "sterol" refers to any naturally occurring sterol of plant (phytosterols) or animal (zoosterols) origin as well as non-naturally occurring synthetic sterols, all of which are characterized by the presence of a hydroxyl group at the 3-position of the steroid A-ring. The sterol can be any sterol conventionally used in the field of liposome, lipid vesicle or lipid particle preparation, most commonly cholesterol. Phytosterols may include campesterol, sitosterol, and stigmasterol. Sterols also includes sterol-modified lipids, such as those described in U.S. Patent Application Publication 2011/0177156. In certain embodiments, a sterol may be present in an amount from about 5% by weight of the LNP to about 50% by weight of the lipid nanoparticle or from about 10% by weight of the LNP to about 25% by weight of the LNP.

LNP can comprise a neutral lipid. Neutral lipids may comprise any lipid species which exists either in an uncharged or neutral zwitterionic form at physiological pH. Such lipids include, without limitation, diacylphosphatidylcholine, diacylphosphatidylethanolamine, ceramide, sphingomyelin, dihydrosphingomyelin, cephalin, and cerebrosides. The selection of neutral lipids is generally guided by consideration of, inter alia, particle size and the requisite stability. In certain embodiments, the neutral lipid component may be a lipid having two acyl groups (e.g., diacylphosphatidylcholine and diacylphosphatidylethanolamine).

Lipids having a variety of acyl chain groups of varying chain length and degree of saturation are available or may be isolated or synthesized by well-known techniques. In certain embodiments, lipids containing saturated fatty acids with carbon chain lengths in the range of C14 to C22 may be used. In certain embodiments, lipids with mono or diunsaturated fatty acids with carbon chain lengths in the range of C14 to C22 are used. Additionally, lipids having mixtures of saturated and unsaturated fatty acid chains can be used. Exemplary neutral lipids include, without limitation, 1,2-dioleoyl-sn-glycero-3-phosphatidyl-ethanolamine (DOPE), 1,2-distearoyl-sn-glycero-3-phosphocholine (DSPC), 1-palmitoyl-2-oleoyl-sn-glycero-3-phosphocholine (POPC), or any related phosphatidylcholine. The neutral lipids may also be composed of sphingomyelin, dihydrosphingomyelin, or phospholipids with other head groups, such as serine and inositol.

In certain embodiments, the neutral lipid may be present in an amount from about 0.1% by weight of the lipid nanoparticle to about 75% by weight of the LNP, or from about 5% by weight of the LNP to about 15% by weight of the LNP.

LNP encapsulated protease, glycosidase, or recombinant viral vector can be incorporated into pharmaceutical compositions, *e*.*g*., a pharmaceutically acceptable carrier or excipient. Such pharmaceutical compositions are useful for, among other things, administration and delivery of LNP encapsulated protease, glycosidase, or recombinant viral vector to a subject *in vivo* or *ex vivo.*

Preparations of LNP can be combined with additional components, which may include, for example and without limitation, polyethylene glycol (PEG) and sterols.

The term "PEG" refers to a polyethylene glycol, a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, for example, the following functional PEGs: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH2), monomethoxypolyethylene glycol-tresylate (MePEG-TRES), and monomethoxypolyethylene glycol-imidazolyl-carbonyl (MePEG-IM).

In certain embodiments, PEG may be a polyethylene glycol with an average molecular weight of about 550 to about 10,000 daltons and is optionally substituted by alkyl, alkoxy, acyl or aryl. In certain embodiments, the PEG may be substituted with methyl at the terminal hydroxyl position. In certain embodiments, the PEG may have an average molecular weight from about 750 to about 5,000 daltons, or from about 1,000 to about 5,000 daltons, or from about 1,500 to about 3,000 daltons or from about 2,000 daltons or of about 750 daltons. The PEG can be optionally substituted with alkyl, alkoxy, acyl or aryl. In certain embodiments, the terminal hydroxyl group may be substituted with a methoxy or methyl group.

PEG-modified lipids include the PEG-dialkyloxypropyl conjugates (PEG-DAA) described in U.S. Patent Nos. 8,936,942 and 7,803,397. PEG-modified lipids (or lipid-polyoxyethylene conjugates) that are useful may have a variety of "anchoring" lipid portions to secure the PEG portion to the surface of the lipid vesicle. Examples of suitable PEG-modified lipids include PEG-modified phosphatidylethanolamine and phosphatidic acid, PEG-ceramide conjugates (e.g., PEG-CerC14 or PEG-CerC20) which are described in U.S. Patent No. 5,820,873, PEG-modified dialkylamines and PEG-modified 1,2-diacyloxypropan-3-amines. In certain embodiments, the PEG-modified lipid may be PEG-modified diacylglycerols and dialkylglycerols. In certain embodiments, the PEG may be in an amount from about 0.5% by weight of the LNP to about 20% by weight of the LNP, or from about 5% by weight of the LNP to about 15% by weight of the LNP.

Furthermore, LNP can be a PEG-modified and a sterol-modified LNP. The LNPs, combined with additional components, can be the same or separate LNPs. In other words, the same LNP can be PEG modified and sterol modified or, alternatively, a first LNP can be PEG modified and a second LNP can be sterol modified. Optionally, the first and second modified LNPs can be combined.

In certain embodiments, prior to encapsulating LNPs may have a size in a range from about 10 nm to 500 nm, or from about 50 nm to about 200 nm, or from 75 nm to about 125 nm. In certain embodiments, LNP encapsulated protease, glycosidase, or recombinant viral vector may have a size in a range from about 10 nm to 500 nm.

The terms "nucleic acid" and "polynucleotide" are used interchangeably herein to refer to all forms of nucleic acid, oligonucleotides, including deoxyribonucleic acid (DNA) and ribonucleic acid (RNA). Nucleic acids include genomic DNA, cDNA and antisense DNA, and spliced or unspliced mRNA, rRNA tRNA and inhibitory DNA or RNA (RNAi, *e*.*g*., small or short hairpin (sh)RNA, microRNA (miRNA), small or short interfering (si)RNA, trans-splicing RNA, or antisense RNA).

Nucleic acids include naturally occurring, synthetic, and intentionally modified or altered polynucleotides. Nucleic acids can be single, double, or triplex, linear or circular, and can be of any length. In discussing nucleic acids, a sequence or structure of a particular polynucleotide may be described herein according to the convention of providing the sequence in the 5' to 3' direction.

A "heterologous" nucleic acid sequence refers to a polynucleotide inserted into a plasmid or vector for purposes of vector mediated transfer/delivery of the polynucleotide into a cell. Heterologous nucleic acid sequences are distinct from viral nucleic acid, *i.e.,* are non-native with respect to viral nucleic acid. Once transferred/delivered into the cell, a heterologous nucleic acid sequence, contained within the vector, can be expressed (e.g., transcribed, and translated if appropriate). Alternatively, a transferred/delivered heterologous polynucleotide in a cell, contained within the vector, need not be expressed. Although the term "heterologous" is not always used herein in reference to nucleic acid sequences and polynucleotides, reference to a nucleic acid sequence or polynucleotide even in the absence of the modifier "heterologous" is intended to include heterologous nucleic acid sequences and polynucleotides in spite of the omission.

A "transgene" is used herein to conveniently refer to a nucleic acid that is intended or has been introduced into a cell or organism. Transgenes include any nucleic acid, such as a heterologous polynucleotide sequence or a heterologous nucleic acid encoding a protein or peptide. The term transgene and heterologous nucleic acid/polynucleotide sequences are used interchangeably herein.

In certain embodiments, a heterologous polynucleotide encodes a protein selected from the group consisting of GAA (acid alpha-glucosidase) for treatment of Pompe disease; ATP7B (copper transporting ATPase2) for treatment of Wilson's disease; alpha galactosidase for treatment of Fabry's disease; ASS1 (arginosuccinate synthase) for treatment of Citrullinemia Type 1; beta-glucocerebrosidase for treatment of Gaucher disease Type 1; beta-hexosaminidase A for treatment of Tay Sachs disease; SERPING1 (C1 protease inhibitor or C1 esterase inhibitor) for treatment of hereditary angioedema (HAE), also known as C1 inhibitor deficiency type I and type II); and glucose-6-phosphatase for treatment of glycogen storage disease type I (GSDI).

In certain embodiments, a heterologous polynucleotide encodes a protein selected from the group consisting of insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor α (TGFα), platelet-derived growth factor (PDGF), insulin growth factors I and II (IGF-I and IGF-II), TGFβ, activins, inhibins, bone morphogenic protein (BMP), nerve growth factor (NGF), brain-derived neurotrophic factor (BDNF), neurotrophins NT-3 and NT4/5, ciliary neurotrophic factor (CNTF), glial cell line derived neurotrophic factor (GDNF), neurturin, agrin, netrin-1 and netrin-2, hepatocyte growth factor (HGF), ephrins, noggin, sonic hedgehog and tyrosine hydroxylase.

In certain embodiments, a heterologous polynucleotide encodes acid α-glucosidase (GAA). Administration of a recombinant viral vector comprising a heterologous polynucleotide encoding GAA to a subject with Pompe or another glycogen storage disease can lead to the expression of the GAA protein. Expression of GAA protein in the patient may serve to suppress, inhibit or reduce the accumulation of glycogen, prevent the accumulation of glycogen or degrade glycogen, which in turn can reduce or decrease one or more adverse effects of Pompe disease, or another glycogen storage disease.

In certain embodiments, a heterologous polynucleotide encodes a protein selected from the group consisting of thrombopoietin (TPO), an interleukin (IL-1 through IL-36, etc.), monocyte chemoattractant protein, leukemia inhibitory factor, granulocyte-macrophage colony stimulating factor, Fas ligand, tumor necrosis factors α and β, interferons α, β, and γ, stem cell factor, flk-2/flt3 ligand, IgG, IgM, IgA, IgD and IgE, chimeric immunoglobulins, humanized antibodies, single chain antibodies, T cell receptors, chimeric T cell receptors, single chain T cell receptors, class I and class II MHC molecules.

In certain embodiments, a heterologous polynucleotide encodes CFTR (cystic fibrosis transmembrane regulator protein), a blood coagulation (clotting) factor (Factor XIII, Factor IX, Factor VIII, Factor X, Factor VII, Factor VIIa, protein C, etc.) a gain of function blood coagulation factor, an antibody, retinal pigment epithelium-specific 65 kDa protein (RPE65), erythropoietin, LDL receptor, lipoprotein lipase, ornithine transcarbamylase, β-globin, α-globin, spectrin, α-antitrypsin, adenosine deaminase (ADA), a metal transporter (ATP7A or ATP7), sulfamidase, an enzyme involved in lysosomal storage disease (ARSA), hypoxanthine guanine phosphoribosyl transferase, β-25 glucocerebrosidase, sphingomyelinase, lysosomal hexosaminidase, branched-chain keto acid dehydrogenase, a hormone, a growth factor, insulin-like growth factor 1 or 2, platelet derived growth factor, epidermal growth factor, nerve growth factor, neurotrophic factor - 3 and -4, brain-derived neurotrophic factor, glial derived growth factor, transforming growth factor α and β, a cytokine, α-interferon, β-interferon, interferon-γ, interleukin-2, interleukin-4, interleukin 12, granulocyte-macrophage colony stimulating factor, lymphotoxin, a suicide gene product, herpes simplex virus thymidine kinase, cytosine deaminase, diphtheria toxin, cytochrome P450, deoxycytidine kinase, tumor necrosis factor, a drug resistance protein, a tumor suppressor protein (*e.g.,* p53, Rb, Wt-1, NF1, Von Hippel-Lindau (VHL), adenomatous polyposis coli (APC)), a peptide with immunomodulatory properties, a tolerogenic or immunogenic peptide or protein Tregitope or hCDR1, insulin, glucokinase, guanylate cyclase 2D (LCA-GUCY2D), Rab escort protein 1 (Choroideremia), LCA 5 (LCA-Lebercilin), ornithine ketoacid aminotransferase (Gyrate Atrophy), Retinoschisin 1 (X-linked Retinoschisis), USH1C (Usher's Syndrome 1C), X-linked retinitis pigmentosa GTPase (XLRP), MERTK (AR forms of RP: retinitis pigmentosa), DFNB 1 (Connexin 26 deafness), ACHM 2, 3 and 4 (Achromatopsia), PKD-1 or PKD-2 (Polycystic kidney disease), TPP1, CLN2, a sulfatase, N-acetylglucosamine-1-phosphate transferase, cathepsin A, GM2-AP, NPC1, VPC2, a sphingolipid activator protein, one or more zinc finger nucleases for genome editing, or one or more donor sequences used as repair templates for genome editing.

In certain embodiments, a heterologous polynucleotide encodes erythropoietin (EPO) for treatment of anemia; interferon-alpha, interferon-beta, and interferon-gamma for treatment of various immune disorders, viral infections and cancer; an interleukin (IL), including any one of IL-1 through IL-36, and corresponding receptors, for treatment of various inflammatory diseases or immuno-deficiencies; a chemokine, including chemokine (C-X-C motif) ligand 5 (CXCL5) for treatment of immune disorders; granulocyte-colony stimulating factor (G-CSF) for treatment of immune disorders such as Crohn's disease; granulocyte-macrophage colony stimulating factor (GM-CSF) for treatment of various human inflammatory diseases; macrophage colony stimulating factor (M-CSF) for treatment of various human inflammatory diseases; keratinocyte growth factor (KGF) for treatment of epithelial tissue damage; chemokines such as monocyte chemoattractant protein-1 (MCP-1) for treatment of recurrent miscarriage, HIV-related complications, and insulin resistance; tumor necrosis factor (TNF) and receptors for treatment of various immune disorders; alpha 1-antitrypsin for treatment of emphysema or chronic obstructive pulmonary disease (COPD); alpha-L-iduronidase for treatment of mucopolysaccharidosis I (MPS I); ornithine transcarbamoylase (OTC) for treatment of OTC deficiency; phenylalanine hydroxylase (PAH) or phenylalanine ammonia-lyase (PAL) for treatment of phenylketonuria (PKU); lipoprotein lipase for treatment of lipoprotein lipase deficiency; apolipoproteins for treatment of apolipoprotein (Apo) A-I deficiency; low-density lipoprotein receptor (LDL-R) for treatment of familial hypercholesterolemia (FH); albumin for treatment of hypoalbuminemia; lecithin cholesterol acyltransferase (LCAT); carbamoyl synthetase I; argininosuccinate synthetase; argininosuccinate lyase; arginase; fumarylacetoacetate hydrolase; porphobilinogen deaminase; cystathionine beta-synthase for treatment of homocystinuria; branched chain ketoacid decarboxylase; isovaleryl-CoA dehydrogenase; propionyl CoA carboxylase; methylmalonyl-CoA mutase; glutaryl CoA dehydrogenase; insulin; pyruvate carboxylase; hepatic phosphorylase; phosphorylase kinase; glycine decarboxylase; H-protein; T-protein; cystic fibrosis transmembrane regulator (CFTR); ATP-binding cassette, sub-family A (ABC1), member 4 (ABCA4) for the treatment of Stargardt disease; or dystrophin.

The terms "polypeptides," "proteins" and "peptides" are used interchangeably herein. The "polypeptides," "proteins" and "peptides" encoded by the "polynucleotide sequences," include full-length native sequences, as with naturally occurring proteins, as well as functional subsequences, modified forms or sequence variants so long as the subsequence, modified form or variant retains some degree of functionality of the native full-length protein. In in the instant invention, such polypeptides, proteins and peptides encoded by the polynucleotide sequences can be, but are not required to be, identical to the endogenous protein that is defective, or whose expression is insufficient, or deficient in the treated mammal.

In certain embodiments, the heterologous polynucleotide encodes an inhibitory nucleic acid selected from the group consisting of a siRNA, an antisense molecule, miRNA, RNAi, a ribozyme and a shRNA.

In certain embodiments, an inhibitory nucleic acid binds to a gene, a transcript of a gene, or a transcript of a gene associated with a polynucleotide repeat disease selected from the group consisting of a huntingtin (HTT) gene, a gene associated with dentatorubropallidoluysian atrophy (atrophin 1, ATN1), androgen receptor on the X chromosome in spinobulbar muscular atrophy, human Ataxin-1, -2, -3, and -7, Cav2.1 P/Q voltage-dependent calcium channel (CACNA1A), TATA-binding protein, Ataxin 8 opposite strand (ATXN8OS), Serine/threonine-protein phosphatase 2A 55 kDa regulatory subunit B beta isoform in spinocerebellar ataxia (type 1, 2, 3, 6, 7, 8, 12 17), FMR1 (fragile X mental retardation 1) in fragile X syndrome, FMR1 (fragile X mental retardation 1) in fragile X-associated tremor/ataxia syndrome, FMR1 (fragile X mental retardation 2) or AF4/FMR2 family member 2 in fragile XE mental retardation; Myotonin-protein kinase (MT-PK) in myotonic dystrophy; Frataxin in Friedreich's ataxia; a mutant of superoxide dismutase 1 (SOD1) gene in amyotrophic lateral sclerosis; a gene involved in pathogenesis of Parkinson's disease and/or Alzheimer's disease; apolipoprotein B (APOB) and proprotein convertase subtilisin/kexin type 9 (PCSK9), hypercholesterolemia; HIV Tat, human immunodeficiency virus transactivator of transcription gene, in HIV infection; HIV TAR, HIV TAR, human immunodeficiency virus transactivator response element gene, in HIV infection; C-C chemokine receptor (CCR5) in HIV infection; Rous sarcoma virus (RSV) nucleocapsid protein in RSV infection, liver-specific microRNA (miR-122) in hepatitis C virus infection; p53, acute kidney injury or delayed graft function kidney transplant or kidney injury acute renal failure; protein kinase N3 (PKN3) in advance recurrent or metastatic solid malignancies; LMP2, LMP2 also known as proteasome subunit beta-type 9 (PSMB 9), metastatic melanoma; LMP7,also known as proteasome subunit beta-type 8 (PSMB 8), metastatic melanoma; MECL1 also known as proteasome subunit beta-type 10 (PSMB 10), metastatic melanoma; vascular endothelial growth factor (VEGF) in solid tumors; kinesin spindle protein in solid tumors, apoptosis suppressor B-cell CLL/lymphoma (BCL-2) in chronic myeloid leukemia; ribonucleotide reductase M2 (RRM2) in solid tumors; Furin in solid tumors; polo-like kinase 1 (PLK1) in liver tumors, diacylglycerol acyltransferase 1 (DGAT1) in hepatitis C infection, beta-catenin in familial adenomatous polyposis; beta2 adrenergic receptor, glaucoma; RTP801/Redd1 also known as DAN damage-inducible transcript 4 protein, in diabetic macular edema (DME) or age-related macular degeneration; vascular endothelial growth factor receptor I (VEGFR1) in age-related macular degeneration or choroidal neovascularization, caspase 2 in non-arteritic ischaemic optic neuropathy; Keratin 6A N17K mutant protein in pachyonychia congenital; influenza A virus genome/gene sequences in influenza infection; severe acute respiratory syndrome (SARS) coronavirus genome/gene sequences in SARS infection; respiratory syncytial virus genome/gene sequences in respiratory syncytial virus infection; Ebola filovirus genome/gene sequence in Ebola infection; hepatitis B and C virus genome/gene sequences in hepatitis B and C infection; herpes simplex virus (HSV) genome/gene sequences in HSV infection, coxsackievirus B3 genome/gene sequences in coxsackievirus B3 infection; silencing of a pathogenic allele of a gene (allele-specific silencing) like torsin A (TOR1A) in primary dystonia, pan-class I and HLA-allele specific in transplant; and mutant rhodopsin gene (RHO) in autosomal dominantly inherited retinitis pigmentosa (adRP).

Recombinant viral vector doses can be administered at any appropriate dose. Generally, doses will range from at least 1×10⁸, or more, for example, 1×10⁹, 1×10¹⁰, 1×10¹¹, 1×10¹², 1×10¹³ or 1×10¹⁴, or more, vector genomes per kilogram (vg/kg) of the weight of the subject, to achieve a therapeutic effect. AAV dose in the range of 1×10¹⁰-1×10¹¹ vg/kg in mice, and 1×10¹²-1×10¹³ vg/kg in dogs have been effective. More particularly, a dose from about 1×10¹¹ vg/kg to about 5×10¹⁴ vg/kg inclusive, or from about 5×10¹¹ vg/kg to about 1×10¹⁴ vg/kg inclusive, or from about 5×10¹¹ vg/kg to about 5×10¹³ vg/kg inclusive, or from about 5×10¹¹ vg/kg to about 1×10¹³ vg/kg inclusive, or from about 5×10¹¹ vg/kg or about 5×10¹² vg/kg inclusive, or from about 5×10¹¹ vg/kg to about 1×10¹² vg/kg inclusive. Doses can be, for example, about 5×10¹⁴ vg/kg, or less than about 5×10¹⁴ vg/kg, such as a dose from about 2×10¹¹ to about 2×10¹⁴ vg/kg inclusive, in particular, for example, about 2×10¹² vg/kg, about 6×10¹² vg/kg, or about 2×10¹³ vg/kg.

In certain embodiments, administration of a protease and/or glycosidase to a subject reduces the dose of a recombinant viral vector comprising a heterologous polynucleotide required to be effective for treatment of a subject. In certain embodiments, administration of a protease and/or glycosidase to a subject allows for administration of an increased dose of a recombinant viral vector comprising a heterologous polynucleotide.

Doses can vary and depend upon the type, onset, progression, severity, frequency, duration, or probability of the disease to which treatment is directed, the clinical endpoint desired, previous or simultaneous treatments, the general health, age, gender, race or immunological competency of the subject and other factors that will be appreciated by the skilled artisan. The dose amount, number, frequency or duration may be proportionally increased or reduced, as indicated by any adverse side effects, complications or other risk factors of the treatment or therapy and the status of the subject. The skilled artisan will appreciate the factors that may influence the dosage and timing required to provide an amount sufficient for providing a therapeutic or prophylactic benefit.

The dose to achieve a therapeutic effect, *e.g.,* the dose in vector genomes/per kilogram of body weight (vg/kg), will vary based on several factors including, but not limited to: route of administration, the level of heterologous polynucleotide expression required to achieve a therapeutic effect, the specific disease treated, any host immune response to the recombinant viral vector, a host immune response to the heterologous polynucleotide or expression product (protein or peptide), and the stability of the protein or peptide expressed. One skilled in the art can determine a recombinant viral vector genome dose range to treat a patient having a particular disease or disorder based on the aforementioned factors, as well as other factors.

An "effective amount" or "sufficient amount" refers to an amount that provides, in single or multiple doses, alone or in combination, with one or more other compositions, treatments, protocols, or therapeutic regimens agents, a detectable response of any duration of time (long or short term), an expected or desired outcome in or a benefit to a subject of any measurable or detectable degree or for any duration of time *(e.g.,* for minutes, hours, days, months, years, or cured). The doses of an "effective amount" or "sufficient amount" for treatment (e.g., to ameliorate or to provide a therapeutic benefit or improvement) typically are effective to provide a response to one, multiple or all adverse symptoms, consequences or complications of the disease, one or more adverse symptoms, disorders, illnesses, pathologies, or complications, for example, caused by or associated with the disease, to a measurable extent, although decreasing, reducing, inhibiting, suppressing, limiting or controlling progression or worsening of the disease is a satisfactory outcome.

An effective amount or a sufficient amount can but need not be provided in a single administration, may require multiple administrations, and, can but need not be, administered alone or in combination with another composition (e.g., agent), treatment, protocol or therapeutic regimen. For example, the amount may be proportionally increased as indicated by the need of the subject, type, status and severity of the disease treated or side effects (if any) of treatment. In addition, an effective amount or a sufficient amount need not be effective or sufficient if given in single or multiple doses without a second composition (e.g., another drug or agent), treatment, protocol or therapeutic regimen, since additional doses, amounts or duration above and beyond such doses, or additional compositions (e.g., drugs or agents), treatments, protocols or therapeutic regimens may be included in order to be considered effective or sufficient in a given subject. Amounts considered effective also include amounts that result in a reduction of the use of another treatment, therapeutic regimen or protocol, such as administration of recombinant GAA for treatment of a lysosomal storage disease (e.g., Pompe disease), or administration of a recombinant clotting factor protein *(e.g.,* FVIII or FIX) for treatment of a clotting disorder *(e.g.,* hemophilia A (HemA) or hemophilia B (HemB)).

For Pompe disease, an effective amount would be an amount of GAA that inhibits or reduces glycogen production or accumulation, enhances or increases glycogen degradation or removal, reduces lysosomal alterations in tissues of the body of a subject, or improves muscle tone and/or muscle strength and/or respiratory function in a subject, for example. Effective amounts can be determined, for example, by ascertaining the kinetics of GAA uptake by myoblasts from plasma. Myoblasts GAA uptake rates (*K uptake*) of about 141-147 nM may appear to be effective (see, *e.g.,* Maga et al., J. Biol. Chem. 2012) In animal models, GAA activity levels in plasma of greater than about 1,000 nmol/hr/mL, for example, about 1,000 to about 2,000 nmol/hr/mL have been observed to be therapeutically effective.

For HemA and HemB, generally speaking, it is believed that, in order to achieve a therapeutic effect, a blood coagulation factor concentration that is greater than 1% of factor concentration found in a normal individual is needed to change a severe disease phenotype to a moderate one. A severe phenotype is characterized by joint damage and life-threatening bleeds. To convert a moderate disease phenotype into a mild one, it is believed that a blood coagulation factor concentration greater than 5% of normal is needed.

FVIII and FIX levels in normal humans are about 150-200 ng/mL plasma, but may be less *(e.g.,* range of about 100-150 ng/mL) or greater *(e.g.,* range of about 200-300 ng/mL) and still considered normal, due to functional clotting as determined, for example, by an activated partial thromboplastin time (aPTT) one-stage clotting assay. Thus, a therapeutic effect can be achieved such that the total amount of FVIII or FIX in the subject/human is greater than 1% of the FVIII or FIX present in normal subjects/humans, *e*.*g*., 1% of 100-300 ng/mL.

The composition can be administered to a subject as a combination composition, or administered separately, such as concurrently or in series or sequentially (prior to or following) delivery or administration of a recombinant viral vector comprising a heterologous polynucleotide. The instant invention provides combinations in which a method or use of the instant invention is in a combination with any compound, agent, drug, therapeutic regimen, treatment protocol, process, remedy or composition, set forth herein or known to one of skill in the art. The compound, agent, drug, therapeutic regimen, treatment protocol, process, remedy or composition can be administered or performed prior to, substantially contemporaneously with or following administration of a recombinant viral vector comprising a heterologous polynucleotide, to a subject.

Accordingly, the instant invention includes, *inter alia,* methods and uses that result in a reduced need or use of another compound, agent, drug, therapeutic regimen, treatment protocol, process, or remedy. For example, for a blood clotting disease, a method of treatment according to the instant invention has a therapeutic benefit if in a given subject a less frequent or reduced dose or elimination of administration of a recombinant clotting factor protein to supplement for the deficient or defective (abnormal or mutant) endogenous clotting factor in the subject. In another example, for a lysosomal storage disease, such as Pompe disease, a methods of treatment according to the instant invention has a therapeutic benefit even if a less frequent or reduced dose of a recombinant viral vector comprising GAA has been previously administered, or continues to be administered to a subject. Thus, reducing the need for, or the use of, another treatment or therapy is included in the instant invention.

An effective amount or a sufficient amount need not be effective in each and every subject treated, nor a majority of treated subjects in a given group or population. An effective amount or a sufficient amount means effectiveness or sufficiency in a particular subject, not a group or the general population. As is typical for such methods, some subjects will exhibit a greater response, or less or no response to a given treatment method or use.

The term "ameliorate" means a detectable or measurable improvement in a subject's disease or symptom thereof, or an underlying cellular response. A detectable or measurable improvement includes a subjective or objective decrease, reduction, inhibition, suppression, limit or control in the occurrence, frequency, severity, progression, or duration of the disease, or complication caused by or associated with the disease, or an improvement in a symptom or an underlying cause or a consequence of the disease, or a reversal of the disease. For Pompe, an effective amount would be an amount that inhibits or reduces glycogen production or accumulation, enhances or increases glycogen degradation or removal, improves muscle tone and/or muscle strength and/or respiratory function, for example. For HemA or HemB, an effective amount would be an amount that reduces frequency or severity of acute bleeding episodes in a subject, for example, or an amount that reduces clotting time as measured by a clotting assay, for example.

Accordingly, pharmaceutical compositions of the instant invention include compositions wherein the active ingredients are contained in an effective amount to achieve the intended therapeutic purpose. Determining a therapeutically effective dose is well within the capability of a skilled medical practitioner using techniques and guidance known in the art and using the teachings provided herein.

Therapeutic doses will depend on, among other factors, the age and general condition of the subject, the severity of the aberrant phenotype, and the strength of the control sequences regulating expression levels. Thus, a therapeutically effective amount in humans will fall in a relatively broad range that may be determined by a medical practitioner based on the response of an individual patient to a vector-based treatment. Such doses may be alone or in combination with an immunosuppressive agent or drug.

Compositions such as pharmaceutical compositions may be delivered to a subject, so as to allow transgene expression and optionally production of encoded protein. In certain embodiments, pharmaceutical compositions comprising sufficient genetic material to enable a subject to produce a therapeutically effective amount of a blood-clotting factor to improve hemostasis in the subject. In certain embodiments, pharmaceutical compositions comprising sufficient heterologous polynucleotide to enable a subject to produce a therapeutically effective amount of GAA.

In certain embodiments, a therapeutic effect in a subject is sustained for a period of time, *e.g.,* 2-4, 4-6, 6-8, 8-10, 10-14, 14-20, 20-25, 25-30, or 30-50 days or more, for example, 50-75, 75-100, 100-150, 150-200 days or more. Accordingly, in certain embodiments, a recombinant viral vector provides a therapeutic effect.

In certain embodiments, a recombinant viral vector provides a therapeutic effect without an immunosuppressive agent. In certain embodiments, at least one immunosuppressive agent is administered to a subject prior to, substantially contemporaneously with or after administration of a recombinant viral vector to the subject.

In certain embodiments, an immunosuppressive agent is an anti-inflammatory agent. In certain embodiments, an immunosuppressive agent is a steroid. In certain embodiments, an immunosuppressive agent is prednisone, cyclosporine (e.g., cyclosporine A), mycophenolate, rituximab, rapamycin or a derivative thereof. Additional particular agents include a stabilizing compound. Other immunosuppressive agents that can be used in methods according to the instant invention include, for example and without limitation, a B cell targeting antibody, *e*.*g*., rituximab; a proteasome inhibitor, *e.g.,* bortezomib; a mammalian target of rapamycin (mTOR) inhibitor, *e.g.,* rapamycin; a tyrosine kinase inhibitor, *e*.*g*., ibrutinib; an inhibitor of B-cell activating factor (BAFF); and an inhibitor of a proliferation-inducing ligand (APRIL).

Compositions may be administered in any sterile, biocompatible pharmaceutical carrier, including, but not limited to, saline, buffered saline, dextrose, and water. The compositions may be administered to a patient alone, or in combination with other agents, which influence dosage amount, administration frequency and/or therapeutic efficacy.

Methods and uses of the instant invention include delivery and administration systemically, regionally or locally, or by any route, for example, by injection or infusion. Delivery of the pharmaceutical compositions *in vivo* may generally be accomplished via injection using a conventional syringe, although other delivery methods such as convection-enhanced delivery are envisioned (See *e.g.,* U.S. Pat. No. 5,720,720). For example, compositions may be delivered subcutaneously, epidermally, intradermally, intrathecally, intraorbitally, intramucosally, intraperitoneally, intravenously, intra-pleurally, intraarterially, orally, intrahepatically, via the portal vein, or intramuscularly. Other modes of administration include oral and pulmonary administration, suppositories, and transdermal applications. A clinician specializing in the treatment of patients with blood coagulation disorders may determine the optimal route for administration of the adenoviral-associated vectors based on a number of criteria, including, but not limited to: the condition of the patient and the purpose of the treatment (e.g., increased GAA, enhanced blood coagulation, etc.).

Methods of treatment according to the instant invention include combination therapies that include the additional use of any compound, agent, drug, treatment or other therapeutic regimen or protocol having a desired therapeutic, beneficial, additive, synergistic or complementary activity or effect. Exemplary combination compositions and treatments include second actives, such as, biologics (proteins), agents (e.g., immunosuppressive agents) and drugs. Such biologics (proteins), agents, drugs, treatments and therapies can be administered or performed prior to, substantially contemporaneously with or following any other method of treatment according to the instant invention, for example, a therapeutic method of treating a subject for a lysosomal storage disease such as Pompe, or a therapeutic method of treating a subject for a blood clotting disease such as HemA or HemB.

The compound, agent, drug, treatment or other therapeutic regimen or protocol can be administered as a combination composition, or administered separately, such as concurrently or in series or sequentially (prior to or following) delivery or administration of a nucleic acid, vector, recombinant vector (e.g., recombinant viral vector), or recombinant virus particle. The instant invention therefore provides combinations in which a method of treatment according to the instant invention is in a combination with any compound, agent, drug, therapeutic regimen, treatment protocol, process, remedy or composition, set forth herein or known to one of skill in the art. The compound, agent, drug, therapeutic regimen, treatment protocol, process, remedy or composition can be administered or performed prior to, substantially contemporaneously with or following administration of a nucleic acid, vector, recombinant vector (e.g., recombinant viral vector), or recombinant virus particle administered to a patient according to the instant invention.

In certain embodiments, administration of a protease and/or glycosidase to a subject may lead to prevention of development of neutralizing antibodies, antibodies that bind to the heterologous polynucleotide and/or antibodies that bind to a protein or peptide encoded by the heterologous polynucleotide. As set forth herein, administration of the protease and/or glycosidase to such a subject can be prior to administration of a viral vector, substantially contemporaneously at the time of administration of a viral vector, or after administration of a viral vector to the subject.

In certain embodiments, administration of a protease and/or glycosidase to a subject with pre-existing antibodies leads to reduction of neutralizing antibodies, antibodies that bind to the heterologous polynucleotide and/or antibodies that bind to a protein or peptide encoded by the heterologous polynucleotide. After administration of the protease and/or glycosidase, such subjects can then be administered a recombinant viral vector in accordance with the methods herein. Such subjects may optionally be evaluated for presence of remaining pre-existing antibodies after administration of a recombinant viral vector. Alternatively, such subjects can be administered the recombinant viral vector after a predetermined amount of time has passed during which the protease and/or glycosidase reduces or eliminates any such pre-existing antibodies in the subject.

In certain embodiments, administration of a protease and/or glycosidase to a subject may lead to degradation or digestion of at least 20% to 50%, at least 55%, at least 60%, at least 65%, at least 70%, at least 75%, at least 80%, at least 85%, at least 90%, at least 95%, at least 98%, at least 99%, or 100% of neutralizing antibodies, antibodies that bind to the heterologous polynucleotide and/or antibodies that bind to a protein or peptide encoded by the heterologous polynucleotide, as reflected by measurement of such antibodies in a biological sample obtained from a subject administered a recombinant viral vector. In certain embodiments, a method according to the instant invention degrades or digests at least 80%, at least 85%, at least 90%, at least 95%, at least 98%, at least 99%, or 100% of the neutralizing antibodies, and/or antibodies that bind to the heterologous polynucleotide and/or antibodies that bind to a protein or peptide encoded by the heterologous polynucleotide.

Non-limiting examples of a biological sample from a subject that may be analyzed include whole blood, serum, plasma, the like, and a combination thereof. A biological sample may be devoid of cells, or may include cells *(e.g.,* red blood cells, platelets and/or lymphocytes).

In certain embodiments, neutralizing antibodies present in a biological sample of a subject may be degraded or digested to less than about 1:25, where 1 part of the biological sample diluted in 25 of buffer results in 50% recombinant viral vector neutralization. In certain embodiments, neutralizing antibodies present in a biological sample of the subject may be degraded or digested to less than about 1:20, less than about 1:15, less than about 1:10, less than about 1:5, less than about 1:4, less than about 1:3, less than about 1:2, or less than about 1:1, where 1 part of the biological sample diluted in 20, 15, 10, 5, 4, 3, 2, or 1 part, respectively, of buffer results in 50% recombinant viral vector neutralization.

Exemplary analysis and measurement of AAV neutralizing antibodies in a biological sample is disclosed herein and also described in U.S. Patent Application Publication 2016/0123990. Antibody binding to Fc receptor can be measured by determining the equilibrium binding constant. Reduction in Fc receptor binding of an antibody is determined by an increase in the equilibrium binding constant for the IgG:FcR interaction.

Methods according to the instant invention are applicable to both loss of function and gain and function genetic defects. The term "loss-of-function" in reference to a genetic defect as used herein, refers to any mutation in a gene in which the protein encoded by said gene *(i.e.,* the mutant protein) exhibits either a partial or a full loss of function that is normally associated with the wild-type protein. The term "gain-of-function" in reference to a genetic defect as used herein, refers to any mutation in a gene in which the protein encoded by said gene *(i.e.,* the mutant protein) acquires a function not normally associated with the protein *(i.e.,* the wild type protein) causes or contributes to a disease or disorder. The gain-of-function mutation can be a deletion, addition, or substitution of a nucleotide or nucleotides in the gene, which gives rise to the change in the function of the encoded protein. In certain embodiments, the gain-of-function mutation changes the function of the mutant protein or causes interactions with other proteins. In certain embodiments, the gain-of-function mutation causes a decrease in or removal of normal wild-type protein, for example, by interaction of the altered, mutant protein with said normal, wild-type protein.

Diseases and disorders that may be treated by methods according to the instant invention include, for example and without limitation, lung disease (e.g., cystic fibrosis), a bleeding disorder *(e.g.,* hemophilia A or hemophilia B with or without inhibitors), thalassemia, a blood disorder (*e.g.,* anemia), Alzheimer's disease, Parkinson's disease, Huntington's disease, amyotrophic lateral sclerosis (ALS), epilepsy, a lysosomal storage disease )*e*.*g*., aspartylglucosaminuria, Batten disease, late infantile neuronal ceroid lipofuscinosis type 2 (CLN2), cystinosis, Fabry disease, Gaucher disease types I, II, and III, glycogen storage disease II (Pompe disease), GM2-gangliosidosis type I (Tay Sachs disease), GM2-gangliosidosis type II (Sandhoff disease), mucolipidosis types I (sialidosis type I and II), II (I-cell disease), III (pseudo-Hurler disease) and IV, mucopolysaccharide storage diseases (Hurler disease and variants, Hunter, Sanfilippo Types A,B,C,D, Morquio Types A and B, Maroteaux-Lamy and Sly diseases), Niemann-Pick disease types A/B, C1 and C2, and Schindler disease types I and II), hereditary angioedema (HAE), a copper or iron accumulation disorder (e.g., Wilson's or Menkes disease), lysosomal acid lipase deficiency, a neurological or neurodegenerative disorder, cancer, type 1 or type 2 diabetes, adenosine deaminase deficiency, a metabolic defect *(e.g.,* glycogen storage diseases), a disease of solid organs *(e.g.,* brain, liver, kidney, heart), or an infectious viral *(e.g.,* hepatitis B and C, HIV, etc.), bacterial or fungal disease.

Glycogen storage disease type II, also called Pompe disease may be treated by methods according to the instant invention. Pompe disease is an autosomal recessive disorder caused by mutations in the gene encoding the lysosomal enzyme acid α-glucosidase (GAA), which catalyzes the degradation of glycogen. The resulting enzyme deficiency leads to pathological accumulation of glycogen and lysosomal alterations in all tissues of the body, resulting in cardiac, respiratory, and skeletal muscle dysfunction (van der Ploeg & Reuser, 2008).

Blood clotting disorders which may be treated by methods according to the instant invention, include, for example and without limitation, hemophilia A, hemophilia A with inhibitory antibodies, hemophilia B, hemophilia B with inhibitory antibodies, a deficiency in any coagulation Factor: VII, VIII, IX, X, XI, V, XII, II, von Willebrand factor, or a combined FV/FVIII deficiency, thalassemia, vitamin K epoxide reductase C1 deficiency or gamma-carboxylase deficiency.

Other diseases and disorders that may be treated by methods according to the instant invention include, for example and without limitation, anemia, bleeding associated with trauma, injury, thrombosis, thrombocytopenia, stroke, coagulopathy, disseminated intravascular coagulation (DIC); over-anticoagulation associated with heparin, low molecular weight heparin, pentasaccharide, warfarin, small molecule antithrombotics *(i.e.,* FXa inhibitors), or a platelet disorder such as, Bernard Soulier syndrome, Glanzmann thrombasthenia, or storage pool deficiency.

Other diseases and disorders that may be treated by methods according to the instant invention include, for example and without limitation, proliferative diseases (cancers, tumors, dysplasias, etc.), Crigler-Najjar and metabolic diseases like metabolic diseases of the liver, Friedreich ataxia, infectious diseases, viral diseases (induced, *e.g.,* by hepatitis B or C viruses, HIV, herpes, retroviruses, etc.), genetic diseases (cystic fibrosis, dystroglycanopathies, myopathies such as Duchenne muscular myopathy or dystrophy, myotubular myopathy, sickle-cell anemia, sickle cell disease, Fanconi's anemia, diabetes, amyotrophic lateral sclerosis (ALS), myotubularin myopathy, motor neuron diseases such as spinal muscular atrophy (SMA), spinobulbar muscular atrophy, or Charcot-Marie-Tooth disease, arthritis, severe combined immunodeficiencies (such as RS-SCID, ADA-SCID or X-SCID), Wiskott-Aldrich syndrome, X-linked thrombocytopenia, X-linked congenital neutropenia, chronic granulomatous disease, etc.), clotting factor deficiencies, cardiovascular disease (restenosis, ischemia, dyslipidemia, homozygous familial hypercholesterolemia, etc.), eye diseases such as retinitis pigmentosa, Leber congenital amaurosis, Leber hereditary optic neuropathy, and Stargardt disease; lysosomal storage diseases such as San Filippo syndrome; hyperbilirubinemia such as CN type I or II or Gilbert's syndrome, glycogen storage disease such as GSDI, GSDII (Pompe disease), GSDIII, GSDIV, GSDV, GSDVI, GSDVII, GSDVIII or lethal congenital glycogen storage disease of the heart.

In certain embodiments, the subject has a disease that affects or originates in the central nervous system (CNS). In certain embodiments, the disease is a neurodegenerative disease. Non-limiting examples of CNS or neurodegenerative disease include Alzheimer's disease, Huntington's disease, ALS, hereditary spastic hemiplegia, primary lateral sclerosis, spinal muscular atrophy, Kennedy's disease, a polyglutamine repeat disease, or Parkinson's disease. In certain embodiments, the disease is a psychiatric disease, an addition *(e.g.,* to tobacco, alcohol, or drugs), epilepsy, Canavan's disease, or adrenoleukodystrophy. In certain embodiments, the CNS or neurodegenerative disease is a polyglutamine repeat disease such as, for example and without limitation, spinocerebellar ataxia (SCA1, SCA2, SCA3, SCA6, SCA7, or SCA17).

The instant invention may be used in human and veterinary medical applications. Suitable subjects therefore include mammals, such as humans, as well as non-human mammals. The term "subject" refers to an animal, typically a mammal, such as humans, non-human primates (apes, gibbons, gorillas, chimpanzees, orangutans, macaques), a domestic animal (dogs and cats), a farm animal (poultry such as chickens and ducks, horses, cows, goats, sheep, pigs), and experimental animals (mouse, rat, rabbit, guinea pig). Human subjects include fetal, neonatal, infant, juvenile and adult subjects. Subjects also include animal disease models, for example, mouse and other animal models of protein/enzyme deficiencies such as Pompe disease (loss of GAA), and glycogen storage diseases (GSDs) and others known to those of skill in the art.

The instant invention provides compositions, such as kits, that include packaging material and one or more components therein. A kit typically includes a label or packaging insert including a description of the components or instructions for use in vitro, in vivo, or ex vivo, of the components therein. A kit can contain a collection of such components, *e*.*g*., a nucleic acid, recombinant vector, virus (e.g., AAV, lentivirus) vector, or virus particle and a protease and/or glycosidase that degrades or digests antibodies.

A kit refers to a physical structure housing one or more components of the kit. Packaging material can maintain the components sterilely, and can be made of material commonly used for such purposes (e.g., paper, corrugated fiber, glass, plastic, foil, ampules, vials, tubes, etc.).

Labels or inserts can include identifying information of one or more components therein, dose amounts, clinical pharmacology of the active ingredient(s) including mechanism of action, pharmacokinetics and pharmacodynamics. Labels or inserts can include information identifying manufacturer, lot numbers, manufacture location and date, expiration dates. Labels or inserts can include information identifying manufacturer information, lot numbers, manufacturer location and date. Labels or inserts can include information on a disease for which a kit component may be used. Labels or inserts can include instructions for the clinician or subject for using one or more of the kit components in a method, use, or treatment protocol or therapeutic regimen. Instructions can include dosage amounts, frequency or duration, and instructions for practicing any of the methods, uses, treatment protocols or prophylactic or therapeutic regimes described herein.

Labels or inserts can include information on any benefit that a component may provide, such as a prophylactic or therapeutic benefit. Labels or inserts can include information on potential adverse side effects, complications or reactions, such as warnings to the subject or clinician regarding situations where it would not be appropriate to use a particular composition. Adverse side effects or complications could also occur when the subject has, will be or is currently taking one or more other medications that may be incompatible with the composition, or the subject has, will be or is currently undergoing another treatment protocol or therapeutic regimen which would be incompatible with the composition and, therefore, instructions could include information regarding such incompatibilities.

Labels or inserts include "printed matter," *e*.*g*., paper or cardboard, or separate or affixed to a component, a kit or packing material (e.g., a box), or attached to an ampule, tube or vial containing a kit component.

A number of embodiments of the instant invention have been described. Nevertheless, one skilled in the art, without departing from the spirit and scope of the instant invention, can make various changes and modifications of the instant invention to adapt it to various usages and conditions. Accordingly, the following examples are intended to illustrate but not limit the scope of the instant invention claimed in any way.

### Examples

### EXAMPLE 1

### Materials & Methods

### AAV vectors

AAV vectors were prepared as previously described (Ayuso et al. (2010) Gene Ther. 17, 503-10). Genome-containing vectors were produced in roller bottles following a triple transfection protocol with cesium chloride gradient purification (Ayuso *et al.* (2010)). The AAV vector titration was performed by real time PCR (qPCR) performed in ABI PRISM 7900 HT Sequence Detector using Absolute ROX mix (Taqman, Thermo Fisher Scientific, Waltham, MA), except for empty capsids that were titrated by SDS-PAGE followed by silver staining and quantification by densitometry against an AAV capsid used as a standard. For the *in vitro* neutralization assay, an AAV8 vector encoding luciferase (AAV8-Luc) was used as reporter as previously described (Miao et al. (2006) Blood. 108, 19-27). The AAV vectors used in the *in vivo* experiments expressed human FIX or *Gaussia* luciferase, under the control of a liver-specific promoter (Manno *et al.* (2006); Mingozzi et al. (2013) Gene Ther. 20, 417-24).

### Design and production of IdeS endopeptidase

The IdeS coding sequence(CDS) was cloned into a pEX-N-His-tagged cloning vector (OriGene Technologies, Inc., Rockville MD), which was transformed into BL21 competent *E. coli* (NEB, Ipswich, MA) following the manufacturer's instructions. Protein expression was induced at 37 °C for 4 hr by adding IPTG 0.5 mM to the bacterial culture. Detection and purification of the protein were performed using the anti-FabRICATOR antibody A3-AF1-010 (Genovis) and a nickel affinity column (GE Healthcare), respectively. Endotoxin removal was performed using poly(ε-lysine) resin spin columns (Thermo Scientific, Waltham, MA) and residual endotoxin concentration was assessed by LAL test with Endosafe Cartridge Technology (Charles River Laboratories), according to manufacturer's instructions. IdeS endopeptidase was diluted in the injectable solution buffer, composed of 50 mM sodium phosphate, 150 mM NaCl, 0.25% human albumin, pH 6.6.

### Cell culture

The 2V6.11 cells (ATCC CRL-2784) were maintained under 37 °C, 5% CO2 condition in Dulbecco's modified Eagle's Medium (DMEM) supplemented with 10% FBS, 2mM GlutaMAX (Invitrogen Life Technology, Carlsbad, CA) and have been used for the AAV neutralization assay.

### In vitro digestion of IgG by IdeS

Serum from AAV8 seropositive human (NAb titer: 1:31.6) and monkey (NAb titer: 1:100) or IVIg (NAb titer: 1:316) was diluted in PBS to 4-4.5 mg/mL IgG. Fifty microliters of diluted IgG (200 µg) was incubated with IdeS (100 IU, 50 IU/µL) or PBS for 22 hr at 37 °C. Anti-AAV8 IgG were measured by ELISA and in a neutralization assay. In the case of monkey IgG, an antibody specific for gamma chain of monkey IgG conjugated with horseradish peroxidase (HRP) (Fitzgerald, Acton, MA USA) was used.

### Anti-human IgG ELISA

ELISA plates were coated with a goat anti-human kappa antibody (2.5 µg/mL in PBS) and blocked with PBS-1% bovine serum albumin (BSA). IgG samples were incubated in 1/10 dilutions, using a commercial IVIg preparation as a standard. Intact IgG were then revealed using a secondary HRP-conjugated mouse anti-human IgG-Fc antibody and the o-phenylenediamine dihydrochloride (OPD) substrate.

### AAV antibody assay

Anti-AAV IgG capture assay was performed as previously described (Mingozzi et al. (2013) Sci Transl Med. 5, 194ra92). Briefly, recombinant AAV empty capsids were diluted in coating buffer (35 mM bicarbonate, 13 mM carbonate, pH> 9.2) to a final concentration of 2×10¹⁰ vector particles per mL. Fifty microliters were added to each well in a 96-well Nunc Maxisorp Immunoplate (Thermofisher, Waltham, USA). A standard curve made from purified human IgG (Tégéline, LFB, France) was added directly to the plates. Plates were coated overnight at 4 °C. The next day, plates were washed three times with wash buffer (PBS, 0.05% Tween-20) then blocked with blocking buffer (PBS, 6% fat-free milk) for 2 hr at room temperature. Plates were again washed three times with wash buffer (PBS, 0.05% Tween-20) then incubated with serum diluted in blocking buffer, 1 hr at 37 °C After 3 washes, antibody specific for heavy chain of human IgG conjugated with HRP (Southern Biotech, Birmingham, USA) was incubated 1 hr at 37 °C. After incubation, plates were washed three times with wash buffer and revealed with OPD substrate (Sigma, Saint Louis, USA). The reaction was stopped with H₂SO₄ 3M solution and optical density (OD) measurements were done at 492 nm using a microplate reader (ENSPIRE^{™}, Perkin Elmer, Waltham, USA). Anti-AAV IgG concentration was determined against the specific standard curve using 4-parameters regression and results were expressed as µg/mL of IgG. In the case NHP studies, a purified monkey IgG for the standard curve and an antibody specific for gamma chain of monkey IgG conjugated with Horseradish Peroxidase (HRP) (Fitzgerald, Acton, MA USA) for the revelation, have been used.

### AAV neutralization assay

The NAb assay was performed as previously described (Meliani et al. (2015) Hum Gene Ther Methods. 26, 45-53). Briefly, on day 1, 96-well plates were seeded with 2×10⁴ of 2V6.11 cells/well for 24 hr in the presence of ponasterone A (Life Technologies, Carlsbad, USA). Recombinant AAV8-CMV-Luciferase was diluted in serum-free DMEM (Life Technologies, Carlsbad, USA) and incubated with semi-log-fold serial dilutions of the serum samples, and then incubated for 1 hr at 37 °C. Subsequently, the serum-vector mixtures were added in the cells. Each dilution was tested in duplicate or triplicate. After 24 hr, cells were lysed and the luciferase activity was measured on a luminometer (ENSPIRE^{™}, Perkin Elmer, Waltham, USA). Transduction efficiency was measured as Relative Light Unit (RLU) per second. The neutralizing titer was reported as the highest serum dilution that inhibited AAV transduction by ≥50% compared with the control without serum (100% transduction).

### Mouse studies

Mouse studies were performed according to the French and European legislation on animal care and experimentation (2010/63/EU and CE12-037) and approved by the local institutional ethical committee (Protocols n 2017-014-B).

Male C57BL/6 mice aged 6-8 weeks or male and female factor IX (FIX)-deficient Hemophilia B (HB) mice were used. In all experiments, animals were passively immunized by intravenous injection with 9 mg of IVIg (PRIVIGEN, CSL Behring, US-PA) in a final volume of 100 µL. After 30 minutes, mice received 250 UI, 500 UI or 1250 UI of IdeS (Promega, Madison, US-WI) by retro-orbital injection. Next day, 30 hr after IdeS injection, all mice were injected with an AAV8 vector at the dose of 1×10¹² vg/kg (2×10¹⁰ vg/mouse) by tail vein route. Blood samples (D-7, D+7, D+14 and D+28) were collected from the retro-orbital plexus in heparin coated capillary tubes (Sarstedt, Nümbrecht, Germany) or from mandibular way for samples D-1+15mn and D0. At euthanasia, whole livers were collected and snap-frozen for subsequent vector genome copy number assessment.

### NHP study

All procedures using NHP were approved by the institutional animal care and use committee (protocol number 2018120409565637, APAFIS 18092) and were conducted at Nantes veterinary school (Oniris, Nantes, France). Two cynomolgus monkeys (*Macaca facscicuiaris,* 2-3 years old), were selected based on their anti-AAV8 neutralizing antibody titer (between 1:10 to 1:31.6). At day -2, one animal (NHP2) received intravenously slow infusion of IdeS (500 µg/kg). 24 hr later (D-1), a second injection of IdeS was repeated following the same protocol. Next day, 24 hours after the second IdeS injection, 2 monkeys (NHP1, control animal; NHP2 treated animal) were infused with an AAV8-hFIX vector at the dose of 2×10¹² vg/kg. Blood samples were collected at D-7, D-2, D-2+10h, D-1, D-1+10h, D0 to assess the efficacy of IdeS treatment on degradation of IgG antibodies, before (D-2), during the treatment and before AAV administration (D0). Other blood samples were collected in order to follow up the anti-AAV8 humoral response and transgene expression at days D4, D7, D13, D21, D28, D35, D50. At euthanasia (D50), 4 wedge biopsies taken from the 4 lobes of liver, were collected and snap-frozen for subsequent vector genome copy number investigation.

For the re-administration study, 2 AAV8 seropositive monkeys were infused with 5×10¹² vg/kg of AAV8-hFIX vector (D0). Before vector injection, one monkey received a single injection of IdeS (500 µg/kg) at day D-1. Subsequently, both monkeys received double injections of IdeS (500 µg/kg) at days D80 and D81, and 5×10¹² vg/kg of AAV8-hFIX vector at D82. Blood samples were collected at different time points during the experiment. At euthanasia (D105), liver biopsies were collected from 4 lobes for a VGCN assessment.

### Anti-monkey IgG ELISA

Monkey IgG levels were measured by sandwich enzyme linked immunoassay (ELISA). Briefly, we used a goat anti-human IgG F(ab')₂ (Thermo Scientific) and a goat anti-monkey IgG-HRP (Fitzgerald) as capture and detection antibodies, respectively. OPD Peroxidase Substrate System (SIGMAFAST^{™}-OPD, Sigma) was used for revelation.

### Human FIX ELISA

Human FIX (hFIX) protein levels in mouse plasma were measured following manufacturer's instructions. The capture and secondary HRP-antibodies for the ELISA assay were purchased from Affinity Biological (Ancaster, Canada). In NHP samples, an anti-hFIX antibody (MAI-43012, Thermo Fisher Scientific, Waltham, MA) and an anti-hFIX-HRP antibody (CL20040APHP, Tebu-bio, France) were used for coating and detection, respectively. The hFIX antigen levels were determined by adding SIGMAFAST^{™} OPD substrate (Sigma-Aldrich) following the manufacturer's instructions and measuring OD at an absorbance of 492 nm.

### Vector genome copy number (VGCN)

Liver vector genome copy number was determined using a qPCR assay with primers and probes specific for the liver promoter and the mouse Titin or eGlobin genes. The promoter-specific primers and the probe were synthesized by Thermo Scientific (Waltham, MA, USA). Mouse Titin and eGlobin were used as a normalizing gene in mice and NHP studies, respectively, synthesized by Thermo Scientific (Waltham, MA, USA)). DNA from tissues was extracted after whole-organ (mouse) or biopsies (NHP) homogenization using FastPrep machine (MP Biomedicals) and Gentra Puregen Blood Kit (Qiagen, ref 158467). Each sample was tested in triplicate and genome copy number per diploid genome was determined against a standard curve made of linearized plasmid.

### Gaussia luciferase assay

50 µL of plasma samples diluted in PBS (1:50) were loaded on white 96-well microplate (OptiPlate-96 Perkin Elmer, Waltham, USA). *Gaussia* luciferase activity was measured on a luminometer (ENSPIRE^{™}, Perkin Elmer, Waltham, USA). The measurement was done over 1 second after automatic injection of 50 µL of substrate solution (Coelenterazine, Sigma Aldrich). Signal background was subtracted from each value. The *Gaussia* luciferase activity is expressed as RLU/sec and the values are the means +/- SD of duplicates from repeated assays.

### Tail clip bleeding assay

Animals were anesthetized with a mixture of ketamine and xylazine delivered IP and placed in prone position. A distal 3-mm segment of the tail was amputated with a scalpel. The tail was immediately immersed in a 50-mL Falcon tube containing isotonic saline pre-warmed to 37 °C. The position of the tail was vertical with the tip positioned about 2 cm below the body horizon. Each animal was monitored for 20 min even if bleeding ceased, in order to detect any re-bleeding. Bleeding time was determined using a stop clock. If bleeding on/off cycles occurred, the sum of bleeding times within the 20-min period was used. At the end of the experiment, animals were sacrificed by cervical dislocation.

### EXAMPLE 2

### Results with AAV8

We first investigated whether IdeS hydrolyses human IgG *in vivo* following passive immunization of wild-type mice, *i.e.,* in a context where endogenous mouse IgG are present. Wild-type mice were injected with human IgG (IVIg) and 30 min later with IdeS. Intact human IgG were then tested in the serum collected 1, 6 and 24 hours later (Figure 1). Levels of human IgG in non-treated mice demonstrated a progressive decline with time from 9.9±1.2 mg/mL (mean±SD) at 0 hr to 3.7±0.6 mg/mL at 24 hr, concordant with the half-life of human IgG in mice (Roopenian et al. (2003) J Immunol. 170, 3528-33). In contrast, human IgG in IdeS-treated mice demonstrated a rapid elimination from 11.1±1.0 mg/mL at 0 hr to 0.7±0.03 mg/mL at 24 hr. Treatment with IdeS thus resulted in a 5-fold decrease in the concentration of human IgG both 6 and 24 hr after injection.

To investigate the capacity of IdeS to reduce the levels of anti-AAV8 IgG *in vivo,* we used a passive immunization model previously described (Scallan *et al.* (2006)). Wild-type C57BL/6 mice were injected with 9 mg of IVIg. Thirty minutes later, mice were injected with IdeS (250 UI) or PBS as non-treated control group. Thirty hours after IdeS injection, mice were injected with AAV8 vectors encoding the human FIX transgene, which was given at the dose of 1×10¹² vg/kg (Figure 2). Mice were assigned to 4 groups (n=6 mice/group) as outlined in the following table of treatment cohorts:

| Group | IVIg | IdeS | AAV8-hFIX |
|---|---|---|---|
| 1 | + | + | + |
| 2 | + | - | + |
| 3 | - | + | + |
| 4 | - | - | + |

Animals were followed for four weeks after vector delivery and plasma was collected at days -7, 7, 14, 28 in order to measure FIX transgene expression levels (Figure 2). Anti-AAV8 IgG and NAb were measured in serum from blood collected after the reconstitution with human IgG (D-1+15min) and after the injection of IdeS or PBS (D0). As published previously (Scallan *et al.* (2006)), IVIg contained substantial levels of functionally relevant anti-AAV8 IgG, as detected both by ELISA (Figure 3A, 2.56±0.21 µg/mL and 2.83±0.22 µg/mL, means±SD, for Groups 1 and 2, respectively) and by neutralization assays (Figure 3B, 1:22.6 [range: 1:10 to 31.6] titer and 1:19 [range: 1:10 to 31.6], respectively). The injection of IdeS resulted in a drastic decrease in circulating levels of anti-AAV8 IgG. The decrease in anti-AAV8 IgG (0.39 ±0.10 µg/mL and 1.67 ±0.42 µg/mL, for Groups 1 and 2, respectively) and AAV8 NAb was 4.3 and 10 fold, respectively, after 24 hr. The reduced levels of anti-AAV8 IgG overtime in the PBS-treated mice (Group 2) represents the natural elimination of human IgG in mice.

Wild-type mice reconstituted with IVIg or PBS, treated with IdeS or PBS, were then injected with hFIX-encoding AAV8 vectors, and the hFIX plasma levels were measured after 7, 14 and 28 days. As expected, non-reconstituted mice that lack anti-AAV8 IgG demonstrated constant production and accumulation of hFIX in the blood over 4 weeks (Figure 4A). This was accompanied by a substantial gene copy number in the livers of the mice (Figure 4B). In contrast, mice reconstituted with human IgG that contain anti-AAV8 neutralizing IgG (Figure 3A) failed to produce hFIX. Interestingly, the treatment of human IgG reconstituted mice using IdeS was able to restore hFIX production following gene therapy. Levels of hFIX produced in IdeS-treated mice represented 50.31% of the levels produce in control non-reconstituted mice, as assessed by measuring the area under the curve (Figure 4A). Accordingly, the number of gene copies was 2.016±0.620 vg/diploid cell in the liver of human IgG-reconstituted IdeS-treated mice (Figure 4B), while it was 0.005±0.002 vg/diploid cell in human IgG-reconstituted mice that did not receive IdeS (P=0.002).

IdeS hydrolyses IgG from different species including non-human primates. We therefore investigated whether the incubation of IdeS with AAV8-seropositive human and non-human primate (NHP, monkey) serum samples hydrolyses neutralizing anti-AAV8 IgG. As depicted in Figure 5A, IdeS indeed hydrolyzed anti-AAV8 IgG in both the human serum and the monkey serum, with a 15-fold reduction in anti-AAV8 IgG levels in the monkey serum over 22 hr of incubation (0.42±0.01 µg/mL and 6.29±0.10 µg/mL upon treatment with IdeS or PBS, respectively). The anti-AAV8 NAb titer was also reduced in the human serum incubated with IdeS (Figure 5B). No reduction in anti-AAV8 NAb titer was observed upon incubation of monkey serum with IdeS, but there was a decrease of the neutralizing capacity of these antibodies from 76% to 64% after IdeS incubation (Figure 5B). The lack of reduced anti-AAV NAb titer may be due to the fact that functional F(ab')₂ fragments of high affinity monkey anti-AAV8 IgG remaining in the incubation mixture.

### EXAMPLE 3

### Results with AAV2 and AAV9

Anti-AAV2 and anti-AAV9 IgG and NAbs were measured in serum prepared from blood collected after the reconstitution of wild-type mice with 9 mg human IgG (D-1+15min) and after the injection of IdeS (250 IU or 500 IU, for AAV2 or AAV9 respectively) or PBS (D0) (Figure 2). IVIg contained substantial levels of functionally relevant anti-AAV2 and anti-AAV9 IgG, as detected both in ELISA (Figure 3C, anti-AAV2 IgG 9.05±1.20 µg/mL and 8.92±0.63 µg/mL, means±SD; Figure 3E, anti-AAV9 IgG 5.51±0.492 µg/mL and 5.145±0.691 µg/mL, for Groups 1 and 2, respectively) and in a neutralization assay (Figure 3D, 1:886 and 1:772 [range: 1:316 to 1000] anti-AAV2 NAb titer; Figure 3F, 1:15.4 and 1:24.4 [range: 1:10 to 31.6] anti-AAV9 NAb titer, for Groups 1 and 2 respectively). The injection of IdeS resulted in a drastic decrease in circulating levels of anti-AAV2 and AAV9 IgG. The decrease in anti-AAV2 IgG was 6 fold (1.51 ±0.31 µg/mL and 4.58 ±0.45 µg/mL, for Groups 1 and 2, respectively) and in anti-AAV2 NAb was 28 fold, after 24 hr. The decrease in anti-AAV9 IgG was 36 fold (0.153 ±0.074 µg/mL and 2.92 ±0.50 µg/mL, for Groups 1 and 2, respectively) and in anti-AAV9 NAb was 20 fold, after 24 hr. The reduced levels of anti-AAV2 and AAV9 IgG over time in the PBS-treated mice (Group 2) represents the natural elimination of the human IgG in mice.

### EXAMPLE 4

### Results with Gaussia luciferase

Wild-type mice, passively immunized with pooled human IgG, received IdeS (1250 UI/mouse) or PBS prior to injection of GLuc-encoding AAV8. The expression of GLuc was measured in the mice plasma after 7, 14 and 28 days. Non-reconstituted mice that lack anti-AAV8 IgG demonstrated constant production and accumulation of GLuc in the blood over 4 weeks (Figure 4C). This was accompanied by a substantial gene copy number in the livers of the mice (Figure 4D). In contrast, mice reconstituted with human IgG that contained anti-AAV8 neutralizing IgG failed to produce GLuc (Figure 4C). Interestingly, the treatment of passively immunized mice with IdeS restored almost complete gene therapy efficiency in the case of GLuc (Figure 4C).

Accordingly, the number of gene copies was 0.759±0.148 vg/diploid cell in the liver of human IgG-reconstituted IdeS-treated mice (Figure 4D), while it was 0.0006±0.0004 vg/diploid cell in human IgG-reconstituted mice that did not received IdeS (P<0.0001).

### EXAMPLE 5

### HB mouse study

Factor IX-deficient hemophilia B (HB) mice, reconstituted with IVIg or PBS, were treated with IdeS (1250 UI/mouse) or PBS, injected with hFIX-encoding AAV8 vectors, and the hFIX plasma levels were measured after 7 and 14 days. Non-reconstituted HB mice that lack anti-AAV8 IgG demonstrated production and accumulation of hFIX in the blood over 2 weeks (Figure 6A). In contrast, mice reconstituted with human IgG that contain anti-AAV8 neutralizing IgG failed to produce hFIX (Figure 6A). Interestingly, passively immunized mice treated with IdeS recovered complete efficiency of gene therapy and achieved circulating hFIX levels identical to those observed in the case of control non-reconstituted mice (Figure 6A). The levels of endogenously produced hFIX that were achieved following combined IdeS/AAV8 treatment protected mice from acute hemorrhage in a tail clipping assay. Indeed, the volume of blood loss was not statistically significant from that of non-immunized mice treated with AAV8 and of wild-type C57BL/6 mice (dotted line). In contrast, passively immunized mice that did not receive IdeS lost significantly more blood than the mice in the other groups.

### EXAMPLE 6

### NHP study

We investigated the efficacy of IdeS-treatment in non-human primates. One Cynomolgus monkey (NHP2) was treated with two IV injections of IdeS (500 µg/kg), as depicted in the experimental scheme shown in Figure 7. Intact IgG was measured in serum by ELISA and is expressed as mg/mL using purified monkey IgG as a standard (Figure 8A). The data show the elimination of total NHP2 IgG with some intact IgG remaining, even after the second injection of IdeS (D-1+10h, D0). IgG recovery started at D4 with a total recovery 21 days after treatment. Anti-AAV8 IgG was measured before and after IdeS treatment. Anti-AAV8 IgG levels in the treated monkey (NHP2) were 2.7-fold reduced after the second injection of IdeS (from 2.86 to 1.05 µg/mL) as measured by ELISA (Figure 8B). Likewise, the anti-AAV8 NAb titer was reduced by 3 folds (titer from 17.2 to 5.4) as measured in a neutralization assay (Figure 8C).

NHP2 and the control monkey (NHP1) that was not treated with IdeS received hFIX-encoding AAV8 (2×10¹² vg/kg) and the levels of hFIX in plasma were measured over time for 50 days. Compared to the control monkey (NHP1), the monkey having received IdeS treatment (NHP2) had significantly higher hFIX expression levels over time and until sacrifice (Figure 9A). This result is consistent with the assessment of liver VGCN. Indeed, the treated monkey (NHP2) showed more efficient liver transduction than the control non-treated monkey (NHP1) (Figure 9B, 10.1±1.6 vs. 1.6±0.2). We also followed the levels of neutralizing anti-AAV8 IgG in the blood of the NHP1 and NHP2 monkeys after AAV8-mediated gene therapy. In the IdeS-treated monkey (NHP2), the anti-AAV8 IgG humoral response was 11-fold reduced as compared with the untreated monkey (NHP1) (Figure 10A, 75.4±0.3 vs. 816.7±122). A similar reduction in the humoral response was observed with anti-AAV8 NAb (Figure 10B: NHP2, 1:316 vs. NHP1, 1:3160).

We then developed a protocol of re-administration of IdeS and AAV8 (Figure 11). To this end, two monkeys with similar initial levels of anti-AAV8 IgG were treated with hFIX-encoding AAV8 with (NHP4) or without (NHP3) previous IdeS treatment. As expected, the endogenous levels of hFIX after the first AAV8 injection were greater in the IdeS/AAV8-treated monkey (NHP4) than in the AAV8-treated control monkey that did not receive IdeS (NHP3) (Figure 12A). Both monkeys then received two injections of IdeS and re-dosing of hFIX-encoding AAV8. NHP3 failed to produce endogenous hFIX, which was associated with elevated levels of neutralizing anti-AAV8 IgG, poor liver transduction efficiency, as well as development of anti-hFIX antibodies (data not shown). Conversely, NHP4 experienced increased production of hFIX following a second treatment with IdeS/AAV8, which was consistent with the low lewels of anti-AAV8 IgG and NAb. Interestingly, hFIX levels were more stable after vector re-administration than after the first injection of the vector. These results are consistent with the assessment of liver VGCN (Figure 12B). We did a follow-up of the levels of neutralizing anti-AAV8 IgG in the blood of the monkeys over 105 days. In the IdeS-treated monkey (NHP4), the anti-AAV8 IgG humoral response was almost undetectable at the end of the experiment (Figure 13A, 2.25±0.071 µg/mL for NHP4 vs. 1552.5±337.9 µg/mL for NHP3). A similar reduction in the humoral response was observed with anti-AAV8 NAb (Figure 13B, 1:17.2 for NHP4 vs. 1:10000 for NHP3).

### Conclusion

The data show that IdeS treatment is effective to reduce neutralizing antibodies that inhibit gene therapy in the context of hemophilia B with an AAV8 vector encoding factor IX (see Examples 1, 4 and 5). The data support use of IdeS treatment for gene therapy to other AAV serotypes (see Example 2).

### EXAMPLE 7

### Methods

*Cleavage of immunoglobulin G (IgG) by endopeptidase in vitro:* Human serum or non-human primate (NHP) plasma samples with or without neutralizing antibodies (NAb) to Spk2 capsid were incubated with increasing doses of immunoglobulin-degrading enzyme from *Streptococcus pyogenes* (IdeS; Promega) for 1 hr at 37 °C. As per Promega's indications, one unit is defined as cleaving ≥ 95% of 1 µg of recombinant monoclonal IgG in 30 min at 37 °C. The reaction volume was adjusted with PBS. Cleavage of total IgG was assessed by SDS-PAGE and Coomassie stain.

*SDS-PAGE analysis of cleaved IgG:* Cleaved samples were prepared for non-reducing SDS-PAGE with NuPAGE^{®} LDS Sample Buffer (4X) (ThermoFisher Scientific) and heated to 70 °C for 10 min. Samples were then analyzed by NuPAGE^{®} Novex 4-12% Bis-Tris gel using MOPS SDS Running Buffer. Gels were stained with Coomassie Blue.

*Anti-AAV Capsid Neutralizing Antibody (NAb) Titer:* Neutralizing antibodies to AAV-Spk1 or AAV-Spk2 capsid were quantified using a cell-based, *in vitro* assay and either an AAV-Spk1 or AAV-Spk2, respectively, reporter-vector encapsidating a *Renilla* luciferase transgene. In brief, early passage (passage less than #26) 293E4 cells were thawed and plated in a flat-bottom white 96-well plate at 2×10⁴ cells/200 µL/well. Ponasterone A (Invitrogen; cat. # H101-01) was added to a final concentration of 1 µg/mL to each well in order to induce expression of the helper virus protein, human adenovirus E4. Cells were then cultured overnight in a 37 °C/5% CO₂ incubator. The following day, samples were heat-inactivated at 56 °C for 30 min, then a 4-point dilution (from 1:1 to 1:5) was prepared using FBS as the diluent. Factor Assay Control Plasma (FACT; King George Bio-Medical, Inc.) was prepared in a 3.16-fold (half-log) serial dilution to assess assay performance. AAV-luciferase vector was diluted to 7.5×10⁷ vg/mL in DMEM and then added to FACT controls and samples. Vector and controls/samples were incubated at 37 °C for 60 min. Volumes 7.5 µL per well of the "neutralized" controls/samples were transferred to each well of the plate seeded with cells, and the cells were returned to the incubator for overnight incubation. The next day cells were washed once in PBS, lysed in *Renilla* Assay Lysis Buffer, and luciferase activity was measured with the *Renilla* Luciferase Assay System (Promega) and read on a SpectraMax L microplate reader.

*Artificial Immunization Mouse Model Using IVIg:* To create an artificial NAb titer in male C57BL/6 mice, intravenous immune globulin (IVIg; Gamunex), containing 10% immunoglobulin G (IgG) purified from human blood, was injected intraperitoneally (IP) one day prior to intravenous (IV) vector administration. To determine whether *in vitro* cleavage of IgG by IdeS, or IdeZ-a similar endopeptidase from *Streptococcus equi,* which has improved activity against mouse IgG2a and IgG3 compared to IdeS-can rescue transduction efficiency *in vivo,* IVIg was treated with 125 units of IdeZ (Promega) overnight at 37 °C prior to IP dosing. Cleavage of total IgG was assessed by SDS-PAGE and Coomassie stain. One day post IVIg dose, vector (AAV-Spk1-GAA) was administered at 2×10¹² vg/kg. Mouse plasma samples were collected weekly, and samples were analyzed for activity of the transgene (GAA enzyme). To determine whether *in vivo* cleavage of IgG by IdeS can rescue transduction efficiency *in vivo,* 300 mg/kg IVIg was infused by IP dosing. After 24 hours, either 0.4 or 4 mg/kg IdeS was infused by IV dosing. Then 24 hours after IdeS infusion, vector (AAV-Spk1-GAA) was administered at 2×10¹² vg/kg.

*GAA Activity Assay:* GAA activity was assessed by measurement of cleavage of the substrate 4-methyl-umbelliferyl-α-D-glucoside at pH 4 (Galjaard et al., Clin Chim Acta 1973; 49(3):361-75). Briefly, the reaction was initiated by addition of 20 µL of substrate to 10 µL plasma sample diluted 1:250 in MilliQ water. The reaction mixture was incubated at 37 °C for 1 hr and then subsequently stopped by carbonate buffer at pH 10.5. The standard curve was plated thereafter with 4-methylumbelliferone, the blue fluorescent dye liberated from 4-methyl-umbelliferyl-α-D-glucoside, which produces a fluorescent emission at 440 nm when excited at 370 nm.

*Anti-AAV Capsid IgG antibodies:* Anti-AAV capsid total IgG formation was measured with a capture assay. ELISA plate wells were coated with 50 µL of a solution containing 1 µg/mL of AAV-Spk1 capsid particles. Total human IgG (Southern Biotech, 0150-01) was diluted to generate a 10-point standard curve ranging from 10,000 ng/mL to 0.5 ng/mL and added to the plate. The limit of quantitation of the assay was 460 ng/mL after back-calculation. Three levels of quality control samples were prepared and included on each plate to assess assay performance. Capsid particles, standards, and quality controls (QCs) were incubated overnight at 4 °C. After washing, wells were blocked with 2% BSA, 0.05% Tween-20 in PBS for 2 hrs at room temperature. Then, serial dilutions of samples in blocking buffer were loaded on the plate and incubated at room temperature for 2 hours. A horseradish peroxidase (HRP)-conjugated sheep anti-human IgG antibody (GE Healthcare, cat. # NA933V) diluted 1:5000 in blocking buffer was used as detecting antibody and incubated on the plate for 1 hr at room temperature. Following washing, the peroxidase activity was revealed following a 10-minute incubation at room temperature with 3,3',5,5'-tetramethylbenzidine substrate (TMB). The reaction was stopped with 1M sulfuric acid, and then the plate was read by an absorbance plate reader for optical density (OD) at 450 nm. IgG concentration was determined against a standard curve made with serial dilution of purified human total IgG.

### EXAMPLE 8

### IdeS cleaves IgG from human, NHP and hamster samples in vitro

The immunoglobulin G (IgG)-degrading enzyme from *Streptococcus pyogenes* (IdeS) is a cysteine protease that cleaves all four human subclasses of IgG with high specificity. IdeS hydrolyzes human IgG at Gly236 in the lower hinge region of the IgG heavy chains.

To analyze the ability of IdeS to cleave IgG in serum, increasing doses of IdeS (0-100 units; Promega) were added to human serum and NHP (rhesus macaque) plasma samples with or without an anti-Spk2 NAb titer. In human samples that were naive (<1:1) or had relative mid-range (1:5-1:10) or relative high (1:20-1:40) NAb titers, the lowest dose of IdeS cleaved all total IgG (~150kD) to liberate the Fc fragment (~25kD) (Figure 14A). In the NHP samples, IgG was similarly cleaved in all NAb titer groups (<1:1, 1:50-1:100, >1:100, Figure 14B).

Hamsters are expected to provide a better model than mice for examining IdeS treatment for AAV redosing. IdeS was tested for the ability to cleave hamster IgG, by incubating increasing amounts of IdeS (0 to 50 units; Promega), with pooled hamster plasma (and pooled human plasma as a positive control). Samples were analyzed by non-reducing SDS-PAGE and Coomassie Blue staining. IdeS was effective to cleave the IgG in the pooled hamster plasma and pooled human plasma *in vitro* (Figure 14C).

These results demonstrate that IdeS is a highly efficient and specific protease of human IgG, rhesus IgG and hamster IgG.

### EXAMPLE 9

### Cleavage of IgG by IdeS results in a reduced NAb titer in vitro

To analyze whether cleavage of IgG by IdeS is sufficient to reduce neutralization, AAV vector transduction efficiency was assayed *in vitro.* In this assay, serum or plasma samples from various species can be assessed for the presence of neutralizing antibodies to the AAV capsid by pre-incubating AAV vectors encoding *Renilla* luciferase with plasma or sera, transducing human cells in culture with these mixtures, and subsequently assessing levels of luciferase activity.

Human patient serum samples that were naive (<1:1) or had a high anti-Spk2 NAb titer (1:10-1:20) were pretreated with or without excess IdeS (50 units), and then NAb titers were assessed. Interestingly, the patient sample with a previously-reported NAb titer of 1:10-1:20 showed at least a two-fold decrease with IdeS pretreatment to a 1:5-1:10 NAb titer in one study (Table 1). These results demonstrate that AAV vector transduction is increased when IgG antibodies are cleaved by IdeS.

**Table 1 - Anti-Spk2 NAb titer analysis of human sera incubated with excess IdeS.**

| | Study 1 | | | | | |
|---|---|---|---|---|---|---|
| **Sample #** | | **Spark ID** | **IdeS** | **Vector** | **Previous Titer** | **Titer Range** |
| FACT Plate 1 | | NA | NA | NA | NA | 1:100 - 1:316 |
| S1 | | 397 | No | Spk2 | <1:1 | <1:1 |
| S2 | | 427 | No | Spk2 | 1:10 - 1:20 | 1:10 - 1:20 |
| S3 | | 397 | Yes | Spk2 | <1:1 | <1:1 |
| S4 | | 427 | Yes | Spk2 | 1:10 - 1:20 | 1:5 - 1:10 |

| Study 2 | | | | | | |
|---|---|---|---|---|---|---|
| **Sample #** | **Spark ID** | **IdeS** | **Vector** | **Previous Titer** | **Titer Range** | |
| S1 | FBS | No | Spk2 | NA | <1:1 | |
| S2 | FBS | Yes | Spk2 | NA | <1:1 | |
| S3 | BRH1450399 | No | Spk2 | 1:2.5 | 1:2.5 | |
| S4 | BRH1450399 | Yes | Spk2 | 1:2.5 | 1:1 | |
| S5 | BRH1450436 | No | Spk2 | ~1:5 | 1:5 | |
| S6 | BRH1450436 | Yes | Spk2 | ~1:5 | 1:1 | |
| S7 | BRH1450427 | No | Spk2 | 1:10 | 1:10 | |
| S8 | BRH1450427 | Yes | Spk2 | 1:10 | 1:10 | |

Anti-Spk2 NAb titer analysis following treatment with IdeS endopeptidase. Human patient samples (designated by Spark ID) were pretreated with and without IdeS. NAb titers were later assessed by *in vitro* vector transduction assay.

### EXAMPLE 10

### Degradation of IVIg by IdeZ in vitro increases the transduction efficiency of vector in vivo

The effector functions of IgG antibodies, such as cytotoxicity and complement fixation, are mediated by the Fc portion. Neutralization relies on the variable regions of the heavy and light chains for specificity to antigen. While the F(ab')₂ fragment still contains intact antigen-binding regions, data suggest that liberation of the F(ab')₂ fragment by IdeS or IdeZ, a similar endopeptidase in *Streptococcus equi,* which has improved activity against mouse IgG2a and IgG3, causes reduced stability without the Fc portion and therefore quicker clearance of the F(ab')₂ fragment from circulation than intact IgG. This assay tested whether administration of neutralizing antibodies that were pre-cleaved by IdeS or IdeZ should result in a reduction of neutralizing activity to AAV in the *in vivo* setting.

Mice were immunized with IVIg, a pool of human IgGs that includes anti-AAV capsid neutralizing antibodies that were pretreated with or without 0.1 mg/kg IdeZ. Mice were then administered 2×10¹² vg/kg AAV-Spk1-GAA. Mice treated with 1.0 mg or 5.0 mg IVIg resulted in reduced GAA activity levels in plasma (10,951 ± 1,554 nmol/hr/mL and 1,041 ± 553 nmol/hr/mL respectively) compared with control mice (33,551 ± 13,635 nmol/hr/mL) showing that vector neutralization by IVIg is dose dependent (Figure 15).

Pretreatment of 40 mg/kg IVIg with IdeZ rescued transduction efficiency, resulting in GAA activity levels (37,707 ± 11,449 nmol/hr/mL) that were comparable to control. IdeZ pretreatment of 200 mg/kg IVIg partially alleviated vector neutralization (13,440 nmol/hr/mL ± 15,543) with one animal completely recovering activity (41,025 nmol/hr/mL). Of note, IdeZ itself did not interfere with AAV vector transduction efficiency. IVIg dose retains were analyzed by SDS-PAGE with Coomassie stain to confirm cleavage of IgG. These results indicate that *in vitro* cleavage of neutralizing antibodies to the AAV capsid by IdeS/IdeZ can rescue AAV vector transduction and transgene expression *in vivo.*

### EXAMPLE 11

### Degradation of IVIg by IdeS in vivo increases the transduction efficiency of vector in vivo

To analyze whether cleavage of IgG *in vivo* can affect vector transduction and transgene expression/activity in plasma, mice were first infused with intact IVIg to create an artificial titer of human anti-capsid neutralizing IgGs. After 24 hours, mice were infused with IdeS at two concentrations (0.4 mg/kg or 4 mg/kg), and then 24 hours after IdeS infusion, all mice were administered 2×10¹² vg/kg AAV-Spk1-GAA. Both anti-Spkl NAb titers and IgG levels were analyzed pre-IdeS infusion and post-IdeS infusion (immediately prior to vector administration).

IdeS infusion induced a dose-dependent decrease in both NAb (Figure 16) and IgG levels (Figure 17). The highest dose of IdeS (4 mg/kg) was capable of reducing NAb titers of at least 1:40 down to <1:1.

When GAA activity was measured one week post vector infusion, control mice administered only vector demonstrated GAA activity levels of 49,387 ± 7,345 nmol/hr/mL (Figure 18). Mice that were injected with 300 mg/kg IVIg exhibited GAA transgene activity levels in plasma (1,702 ± 336 nmol/hr/mL) consistent with almost complete inhibition of transduction. IdeS displayed a dose-dependent rescue of transgene activity levels; 0.4 mg/kg IdeS resulted in a 70% rescue of GAA activity (34,408 ± 10,562 nmol/hr/mL), while 4 mg/kg IdeS rescued 99% GAA activity (48,948 ± 5,322 nmol/hr/mL). These results demonstrate that IdeS treatment *in vivo* reduces neutralizing antibody titers and allows for dosing and transduction of AAV vectors in animals that are refractory to treatment.

### EXAMPLE 12 Degradation of IVIg by IdeS in vivo increases the transduction efficiency of vector in vivo

IdeS was evaluated for ability to cleave higher titers of anti-capsid IgG *in vivo* and to rescue AAV transduction in the context of a higher degree of AAV vector neutralization. Mice (male C57BL/6) were infused with varying doses of intact human IVIg (300 mg/kg (low), 800 mg/kg (mid), or 1600 mg/kg (high)) to create an artificial titer of human anti-capsid neutralizing IgGs. After 24 hours, mice were infused with IdeS at three concentrations (0.4 mg/kg (low), 1 mg/kg (mid), or 2 mg/kg (high)). 24 hours after IdeS infusion, mice were administered AAV-Spk1-GAA at 2×10¹² vg/kg. Anti- Spk1 NAb titers were determined at both pre-IdeS infusion and post-IdeS infusion (immediately prior to vector administration), using the Anti-AAV Capsid NAb Titer assay described in Example 1. AAV transduction was assessed by measurement of transgene product (GAA) activity in plasma using the GAA Activity Assay, as described in Example 1, two weeks post vector administration.

For all doses of IVIg, pre-treatment with IdeS yielded a dose-dependent decrease in AAV NAb titer (Table 2). Table 2 presents the neutralizing anti-Spkl antibody (NAb) titer pre- and post-IdeS infusion for each animal in each group. AAV NAb titers are designated as low (<1:1, 1:1-1:2.5), low-to-mid range (1:2.5-1:5), mid-to-high range (1:5-1:10) and high (>1:10-1:20). The highest dose of IdeS (2 mg/kg) was capable of reducing NAb titers of > 1:160 (generated with 1600 mg/kg IVIg) down to 1:1-1:2.5.

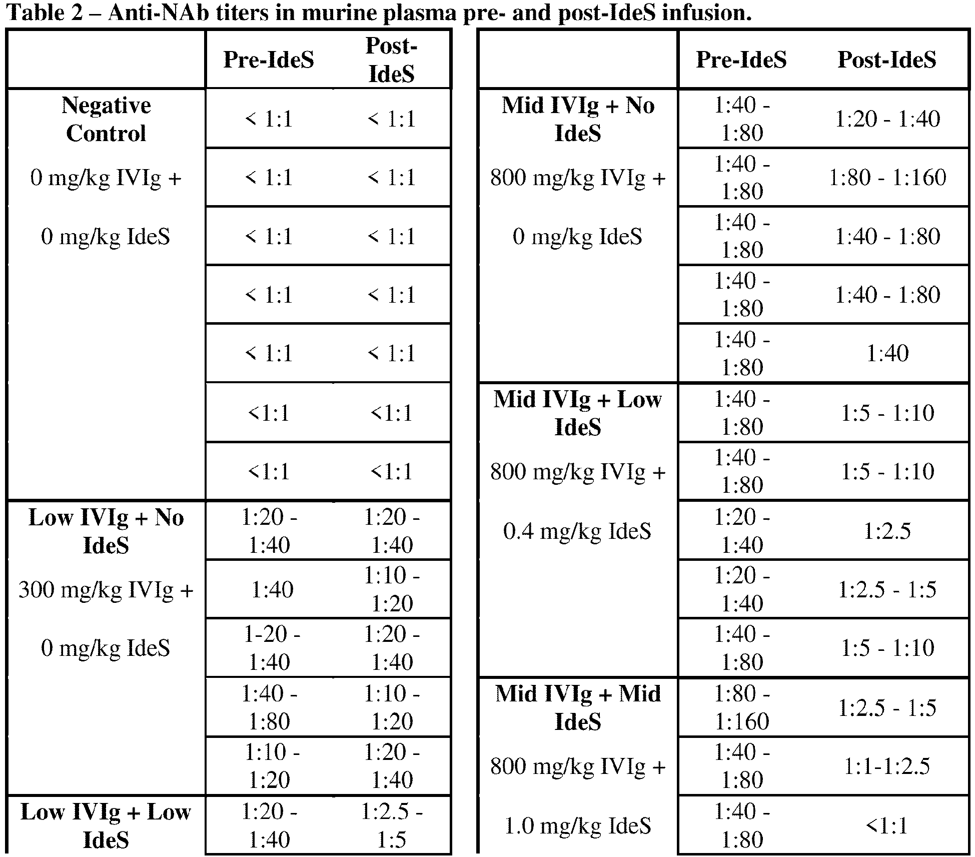

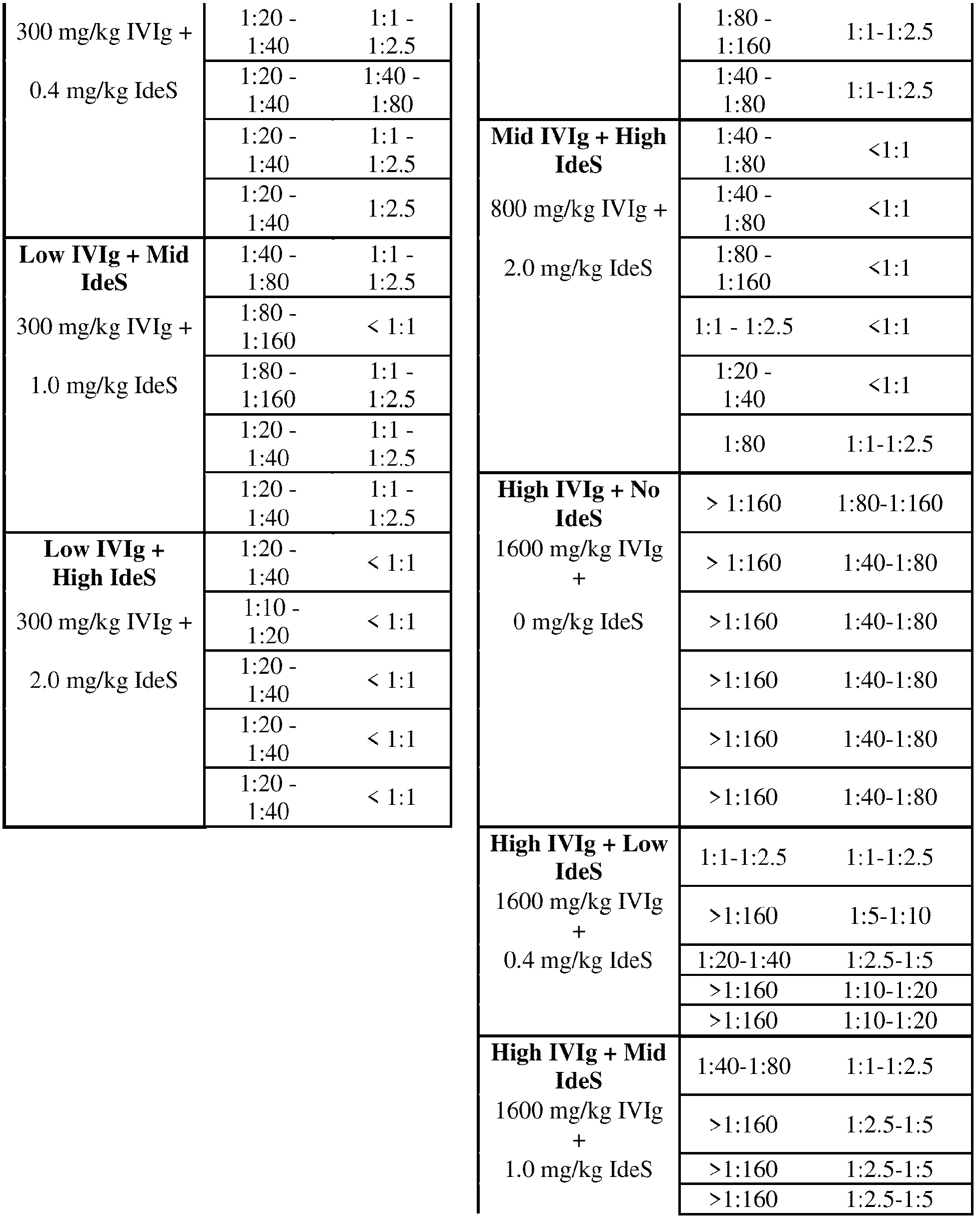

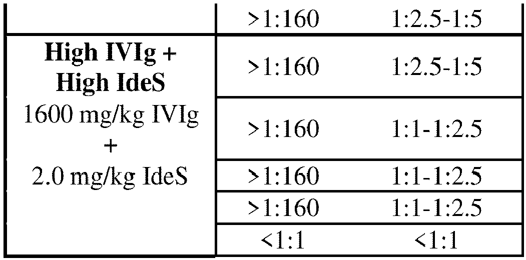

The results for GAA activity, measured two weeks post vector infusion, are shown in Figure 19. The plasma of negative control mice (administered vector only) demonstrated GAA activity levels of 26,689 ± 12,420 nmol/hr/mL. Mice injected with 300 mg/kg (and higher) IVIg, and receiving no IdeS, exhibited plasma GAA activity levels of only 436 ± 41 nmol/hr/mL, consistent with NAb inhibition of AAV vector transduction. Consistent with the results in the previous Examples, IdeS pre-treatment resulted in a dose-dependent rescue of AAV vector transduction, as measured by GAA activity levels in plasma: 0.4 mg/kg IdeS resulted in GAA activity levels of 7,702 ± 4,710 nmol/hr/mL, 1 mg/kg IdeS resulted in GAA activity levels of 15,444 ± 4,226 nmol/hr/mL, and 2 mg/kg IdeS resulted in GAA activity levels of 14,375 ± 2,572 nmol/hr/mL.

Groups that received higher doses of IVIg (either 800 or 1600 mg/kg IVIg) demonstrated similar trends of a dose-dependent increase in GAA activity levels with increasing IdeS. With 800 mg/kg IVIg, 0.4 mg/kg IdeS resulted in GAA activity levels of 4,188 ± 2,549 nmol/hr/mL, 1 mg/kg IdeS resulted in GAA activity levels of 17,813 ± 11,283 nmol/hr/mL, and 2 mg/kg IdeS resulted in GAA activity levels of 26,846 ± 7,354 nmol/hr/mL. With 1600 mg/kg IVIg, 0.4 mg/kg IdeS resulted in GAA activity levels of 580 ± 217 nmol/hr/mL, 1 mg/kg IdeS resulted in GAA activity levels of 12,511 ± 1,602 nmol/hr/mL and 2 mg/kg IdeS resulted in GAA activity levels of 11,573 ± 1,313 nmol/hr/mL. At the highest dose of IVIg (1600 mg/kg), the lowest IdeS dose (0.4 mg/kg) failed to rescue vector transduction. At the 1600 mg/kg dose of IVIg, however, there are supraphysiological levels of total IgG present in circulation, which likely reduced the effectiveness of IdeS at the 0.4 mg/kg dose due to the increased amount of its substrate.

These results show that IdeS treatment *in vivo* reduces neutralizing antibody titers and allows for dosing and transduction of viral vectors in animals that are refractory to viral vector gene therapy treatment methods.

### EXAMPLE 13

Applications of this co-therapy are wide ranging. For example, overcoming NAbs to the AAV capsid or other mode of gene delivery by IdeS has the potential to enable treatment of patients with pre-existing neutralizing antibody titers as well as repeat dosing of patients previously administered with a gene therapy product where effective levels have either not been achieved or have been lost due to time or other confounding issue. Additionally, the methods herein enable hepatic gene transfer to the pediatric population, which has been seen as intractable to gene therapy due to hepatocyte expansion and potential loss of transgene expression during development. These results indicate that IdeS administration will result in reducing or clearing neutralizing antibodies against the AAV capsid and enable treatment of patients previously viewed as not eligible for gene therapy or that develop AAV antibodies after AAV gene therapy.

### EXAMPLE 14

### Hamster model of AAV redosing

Hamsters are infused with rAAV vector particles carrying a transgene of interest, are dosed with IdeS after the development of anti-AAV NAb *(e.g.,* 4 weeks), and infused with additional rAAV particles carrying another transgene. This model allows for analysis of transduction efficacy at varying stages, including before and after IdeS treatment and before and after redosing with rAAV.

To assess the ability of IdeS to degrade or reduce the effects of neutralizing antibodies to the AAV capsid, a study is performed in Syrian Golden hamsters, a species whose IgG is efficiently cleaved by the endopeptidase IdeS. Hamsters are first infused with 2×10¹² vg/kg Spk1-FVIII, Spk1-FIX, or other Spk1 encapsidated vector. Animals are monitored for expression of the transgene product (*i.e.,* FVIII, FIX, etc.) in plasma, in addition to measurement of the development of NAbs to the Spk1 capsid by anti-Spkl IgG ELISA or a cell-based neutralizing antibody assay. Following the development of NAb titer, within 3-5 weeks of vector infusion, animals are infused by intravenous, subcutaneous, intraperitoneal or other route of administration with single or multiple ascending doses of IdeS of 0, 0.5, 1, and 2 mg/kg (and higher doses). After IdeS administration, animals are followed by measuring anti-Spkl capsid IgG and/or NAbs to Spk1. When animals display a sufficient decrease in NAb levels, they are infused with 2×10¹² vg/kg Spk1-GAA. Following transduction, GAA expression is measured in plasma by GAA activity assay and/or GAA antigen level measurement to determine the level of transduction attained.

These studies show if IdeS reduces AAV capsid-specific NAbs *in vivo* to a level low enough to enable redosing. Measurement of anti-FVIII IgG (if developed *in vivo*)*,* provides information regarding the effectiveness of IdeS in reducing transgene product-targeting NAbs, and the number of rounds of IdeS redosing permissible before loss of effectiveness.

### EXAMPLE 15

### Cynomolgus monkey model of AAV redosing

To assess the ability of IdeS to degrade or reduce the effects of NAbs against the AAV capsid in a large animal model, a study is performed in cynomolgus monkeys (*Macaca fascicularis*)*.* Monkeys are first screened for pre-existing NAbs to the Spk1 capsid. NAb positive animals likely result from exposure to naturally occurring AAV in the wild or in group housing. Animals are placed into groups based on negative or positive NAb titer, and, if positive, how high the pre-existing NAb titer is.

In the re-dosing arm of the study, animals are dosed with 2×10¹² vg/kg Spk1-FIX, or other Spk1 encapsidated vector. Animals are monitored for expression of the transgene product *(i.e.,* FIX or other) in plasma, in addition to measurement of the development of NAbs to the Spk1 capsid by anti-Spkl IgG ELISA or a cell-based NAb assay. Following development of NAb titer, within 3-5 weeks of vector infusion, animals are infused by intravenous, subcutaneous, intraperitoneal or other route of administration with single or multiple ascending doses of IdeS of 0, 0.5, 1, and 2 mg/kg, and higher doses. After IdeS administration, animals are followed by measuring anti-Spkl capsid IgG and/or NAbs to Spk1. When animals display a sufficient decrease in NAb levels, they are infused with 2×10¹² vg/kg Spk1-GAA. Following transduction, GAA expression is measured in plasma by GAA activity assay and/or GAA antigen level assessment to determine the level of transduction attained.

A separate arm of the study evaluates the ability of IdeS to overcome pre-existing NAb titers. Animals displaying different NAb titer levels are grouped based on titer and infused by intravenous, subcutaneous, intraperitoneal or other route of administration with single or multiple ascending doses of IdeS of 0, 0.5, 1, and 2 mg/kg (and higher doses). Following IdeS administration, animals are followed by measuring anti-Spkl capsid IgG and/or NAbs to Spk1 and, when animals display a sufficient decrease in NAb levels, they are infused with 2×10¹² vg/kg Spk1-GAA. Following transduction, GAA expression is measured in plasma by GAA activity assay and/or GAA antigen level measurement to determine the level of transduction attained.

These studies show if IdeS reduces AAV capsid-specific NAbs *in vivo* to a level low enough to enable redosing in cynomolgus monkeys, a species that is an excellent model of human AAV administration. These studies also show the maximal pre-existing NAb titer that can be overcome by IdeS administration. Measurement of anti-FIX IgG (if developed *in vivo*)*,* provides information regarding the effectiveness of IdeS in reducing transgene product-targeting NAbs, and the number of rounds of IdeS redosing permissible before loss of effectiveness.

### EXAMPLE 16

### Mouse study with IdeS and AAV-Spk1-hFVIII

Two different preparations of IdeS (Lot 1 and Lot 2) were tested in mice having an artificial titer of human anti-capsid neutralizing IgGs. C57BL/6 mice were injected with 300 mg/kg of IVIg at Day -2, followed by 1 mg/kg IdeS at Day -1 (pre-dosing with AAV), and finally with 5×10¹⁰ vector genomes of an AAV-Spk1 vector encoding a human Factor VIII (AAV-Spk1-hFVIII) at Day 0 (post-dose). Negative control animals received no IVIg or IdeS treatment, and the "No IdeS" group received only IVIg and AAV-Spk1-hFVIII vector. Neutralizing antibody titers in plasma were determined pre- and post-IdeS administration, using an anti-AAV capsid neutralizing assay similar to that described in Example 7, using an 8-point titer (1:1 to 1:160) on the samples, and luminescence was read on a GloMax^{®} Discover Microplate Reader (Promega). Titer was determined as the highest dilution or range where luminescence was inhibited by > 50%. NAb titers pre- and post-IdeS treatment shows that both lots of IdeS were effective in decreasing the NAb titer in the mice (Fig. 20).

Human FVIII antigen levels were measured by ELISA pre-vector infusion and at one and two weeks post vector infusion (Fig. 21). Both lots of IdeS treatment *in vivo* reduced neutralizing antibody titers and to allow for dosing with an AAV vector and expression of the transgene.

### EXAMPLE 17

### Mouse study with anti-AAV-Spkl IgG

C57BL/6 mice were given IVIg to induce an artificial titer of human anti-capsid neutralizing IgG. Three concentrations of IVIg (300 mg/kg (low), 800 mg/kg (mid), and 1600 mg/kg (high) were used, and within each IVIg group, animals were treated with increasing doses of IdeS (0, 0.4, 1.0, 2.0 mg/kg). Anti-Spk1 capsid IgG levels were assessed by ELISA. Briefly, 96-well plates were coated with Spk1 empty capsid, then blocked with BSA, washed, and incubated with plasma, diluted 1:100, for 2 hours. Following incubation, plates were washed and incubated with a secondary antibody conjugated with HRP for 1 hour. Subsequently, plates were washed again and developed using TMB substrate. Plates were read on an absorbance plate reader for optical density (OD) at 450 nm. Luminescence was compared to a standard curve of human IgG to determine antibody concentrations. All three concentrations of IdeS (0.4, 1.0, 2.0 mg/kg) eliminated or significantly reduced serum levels of anti-Spkl capsid IgG for all three concentrations (low, mid and high) of IVIg (Fig. 22).

### EXAMPLE 18

Spk1 (SEQ ID NO:1):

MAADGYLPDWLEDNLSEGIREWWDLKPGAPKPKANQQKQDNGRGLVLPGYKYLGPF
NGLDKGEPVNAADAAALEHDKAYDQQLQAGDNPYLRYNHADAEFQERLQEDTSFGGN
LGRAVFQAKKRVLEPLGLVESPVKTAPGKKRPVEPSPQRSPDSSTGIGKKGQQPAKKRL
NFGQTGDSESVPDPQPIGEPPAAPSGVGPNTMAAGGGAPMADNNEGADGVGSSSGNW
HCDSTWLGDRVITTSTRTWALPTYNNHLYKQISNGTSGGSTNDNTYFGYSTPWGYFDF
NRFHCHFSPRDWQRLINNNWGFRPKRLNFKLFNIQVKEVTQNEGTKTIANNLTSTIQVFT
DSEYQLPYVLGSAHQGCLPPFPADVFMIPQYGYLTLNNGSQAVGRSSFYCLEYFPSQML
RTGNNFEFSYNFEDVPFHSSYAHSQSLDRLMNPLIDQYLYYLSRTQSTGGTAGTQQLLFS
QAGPNNMSAQAKNWLPGPCYRQQRVSTTLSQNNNSNFAWTGATKYHLNGRDSLVNP
GVAMATHKDDEERFFPSSGVLMFGKQGAGKDNVDYSSVMLTSEEEIKTTNPVATEQYG

VVADNLQQQNAAPIVGAVNSQGALPGMVWQNRDVYLQGPIWAKIPHTDGNFHPSPLM
GGFGLKHPPPQILIKNTPVPADPPTTFNQAKLASFITQYSTGQVSVEIEWELQKENSKRW
NPEIQYTSNYYKSTNVDFAVNTEGTYSEPRPIGTRYLTRNL

Spk2 (SEQ ID NO:2):

MAADGYLPDWLEDNLSEGIREWWALQPGAPKPKANQQHQDNARGLVLPGYKYLGPG
NGLDKGEPVNAADAAALEHDKAYDQQLKAGDNPYLKYNHADAEFQERLKEDTSFGG
NLGRAVFQAKKRLLEPLGLVEEAAKTAPGKKRPVDQSPQEPDSSSGVGKSGKQPARKR
LNFGQTGDSESVPDPQPLGEPPAAPTSLGSNTMASGGGAPMADNNEGADGVGNSSGNW
HCDSQWLGDRVITTSTRTWALPTYNNHLYKQISSQSGASNDNHYFGYSTPWGYFDFNR
FHCHFSPRDWQRLINNNWGFRPKKLSFKLFNIQVKEVTQNDGTTTIANNLTSTVQVFTD
SEYQLPYVLGSAHQGCLPPFPADVFMVPQYGYLTLNNGSQAVGRSSFYCLEYFPSQMLR
TGNNFQFSYTFEDVPFHSSYAHSQSLDRLMNPLIDQYLYYLNRTQGTTSGTTNQSRLLFS
QAGPQSMSLQARNWLPGPCYRQQRLSKTANDNNNSNFPWTAASKYHLNGRDSLVNPG
PAMASHKDDEEKFFPMHGNLIFGKEGTTASNAELDNVMITDEEEIRTTNPVATEQYGTV
ANNLQSSNTAPTTRTVNDQGALPGMVWQDRDVYLQGPIWAKIPHTDGHFHPSPLMGGF
GLKHPPPQIMIKNTPVPANPPTTFSPAKFASFITQYSTGQVSVEIEWELQKENSKRWNPEI
QYTSNYNKSVNVDFTVDTNGVYSEPRPIGTRYLTRPL

Sequence of IdeS including N terminal methionine and signal sequence. (SEQ ID NO:3, NCBI Reference Sequence no. WP_010922160.1):

MRKRCYSTSAAVLAAVTLFVLSVDRGVIADSFSANQEIRYSEVTPYHVTSVWTKGVTPP
ANFTQGEDVFHAPYVANQGWYDITKTFNGKDDLLCGAATAGNMLHWWFDQNKDQIK
RYLEEHPEKQKINFNGEQMFDVKEAIDTKNHQLDSKLFEYFKEKAFPYLSTKHLGVFPD
HVIDMFINGYRLSLTNHGPTPVKEGSKDPRGGIFDAVFTRGDQSKLLTSRHDFKEKNLKE
ISDLIKKELTEGKALGLSHTYANVRINHVINLWGADFDSNGNLKAIYVTDSDSNASIGMK
KYFVGVNSAGKVAISAKEIKEDNIGAQVLGLFTLSTGQDSWNQTN

Mature sequence of IdeS, lacking the N terminal methionine and signal sequence (GenBank Accession No. ADF13949.1). (SEQ ID NO:4):

DSFSANQEIRYSEVTPYHVTSVWTKGVTPPANFTQGEDVFHAPYVANQGWYDITKTFN
GKDDLLCGAATAGNMLHWWFDQNKDQIKRYLEEHPEKQKINFNGEQMFDVKEAIDTK

NHQLDSKLFEYFKEKAFPYLSTKHLGVFPDHVIDMFINGYRLSLTNHGPTPVKEGSKDPR
GGIFDAVFTRGDQSKLLTSRHDFKEKNLKEISDLIKKELTEGKALGLSHTYANVRINHVI
NLWGADFDSNGNLKAIYVTDSDSNASIGMKKYFVGVNSAGKVAISAKEIKEDNIGAQV
LGLFTLSTGQDSWNQTN

SEQ ID NOs:5 to 18 are the sequences of exemplary IdeS polypeptides from Table C of WO 2016/128558.
SEQ ID NO:5: 
DSFSANQEIRYSEVTPYHVTSVWTKGVTPPANFTQGEDVFHAPYVANQGWYDITKTFN
GKDDLLCGAATAGNMLHWWFDQNKDQIKRYLEEHPEKQKINFRGEQMFDVKEAIDTK
NHQLDSKLFEYFKEKAFPYLSTKHLGVFPDHVIDMFINGYRLSLTNHGPTPVKEGSKDPR
GGIFDAVFTRGDQSKLLTSRHDFKEKNLKEISDLIKKELTEGKALGLSHTYANVRINHVI
NLWGADFDSNGNLKAIYVTDSDSNASIGMKKYFVGVNSAGKVAISAKEIKEDNIGAQV
LGLFTLSTGQDSWNQTN
SEQ ID NO:6: 
DSFSANQEIRYSEVTPYHVTSVWTKGVTPPANFTQGEDVFHAPYVANQGWYDITKTFN
GKDDLLCGAATAGNMLHWWFDQNKDQIKRYLEEHPEKQKINFKGEQMFDVKEAIDTK
NHQLDSKLFEYFKEKAFPYLSTKHLGVFPDHVIDMFINGYRLSLTNHGPTPVKRGSKDP
RGGIFDAVFTRGNQSKLLTSRHDFKEKNLKEISDLIKKELTEGKALGLSHTYANVRINHV
INLWGADFDSNGNLKAIYVTDSDSNASIGMKKYFVGVNSAGKVAISAKEIKEDNIGAQV
LGLFTLSTGQDSWNQTN
SEQ ID NO:7: 
DSFSANQEIRYSEVTPYHVTSVWTKGVTPPANFTQGEDVFHAPYVANQGWYDITKTFN
GKDDLLCGAATAGNMLHWWFDQNKDQIKRYLEEHPEKQKINFRGEQMFDVKEAIDTK
NHQLDSKLFEYFKEKAFPYLSTKHLGVFPDHVIDMFINGYRLSLTNHGPTPVKKGSKDP
RGGIFDAVFTRGNQSKLLTSRHDFKEKNLKEISDLIKKELTEGKALGLSHTYANVRINHV
INLWGADFDSNGNLKAIYVTDSDSNASIGMKKYFVGVNKAGKVAISAKEIKEDNIGAQV
LGLFTLSTGQDSWNQTN
SEQ ID NO:8: 
DSFSANQEIRYSEVTPYHVTSVWTKGVTPPANFTQGEDVFHAPYVANQGWYDITKTFN
GKDDLLCGAATAGNMLHWWFDQNKDQIKRYLREHPEKQKINFNGEQMFDVKEAIDTK
NHQLDSKLFEYFKEKAFPYLSTKHLGVFPDHVIDMFINGYRLSLTNHGPTPVKEGSKDPR
GGIFDAVFTRGNQSKLLTSRHDFKEKNLKEISDLIKKELDEGKALGLSHTYANVRINHVI
NLWGADFDSNGNLKAIYVTDSDSNASIGMKKYFVGVNSAGKVAISAKEIKEDNIGAQV
LGLFTLSTGQDSWNQTN
SEQ ID NO:9: 
DSFSANQEIRYSEVTPYHVTSVWTKGVTPPANFTQGEDVFHAPYVANQGWYDITKTFN
GKDDLLCGAATAGNMLHWWFDQNKDQIKRYLKEHPEKQKINFNGEQMFDVKEAIRTK
NHQLDSKLFEYFKEKAFPYLSTKHLGVFPDHVIDMFINGYRLSLTNHGPTPVKEGSKDPR
GGIFDAVFTRGNQSKLLTSRHDFKEKNLKEISDLIKKELEEGKALGLSHTYANVRINHVI
NLWGADFDSNGNLKAIYVTDSDSNASIGMKKYFVGVNKAGKVAISAKEIKEDNIGAQV
LGLFTLSTGQDSWNQTN
SEQ ID NO:10: 
DSFSANQEIRYSEVTPYHVTSVWTKGVTPPANFTQGEDVFHAPYVANQGWYDITKTFN
GKDDLLCGAATAGNMLHWWFDQNKDQIERYLEEHPEKQKINFNGEQMFDVKEAIDTK
NHQLDSKLFEYFKEKAFPYLSTKHLGVFPDHVIDMFINGYRLSLTNHGPTPVKEGSKDPR
GGIFDAVFTRGNQSKLLTSRHDFKEKNLKEISDLIKEELTKGKALGLSHTYANVRINHVI
NLWGADFDSNGNLKAIYVTDSDSNASIGMKKYFVGVNSAGKVAISAKEIKEKNIGAQV
LGLFTLSTGQKSWNQTN
SEQ ID NO:11: 
DSFSANQEIRYSEVTPYHVTSVWTKGVTPPANFTQGEDVFHAPYVANQGWYDITKTFN
GKDDLLCGAATAGNMLHWWFDQNKDQIKRYLKEHPEKQKINFRGEQMFDVKEAIRTK
NHQLDSKLFEYFKEKAFPYLSTKHLGVFPDHVIDMFINGYRLSLTNHGPTPVKEGSKDPR
GGIFDAVFTRGNQSKLLTSRHDFKEKNLKEISDLIKSELENGKALGLSHTYANVRINHVI
NLWGADFDSNGNLKAIYVTDSDSNASIGMKKYFVGVNKAGKVAISAKEIKEDNIGAQV
LGLFTLSTGQDSWNQTN
SEQ ID NO:12: 
DSFSANQEIRYSEVTPYHVTSVWTKGVTPPANFTQGEDVFHAPYVANQGWYDITKTFN
GKDDLLCGAATAGNMLHWWFDQNKDQIKRYLKEHPEKQKINFRGEQMFDVKEAIRTK
NHQLDSKLFEYFKEKAFPYLSTKHLGVFPDHVIDMFINGYRLSLTNHGPTPVKKGSKDP
RGGIFDAVFTRGNQSKLLTSRHDFKEKNLKEISDLIKKELEEGKALGLSHTYANVRINHV
INLWGADFDSNGNLKAIYVTDSDSNASIGMKKYFVGVNSAGKVAISAKEIKEDNIGAQV
LGLFTLSTGQDSWNQTN
SEQ ID NO:13: 
DSFSANQEIRYSEVTPYHVTSVWTKGVTPPANFTQGEDVFHAPYVANQGWYDITKTFN
GKDDLLCGAATAGNMLHWWFDQNKDQIERYLEEHPEKQKINFRGEQMFDVKEAIDTK
NHQLDSKLFEYFKEKAFPYLSTKHLGVFPDHVIDMFINGYRLSLTNHGPTPVKKGSKDP
RGGIFDAVFTRGNQSKLLTSRHDFKEKNLKEISDLIKEELTKGKALGLSHTYANVRINHV
INLWGADFDSNGNLKAIYVTDSDSNASIGMKKYFVGVNSAGKVAISAKEIKEDNIGAQV
LGLFTLSTGQKSWNQTN
SEQ ID NO:14: 
DSFSANQEIRYSEVTPYHVTSVWTKGVTPPANFTQGEDVFHAPYVANQGWYDITKTFN
GKDDLLCGAATAGNMLHWWFDQNKDQIKRYLEEHPEKQKINFNGEQMFDVKEAIDTK
NHQLDSKLFEYFKEKAFPYLSTKHLGVFPDHVIDMFINGYRLSLTNHGPTPVKEGSKDPR
GGIFDAVFTRGDQSKLLTSRHDFKEKNLKEISDLIKKELTEGKALGLSHTYANVRINHVI
NLWGADFDSNGNLKAIYVTDSDSNASIGMKKYFVGVNSAGKVAISAKEIKEDNIGAQV
LGLFTLSTGQDSW
SEQ ID NO:15: 
SVWTKGVTPPANFTQGEDVFHAPYVANQGWYDITKTFNGKDDLLCGAATAGNMLHW
WFDQNKDQIKRYLEEHPEKQKINFNGEQMFDVKEAIDTKNHQLDSKLFEYFKEKAFPYL
STKHLGVFPDHVIDMFINGYRLSLTNHGPTPVKEGSKDPRGGIFDAVFTRGDQSKLLTSR
HDFKEKNLKEISDLIKKELTEGKALGLSHTYANVRINHVINLWGADFDSNGNLKAIYVT
DSDSNASIGMKKYFVGVNSAGKVAISAKEIKEDNIGAQVLGLFTLSTGQDSWNQTN
SEQ ID NO:16: 
SVWTKGVTPPANFTQGEDVFHAPYVANQGWYDITKTFNGKDDLLCGAATAGNMLHW
WFDQNKDQIKRYLEEHPEKQKINFKGEQMFDVKEAIDTKNHQLDSKLFEYFKEKAFPYL
 
STKHLGVFPDHVIDMFINGYRLSLTNHGPTPVKEGSKDPRGGIFDAVFTRGNQSKLLTSR
HDFKEKNLKEISDLIKKELTEGKALGLSHTYANVRINHVINLWGADFDSNGNLKAIYVT
DSDSNASIGMKKYFVGVNSAGKVAISAKEIKEDNIGAQVLGLFTLSTGQDSWNQTN
SEQ ID NO:17: 
SVWTKGVTPPANFTQGEDVFHAPYVANQGWYDITKTFNGKDDLLCGAATAGNMLHW
WFDQNKDQIERYLEEHPEKQKINFKGEQMFDVKKAIDTKNHQLDSKLFEYFKEKAFPYL
STKHLGVFPDHVIDMFINGYRLSLTNHGPTPVKEGSKDPRGGIFDAVFTRGNQSKLLTSR
HDFKEKNLKEISDLIKEELTKGKALGLSHTYANVRINHVINLWGADFDSNGNLKAIYVT
DSDSNASIGMKKYFVGVNSAGKVAISAKEIKEDNIGAQVLGLFTLSTGQKSWNQTN
SEQ ID NO:18:

DDYQRNATEAYAKEVPHQITSVWTKGVTPPANFTQGEDVFHAPYVANQGWYDITKTF
NGKDDLLCGAATAGNMLHWWFDQNKDQIERYLEEHPEKQKINFKGEQMFDVKKAIDT
KNHQLDSKLFEYFKEKAFPYLSTKHLGVFPDHVIDMFINGYRLSLTNHGPTPVKEGSKD
PRGGIFDAVFTRGNQSKLLTSRHDFKEKNLKEISDLIKEELTKGKALGLSHTYANVRINH
VINLWGADFDSNGNLKAIYVTDSDSNASIGMKKYFVGVNSAGKVAISAKEIKEDNIGAQ
VLGLFTLSTGQKSWNQTNGGGHHHHHH

IgdE of *S*. *agalactiae* is specific for human IgG1 (WO2017134274) (SEQ ID NO:19,):

NQNNIQETNLVEKNSEDKFIQELNRYKTEIPNFKGFNVWILGDKGYYKNLINLEEIKNIQ
ATLKKERNEEYVFVKLNGKIAHDTTVFLMNKKHKLLKNIEEFKTITQKRLTERGKFPYD
TVHSTFEIKDENFIMERLKSSGLSMGKPVDYMGVNGIPIYTKTLSIDNKFAFENNSKDSS
YSSNINISEDKIKENDQKILDLIVKSGANNQNLTDEEKVIAFTKYIGEITNYDNEAYRARN
VDTEYYRASDLFSVTERKLAMCVGYSVTAARAFNIMGIPSYVVSGKSPQGISHAAVRAY
YNRSWHIIDITASTYWKNGNYKTTYSDFIKEYCIDGYDVYDPAKTNNRFKVKYMESNE
AFENWIHNNGSKSMLFINESAALKDKKPKDDFVPVTEKEKNELIDKYKKLLSQIPENTQ
NPGEKNIRDYLKNEYEEILKKDNLFEHEHAEFKESLNLNESFYLQLKKEEKKPSDNLKKE
EKPRENSVKERETPAENNDFVSVTEKNNLIDKYKELLSKIPENTQNPGEKNIRNYLEKEY
EELLQKDKLFKHEYTEFTKSLNLNETFYSQLKEGEMKLSENPEKGETNTN

IgdE of *S*. *pseudoporcinus* degrades both human IgG1 and porcine IgG (WO2017134274) (SEQ ID NO:20):

RENENVRQLQSENKQMKAVNLQEFSEKLKGEIAENQQFHIFKLGLNNYYIGGVRINELS
DLAKNHDFIMIDNRATHNKYGVPHIIIMNKDDVIVHNQEDYNKEMAELTFAGDKPIQSD
SYLPQKKRIHALFEIGLDSNRRQLLNAAGLKTPENSVIELDTFKIYSHGLAVDNKYYDEY
SHFNNNTNVNITKQRFTENDNLIHNLITTSTAKDQPTDRDKVKTFVMYVANHTIYDWNA
ANNAVSNISDVNYYLGSDLFSITERKKAMCVGFSTTAARAFNMLGIPAYVVEGKNAQG
VDHATARVYYNGKWHTIDGTGFINGNRTRSTLYTESHFRSVGEDSYQLVGLNEDIPFDR
NYMKIDKVYEEWAPKQKTADLLLVNKDKSLVGLDRVAYVEPVYVDKNRQDALTQIYK
KLKETMESSSKKNPSSGGFSSLLGSASSDIAKLEGSSQLTQEEYDKIHRSMTSILTFFAQL
DKDAAEAFEKGNDYKNYLATTKHAQ

The full sequence of IdeZ is available as NCBI Reference Sequence No. WP 014622780.1 (SEQ ID NO:21). This sequence includes an N-terminal methionine followed by a 33 amino acid secretion signal sequence. The N terminal methionine and the signal sequence (a total of 34 amino acids at the N terminus) are typically removed to form the mature IdeZ protein:

MKTIAYPNKPHSLSAGLLTAIAIFSLASSNITYADDYQRNATEAYAKEVPHQITSVWTKG
VTPLTPEQFRYNNEDVIHAPYLAHQGWYDITKAFDGKDNLLCGAATAGNMLHWWFDQ
NKTEIEAYLSKHPEKQKIIFNNQELFDLKAAIDTKDSQTNSQLFNYFRDKAFPNLSARQL
GVMPDLVLDMFINGYYLNVFKTQSTDVNRPYQDKDKRGGIFDAVFTRGDQTTLLTARH
DLKNKGLNDISTIIKQELTEGRALALSHTYANVSISHVINLWGADFNAEGNLEAIYVTDS
DANASIGMKKYFVGINAHGHVAISAKKIEGENIGAQVLGLFTLSSGKDIWQKLS

Sequence of IdeZ without the 34 amino acids from the N-terminus of full sequence (SEQ ID NO:22):

DDYQRNATEAYAKEVPHQITSVWTKGVTPLTPEQFRYNNEDVIHAPYLAHQGWYDITK
AFDGKDNLLCGAATAGNMLHWWFDQNKTEIEAYLSKHPEKQKIIFNNQELFDLKAAID
TKDSQTNSQLFNYFRDKAFPNLSARQLGVMPDLVLDMFINGYYLNVFKTQSTDVNRPY
QDKDKRGGIFDAVFTRGDQTTLLTARHDLKNKGLNDISTIIKQELTEGRALALSHTYAN
VSISHVINLWGADFNAEGNLEAIYVTDSDANASIGMKKYFVGINAHGHVAISAKKIEGE
NIGAQVLGLFTLSSGKDIWQKLS

The sequence of the IdeS/Z hybrid having an N terminal part based on IdeZ, without the N terminal methionine and the signal sequence (a total of 34 amino acids at the N terminus) (SEQ ID NO:23):

DDYQRNATEAYAKEVPHQITSVWTKGVTPLTPEQFRYNNEDVFHAPYVANQGWYDIT
KAFDGKDNLLCGAATAGNMLHWWFDQNKDQIKRYLEEHPEKQKINFNGDNMFDVKK
AIDTKNHQLDSKLFNYFKEKAFPGLSARRIGVFPDHVIDMFINGYRLSLTNHGPTPVKEG
SKDPRGGIFDAVFTRGNQSKLLTSRHDFKNKNLNDISTIIKQELTKGKALGLSHTYANVSI
NHVINLWGADFNAEGNLEAIYVTDSDSNASIGMKKYFVGVNAHGHVAISAKKIEGENIG
AQVLGLFTLSTGQDSWQKLS

SEQ ID NOs:24-43 correspond to peptides with modifications relative to IdeZ of SEQ ID NO:22.
SEQ ID NO:24: 
DDYQRNATEAYAKEVPHQITSVWTKGVTPLTPEQFRYNNEDVIHAPYLANQGWYDITK
AFDGKDNLLCGAATAGNMLHWWFDQNKTEIEAYLSKHPEKQKIIFRNQELFDLKEAIRT
KDSQTNSQLFEYFRDKAFPYLSARQLGVMPDLVLDMFINGYYLNVFKTQSTDVKRPYQ
DKDKRGGIFDAVFTRGNQTTLLTARHDLKNKGLNDISTIIKEELTKGRALALSHTYANVS
ISHVINLWGADFNAEGNLEAIYVTDSDANASIGMKKYFVGINKHGHVAISAKKIEGENIG
AQVLGLFTLSSGKDIWQKLN
SEQ ID NO:25: 
DDYQRNATEAYAKEVPHQITSVWTKGVTPLTPEQFRYNNEDVIHAPYLAHQGWYDITK
TFNGKDNLLCGAATAGNMLHWWFDQNKTEIEAYLSKHPEKQKIIFNNEELFDLKAAIDT
KDSQTNSQLFNYFKEKAFPNLSTRQLGVMPDLVLDMFINGYYLNVFKTQSTDVNRPYQ
DKDKRGGIFDAVFTRGNQTTLLTARHDFKEKGLKDISTIIKQELTEGRALALSHTYANVS
ISHVINLWGADFDAEGNLKAIYVTDSDANASIGMKKYFVGINAHGKVAISAKKIEGENIG
AQVLGLFTLSSGKDIWQQLS
SEQ ID NO:26: 
DSFSANQEIRYSEVTPYHVTSVWTKGVTPLTPEQFRYNNEDVIHAPYLAHQGWYDITKA
FDGKDNLLCGAATAGNMLHWWFDQNKTEIEAYLSKHPEKQKIIFNNQELFDLKAAIDT
KDSQTNSQLFNYFRDKAFPNLSARQLGVMPDLVLDMFINGYYLNVFKTQSTDVNRPYQ
 
DKDKRGGIFDAVFTRGDQTTLLTARHDLKNKGLNDISTIIKQELTEGRALALSHTYANVS
ISHVINLWGADFNAEGNLEAIYVTDSDANASIGMKKYFVGINAHGHVAISAKKIEGENIG
AQVLGLFTLSSGKDIWQKLS
SEQ ID NO:27: 
SVWTKGVTPLTPEQFRYNNEDVIHAPYLAHQGWYDITKAFDGKDNLLCGAATAGNML
HWWFDQNKTEIEAYLSKHPEKQKIIFNNQELFDLKAAIDTKDSQTNSQLFNYFRDKAFP
NLSARQLGVMPDLVLDMFINGYYLNVFKTQSTDVNRPYQDKDKRGGIFDAVFTRGDQT
TLLTARHDLKNKGLNDISTIIKQELTEGRALALSHTYANVSISHVINLWGADFNAEGNLE
AIYVTDSDANASIGMKKYFVGINAHGHVAISAKKIEGENIGAQVLGLFTLSSGKDIWQKL
S
SEQ ID NO:28: 
DDYQRNATEAYAKEVPHQITSVWTKGVTPLTPEQFTQGEDVIHAPYLAHQGWYDITKA
FDGKDNLLCGAATAGNMLHWWFDQNKTEIEAYLSKHPEKQKIIFNNQELFDLKAAIDT
KDSQTNSQLFNYFRDKAFPNLSARQLGVMPDLVLDMFINGYYLNVFKTQSTDVNRPYQ
DKDKRGGIFDAVFTRGDQTTLLTARHDLKNKGLNDISTIIKQELTEGRALALSHTYANVS
ISHVINLWGADFNAEGNLEAIYVTDSDANASIGMKKYFVGINAHGHVAISAKKIEGENIG
AQVLGLFTLSSGKDIWQKLS
SEQ ID NO:29: 
DDYQRNATEAYAKEVPHQITSVWTKGVTPPEQFTQGEDVIHAPYLAHQGWYDITKAFD
GKDNLLCGAATAGNMLHWWFDQNKTEIEAYLSKHPEKQKIIFNNQELFDLKAAIDTKD
SQTNSQLFNYFRDKAFPNLSARQLGVMPDLVLDMFINGYYLNVFKTQSTDVNRPYQDK
DKRGGIFDAVFTRGDQTTLLTARHDLKNKGLNDISTIIKQELTEGRALALSHTYANVSIS
HVINLWGADFNAEGNLEAIYVTDSDANASIGMKKYFVGINAHGHVAISAKKIEGENIGA
QVLGLFTLSSGKDIWQKLS
SEQ ID NO:30: 
DDYQRNATEAYAKEVPHQITSVWTKGVTPPEQFRYNNEDVIHAPYLAHQGWYDITKAF
DGKDNLLCGAATAGNMLHWWFDQNKTEIEAYLSKHPEKQKIIFNNQELFDLKAAIDTK
DSQTNSQLFNYFRDKAFPNLSARQLGVMPDLVLDMFINGYYLNVFKTQSTDVNRPYQD
KDKRGGIFDAVFTRGDQTTLLTARHDLKNKGLNDISTIIKQELTEGRALALSHTYANVSI
 
SHVINLWGADFNAEGNLEAIYVTDSDANASIGMKKYFVGINAHGHVAISAKKIEGENIG
AQVLGLFTLSSGKDIWQKLS
SEQ ID NO:31: 
DDYQRNATEAYAKEVPHQITSVWTKGVTPPEQFTQGEDVIHAPYLAHQGWYDITKAFD
GKDNLLCGAATAGNMLHWWFDQNKTEIEAYLSKHPEKQKIIINNQELFDLKAAIDTKDS
QTNSQLFNYFRDKAFPNLSARQLGVMPDLVLDMFINGYYLNVFKTQSTDVNRPYQDKD
KRGGIFDAVFTRGDQTTLLTARHDLKNKGLNDISTIIKQELTEGRALALSHTYANVSISH
VINLWGADFNAEGNLEAIYVTDSDANASIGMKKYFVGINAHGHVAISAKKIEGENIGAQ
VLGLFTLSSGKDIWQKLS
SEQ ID NO:32: 
DDYQRNATEAYAKEVPHQITSVWTKGVTPPEQFTQGEDVIHAPYLAHQGWYDITKAFD
GKDNLLCGAATAGNMLHWWFDQNKTEIEAYLSKHPEKQKIIFRNQELFDLKAAIDTKD
SQTNSQLFNYFRDKAFPNLSARQLGVMPDLVLDMFINGYYLNVFKTQSTDVNRPYQDK
DKRGGIFDAVFTRGDQTTLLTARHDLKNKGLNDISTIIKQELTEGRALALSHTYANVSIS
HVINLWGADFNAEGNLEAIYVTDSDANASIGMKKYFVGINAHGHVAISAKKIEGENIGA
QVLGLFTLSSGKDIWQKLS
SEQ ID NO:33: 
DDYQRNATEAYAKEVPHQITSVWTKGVTPPEQFTQGEDVIHAPYLAHQGWYDITKAFD
GKDNLLCGAATAGNMLHWWFDQNKTEIEAYLSKHPEKQKIIIRNQELFDLKAAIDTKDS
QTNSQLFNYFRDKAFPNLSARQLGVMPDLVLDMFINGYYLNVFKTQSTDVNRPYQDKD
KRGGIFDAVFTRGDQTTLLTARHDLKNKGLNDISTIIKQELTEGRALALSHTYANVSISH
VINLWGADFNAEGNLEAIYVTDSDANASIGMKKYFVGINAHGHVAISAKKIEGENIGAQ
VLGLFTLSSGKDIWQKLS
SEQ ID NO:34: 
DDYQRNATEAYAKEVPHQITSVWTKGVTPPEQFTQGEDVIHAPYLANQGWYDITKAFD
GKDNLLCGAATAGNMLHWWFDQNKTEIEAYLSKHPEKQKIIFRNQELFDLKEAIRTKDS
QTNSQLFEYFRDKAFPYLSARQLGVMPDLVLDMFINGYYLNVFKTQSTDVKRPYQDKD
KRGGIFDAVFTRGNQTTLLTARHDLKNKGLNDISTIIKEELTKGRALALSHTYANVSISH
 
VINLWGADFNAEGNLEAIYVTDSDANASIGMKKYFVGINKHGHVAISAKKIEGENIGAQ
VLGLFTLSSGKDIWQKLN
SEQ ID NO:35: 
SVWTKGVTPPEQFTQGEDVIHAPYLAHQGWYDITKAFDGKDNLLCGAATAGNMLHW
WFDQNKTEIEAYLSKHPEKQKIIFRNQELFDLKAAIDTKDSQTNSQLFNYFRDKAFPNLS
ARQLGVMPDLVLDMFINGYYLNVFKTQSTDVNRPYQDKDKRGGIFDAVFTRGNQTTLL
TARHDLKNKGLNDISTIIKQELTEGRALALSHTYANVSISHVINLWGADFNAEGNLEAIY
VTDSDANASIGMKKYFVGINAHGHVAISAKKIEGENIGAQVLGLFTLSSGKDIWQKLS
SEQ ID NO:36: 
DDYQRNATEAYAKEVPHQITSVWTKGVTPPEQFTQGEDVIHAPYLAHQGWYDITKAFD
GADNLLCGAATAGNMLHWWFDQNKTEIEAYLSKHPEKQKIIFRNQELFDLKAAIDTKD
SQTNSQLFNYFRDKAFPNLSARQLGVMPDLVLDMFINGYYLNVFKTQSTDVNRPYQDK
DKRGGIFDAVFTRGNQTTLLTARHDLKNKGLNDISTIIKQELTEGRALALSHTYANVSIS
HVINLWGADFNAEGNLEAIYVTDSDANASIGMKKYFVGINAHGHVAISAKKIEGENIGA
QVLGLFTLSSGKDIWQKLS
SEQ ID NO:37: 
SVWTKGVTPPEQFTQGEDVIHAPYLAHQGWYDITKAFDGADNLLCGAATAGNMLHW
WFDQNKTEIEAYLSKHPEKQKIIFRNQELFDLKAAIDTKDSQTNSQLFNYFRDKAFPNLS
ARQLGVMPDLVLDMFINGYYLNVFKTQSTDVNRPYQDKDKRGGIFDAVFTRGNQTTLL
TARHDLKNKGLNDISTIIKQELTEGRALALSHTYANVSISHVINLWGADFNAEGNLEAIY
VTDSDANASIGMKKYFVGINAHGHVAISAKKIEGENIGAQVLGLFTLSSGKDIWQKLS
SEQ ID NO:38: 
DDYQRNATEAYAKEVPHQITSVWTKGVTPPEQFTQGEDVIHAPYLAHQGWYDITKAFD
GKDNLLCGAATAGNMLHWWFDQNKTEIEAYLSKHPEKQKIIFRNQELFDLKAAIDTKD
SQTNSQLFNYFRDKAFPNLSARQLGVMPDLVLDMFINGYYLNVFKTQSTDVNRPYQDK
DKRGGIFDAVFTRGNQTTLLTARHDLKNKGLNDISTIIKQELTEGRALALSHTYANVSIS
HVINLWGADFNAEGNLEAIYVTDSDANASIGMKKYFVGINAHGHVAISAKKIEGENIGA
QVLGLFTLSSGKDIWQKLS
SEQ ID NO:39: 
DDYQRNATEAYAKEVPHQITSVWTKGVTPLTPEQFTQGEDVFHAPYVANQGWYDITKA
FDGKDNLLCGAATAGNMLHWWFDQNKDQIKRYLEEHPEKQKINFNGENMFDVKKAID
TKNHQLDSKLFNYFKEKAFPYLSAKHLGVFPDHVIDMFINGYRLSLTNHGPTPVKEGSK
DPRGGIFDAVFTRGNQSKLLTSRHDFKNKNLNDISTIIKQELTKGKALGLSHTYANVRIN
HVINLWGADFNAEGNLEAIYVTDSDSNASIGMKKYFVGVNAHGHVAISAKKIEGENIGA
QVLGLFTLSTGQDSWQKLS
SEQ ID NO:40: 
DDYQRNATEAYAKEVPHQITSVWTKGVTPLTPEQFTQGEDVFHAPYVANQGWYDITKA
FDGKDNLLCGAATAGNMLHWWFDQNKDQIKRYLEEHPEKQKINFRGENMFDVKEAIR
TKNHQLDSKLFEYFKEKAFPYLSAKHLGVFPDHVIDMFINGYRLSLTNHGPTPVKKGSK
DPRGGIFDAVFTRGNQSKLLTSRHDFKNKNLNDISTIIKSELTNGKALGLSHTYANVRIN
HVINLWGADFNAEGNLEAIYVTDSDSNASIGMKKYFVGVNKHGHVAISAKKIEGENIGA
QVLGLFTLSTGQDSWQKLN
SEQ ID NO:41: 
DDYQRNATEAYAKEVPHQITSVWTKGVTPLTPEQFTQGEDVFHAPYVANQGWYDITKT
FNGKDDLLCGAATAGNMLHWWFDQNKDQIKRYLEEHPEKQKINFNGEQMFDVKEAID
TKNHQLDSKLFEYFKEKAFPYLSTKHLGVFPDHVIDMFINGYRLSLTNHGPTPVKEGSK
DPRGGIFDAVFTRGNQSKLLTSRHDFKEKNLKEISDLIKQELTEGKALGLSHTYANVRIN
HVINLWGADFDAEGNLKAIYVTDSDSNASIGMKKYFVGVNAAGKVAISAKKIEGENIGA
QVLGLFTLSTGQDSWNQTS
SEQ ID NO:42: 
DDYQRNATEAYAKEVPHQITSVWTKGVTPLTPEQFTQGEDVFHAPYVANQGWYDITKT
FNGKDDLLCGAATAGNMLHWWFDQNKDQIKRYLEEHPEKQKINFRGEQMFDVKEAIR
TKNHQLDSKLFEYFKEKAFPYLSTKHLGVFPDHVIDMFINGYRLSLTNHGPTPVKKGSK
DPRGGIFDAVFTRGNQSKLLTSRHDFKEKNLKEISDLIKEELTKGKALGLSHTYANVRIN
HVINLWGADFDAEGNLKAIYVTDSDSNASIGMKKYFVGVNKAGKVAISAKKIEGENIGA
QVLGLFTLSTGQDSWNQTN
SEQ ID NO:43:

DDYQRNATEAYAKEVPHQITSVWTKGVTPPEQFTQGEDVIHAPYVANQGWYDITKAFD
GKDNLLCGAATAGNMLHWWFDQNKDQIKRYLEEHPEKQKINFRGEQMFDVKKAIDTK
NHQLDSKLFNYFKEKAFPGLSARRIGVFPDHVIDMFINGYRLSLTNHGPTPVKEGSKDPR
GGIFDAVFTRGNQSKLLTSRHDFKNKNLNDISTIIKQELTKGKALGLSHTYANVSINHVIN
LWGADFNAEGNLEAIYVTDSDSNASIGMKKYFVGVNAHGHVAISAKKIEGENIGAQVL
GLFTLSTGQDSWQKLS

Protein sequence for endoglycosidase EndoS49 from *Streptococcus pyogenes* is presented in U.S. Patent 9,493,752 (SEQ ID NO:44):

MDKHLLVKRTLGCVCAATLMGAALATHHDSLNTVKAEEKTVQTGKTDQQVGAKLVQ
EIREGKRGPLYAGYFRTWHDRASTGIDGKQQHPENTMAEVPKEVDILFVFHDHTASDSP
FWSELKDSYVHKLHQQGTALVQTIGVNELNGRTGLSKDYPDTPEGNKALAAAIVKAFV
TDRGVDGLDIDIEHEFTNKRTPEEDARALNVFKEIAQLIGKNGSDKSKLLIMDTTLSVEN
NPIFKGIAEDLDYLLRQYYGSQGGEAEVDTINSDWNQYQNYIDASQFMIGFSFFEESASK
GNLWFDVNEYDPNNPEKGKDIEGTRAKKYAEWQPSTGGLKAGIFSYAIDRDGVAHVPS
TYKNRTSTNLQRHEVDNISHTDYTVSRKLKTLMTEDKRYDVIDQKDIPDPALREQIIQQV
GQYKGDLERYNKTLVLTGDKIQNLKGLEKLSKLQKLELRQLSNVKEITPELLPESMKKD
AELVMVGMTGLEKLNLSGLNRQTLDGIDVNSITHLTSFDISHNSLDLSEKSEDRKLLMTL
MEQVSNHQKITVKNTAFENQKPKGYYPQTYDTKEGHYDVDNAEHDILTDFVFGTVTKR
NTFIGDEEAFAIYKEGAVDGRQYVSKDYTYEAFRKDYKGYKVHLTASNLGETVTSKVT
ATTDETYLVDVSDGEKVVHHMKLNIGSGAIMMENLAKGAKVIGTSGDFEQAKKIFDGE
KSDRFFTWGQTNWIAFDLGEINLAKEWRLFNAETNTEIKTDSSLNVAKGRLQILKDTTID
LEKMDIKNRKEYLSNDENWTDVAQMDDAKAIFNSKLSNVLSRYWRFCVDGGASSYYP
QYTELQILGQRLSNDVANTLKD

Protein sequence of mature Endoglycosidase S (EndoS) from *S. pyogenes* is presented in U.S. Patents 8,889,128 and 9,707,279. (SEQ ID NO:45):

EEKTVQVQKGLPSIDSLHYLSENSKKEFKEELSKAGQESQKVKEILAKAQQADKQAQEL
AKMKIPEKIPMKPLHGPLYGGYFRTWHDKTSDPTEKDKVNSMGELPKEVDLAFIFHDW
TKDYSLFWKELATKHVPKLNKQGTRVIRTIPWRFLAGGDNSGIAEDTSKYPNTPEGNKA
LAKAIVDEYVYKYNLDGLDVDVEHDSIPKVDKKEDTAGVERSIQVFEEIGKLIGPKGVD

KSRLFIMDSTYMADKNPLIERGAPYINLLLVQVYGSQGEKGGWEPVSNRPEKTMEERW
QGYSKYIRPEQYMIGFSFYEENAQEGNLWYDINSRKDEDKANGINTDITGTRAERYARW
QPKTGGVKGGIFSYAIDRDGVAHQPKKYAKQKEFKDATDNIFHSDYSVSKALKTVMLK
DKSYDLIDEKDFPDKALREAVMAQVGTRKGDLERFNGTLRLDNPAIQSLEGLNKFKKL
AQLDLIGLSRITKLDRSVLPANMKPGKDTLETVLETYKKDNKEEPATIPPVSLKVSGLTG
LKELDLSGFDRETLAGLDAATLTSLEKVDISGNKLDLAPGTENRQIFDTMLSTISNHVGS
NEQTVKFDKQKPTGHYPDTYGKTSLRLPVANEKVDLQSQLLFGTVTNQGTLINSEADY
KAYQNHKIAGRSFVDSNYHYNNFKVSYENYTVKVTDSTLGTTTDKTLATDKEETYKVD
FFSPADKTKAVHTAKVIVGDEKTMMVNLAEGATVIGGSADPVNARKVFDGQLGSETDN
ISLGWDSKQSIIFKLKEDGLIKHWRFFNDSARNPETTNKPIQEASLQIFNIKDYNLDNLLE
NPNKFDDEKYWITVDTYSAQGERATAFSNTLNNITSKYWRVVFDTKGDRYSSPVVPEL
QILGYPLPNADTIMKTVTTAKELSQQKDKFSQKMLDELKIKEMALETSLNSKIFDVTAIN
ANAGVLKDCIEKRQLLKK

Full sequence including secretion signal of endoglycosidase EndoS from *Streptococcus pyogenes* (GenBank Accession No. AAK00850.1). (SEQ ID NO:46):

MDKHLLVKRTLGCVCAATLMGAALATHHDSLNTVKAEEKTVQVQKGLPSIDSLHYLSE
NSKKEFKEELSKAGQESQKVKEILAKAQQADKQAQELAKMKIPEKIPMKPLHGPLYGG
YFRTWHDKTSDPTEKDKVNSMGELPKEVDLAFIFHDWTKDYSLFWKELATKHVPKLN
KQGTRVIRTIPWRFLAGGDNSGIAEDTSKYPNTPEGNKALAKAIVDEYVYKYNLDGLDV
DVEHDSIPKVDKKEDTAGVERSIQVFEEIGKLIGPKGVDKSRLFIMDSTYMADKNPLIER
GAPYINLLLVQVYGSQGEKGGWEPVSNRPEKTMEERWQGYSKYIRPEQYMIGFSFYEE
NAQEGNLWYDINSRKDEDKANGINTDITGTRAERYARWQPKTGGVKGGIFSYAIDRDG
VAHQPKKYAKQKEFKDATDNIFHSDYSVSKALKTVMLKDKSYDLIDEKDFPDKALREA
VMAQVGTRKGDLERFNGTLRLDNPAIQSLEGLNKFKKLAQLDLIGLSRITKLDRSVLPA
NMKPGKDTLETVLETYKKDNKEEPATIPPVSLKVSGLTGLKELDLSGFDRETLAGLDAA
TLTSLEKVDISGNKLDLAPGTENRQIFDTMLSTISNHVGSNEQTVKFDKQKPTGHYPDTY
GKTSLRLPVANEKVDLQSQLLFGTVTNQGTLINSEADYKAYQNHKIAGRSFVDSNYHY
NNFKVSYENYTVKVTDSTLGTTTDKTLATDKEETYKVDFFSPADKTKAVHTAKVIVGD
EKTMMVNLAEGATVIGGSADPVNARKVFDGQLGSETDNISLGWDSKQSIIFKLKEDGLI

KHWRFFNDSARNPETTNKPIQEASLQIFNIKDYNLDNLLENPNKFDDEKYWITVDTYSA
QGERATAFSNTLNNITSKYWRVVFDTKGDRYSSPVVPELQILGYPLPNADTIMKTVTTA
KELSQQKDKFSQKMLDELKIKEMALETSLNSKIFDVTAINANAGVLKDCIEKRQLLKK

Protein sequence of EndoS isolated from *S*. *pyogenes* AP1, including signal sequence, is described in U.S. Patents 8,889,128 and 9,707,279. (SEQ ID NO:47):

MDKHLLVKRTLGCVCAATLMGAALATHHDSLNTVKAEEKTVQVQKGLPSIDSLHYLSE
NSKKEFKEELSKAGQESQKVKEILAKAQQADKQAQELAKMKIPEKIPMKPLHGPLYGG
YFRTWHDKTSDPTEKDKVNSMGELPKEVDLAFIFHDWTKDYSLFWKELATKHVPKLN
KQGTRVIRTIPWRFLAGGDNSGIAEDTSKYPNTPEGNKALAKAIVDEYVYKYNLDGLDV
DVEHDSIPKVDKKEDTAGVERSIQVFEEIGKLIGPKGVDKSRLFIMDSTYMADKNPLIER
GAPYINLLLVQVYGSQGEKGGWEPVSNRPEKTMEERWQGYSKYIRPEQYMIGFSFYEE
NAQEGNLWYDINSRKDEDKANGINTDITGTRAERYARWQPKTGGVKGGIFSYAIDRDG
VAHQPKKYAKQKEFKDATDNIFHSDYSVSKALKTVMLKDKSYDLIDEKDFPDKALREA
VMAQVGTRKGDLERFNGTLRLDNPAIQSLEGLNKFKKLAQLDLIGLSRITKLDRSVLPA
NMKPGKDTLETVLETYKKDNKEEPATIPPVSLKVSGLTGLKELDLSGFDRETLAGLDAA
TLTSLEKVDISGNKLDLAPGTENRQIFDTMLSTISNHVGSNEQTVKFDKQKPTGHYPDTY
GKTSLRLPVANEKVDLQSQLLFGTVTNQGTLINSEADYKAYQNHKIAGRSFVDSNYHY
NNFKVSYENYTVKVTDSTLGTTTDKTLATDKEETYKVDFFSPADKTKAVHTAKVIVGD
EKTMMVNLAEGATVIGGSADPVNARKVFDGQLGSETDNISLGWDSKQSIIFKLKEDGLI
KHWRFFNDSARNPETTNKPIQEASLQIFNIKDYNLDNLLENPNKFDDEKYWITVDTYSA
QGERATAFSNTLNNITSKYWRVVFDTKGDRYSSPVVPELQILGYPLPNADTIMKTVTTA
KELSQQKDKFSQKMLDELKIKEMALETSLNSKIFDVTAINANAGVLKDCIEKRQLLKK

Mature sequence of IdeS with added N terminal methionine (SEQ ID NO:48):

MDSFSANQEIRYSEVTPYHVTSVWTKGVTPPANFTQGEDVFHAPYVANQGWYDITKTF
NGKDDLLCGAATAGNMLHWWFDQNKDQIKRYLEEHPEKQKINFNGEQMFDVKEAIDT
KNHQLDSKLFEYFKEKAFPYLSTKHLGVFPDHVIDMFINGYRLSLTNHGPTPVKEGSKD
PRGGIFDAVFTRGDQSKLLTSRHDFKEKNLKEISDLIKKELTEGKALGLSHTYANVRINH
VINLWGADFDSNGNLKAIYVTDSDSNASIGMKKYFVGVNSAGKVAISAKEIKEDNIGAQ
VLGLFTLSTGQDSWNQTN

SEQ ID NOs: 49-51 are amino acid sequences of signal sequences that can be fused at the N-terminus of any of the proteases or glycosidases disclosed herein.
SEQ ID NO:49: MRKRCYSTSAAVLAAVTLFVLSVDRGVIA
SEQ ID NO:50 MRKRCYSTSAAVLAAVTLFALSVDRGVIA
SEQ ID NO: 51 MRKRCYSTSAVVLAAVTLFALSVDRGVIA

A polynucleotide sequence that encodes SEQ ID NO:4, mature IdeS (SEQ ID NO:52):

gat agt ttt tct gct aat caa gag att aga tat tcg gaa gta aca cct tat cac gtt act tcc gtt tgg acc aaa gga gtt
act cct cca gca aac ttc act caa ggt gaa gat gtt ttt cac gct cct tat gtt gct aac caa gga tgg tat gat att acc
aaa aca ttc aat gga aaa gac gat ctt ctt tgc ggg gct gcc aca gca ggg aat atg ctt cac tgg tgg ttc gat caa
aac aaa gac caa att aaa cgt tat ttg gaa gag cat cca gaa aag caa aaa ata aac ttc aat ggc gaa cag atg ttt
gac gta aaa gaa gct atc gac act aaa aac cac cag cta gat agt aaa tta ttt gaa tat ttt aaa gaa aaa gct ttc
cct tat cta tct act aaa cac cta gga gtt ttc cct gat cat gta att gat atg ttc att aac ggc tac cgc ctt agt cta
act aac cac ggt cca acg cca gta aaa gaa ggt agt aaa gat ccc cga ggt ggt att ttt gac gcc gta ttt aca aga
ggt gat caa agt aag cta ttg aca agt cgt cat gat ttt aaa gaa aaa aat ctc aaa gaa atc agt gat ctc att aag
aaa gag tta acc gaa ggc aag gct cta ggc cta tca cac acc tac gct aac gta cgc atc aac cat gtt ata aac ctg
tgg gga gct gac ttt gat tct aac ggg aac ctt aaa gct att tat gta aca gac tct gat agt aat gca tct att ggt atg
aag aaa tac ttt gtt ggt gtt aat tcc gct gga aaa gta gct att tct gct aaa gaa ata aaa gaa gat aat att ggt gct
caa gta cta ggg tta ttt aca ctt tca aca ggg caa gat agt tgg aat cag acc aat taa

Unless otherwise defined, all technical and scientific terms used herein have the same meaning as commonly understood by one of ordinary skill in the art to which the instant invention belongs. Although methods and materials similar or equivalent to those described herein can be used in the practice or testing of the instant invention, suitable methods and materials are described herein.

All patents, patent applications, publications and other references, GenBank citations and ATCC citations cited herein are incorporated by reference in their entirety. In case of conflict, the specification, including definitions, will control.

All of the features disclosed herein may be combined in any combination. Each feature disclosed in the specification may be replaced by an alternative feature serving a same, equivalent, or similar purpose. Thus, unless expressly stated otherwise, disclosed features are an example of a genus of equivalent or similar features.

As used herein, the singular forms "a", "and," and "the" include plural referents unless the context clearly indicates otherwise. Thus, for example, reference to "a nucleic acid" includes a plurality of such nucleic acids, reference to "a vector" includes a plurality of such vectors, and reference to "a virus" or "particle" includes a plurality of such viruses/particles.

The term "about" as used herein refers to a value within 10% of the underlying parameter (*i.e*., plus or minus 10%). For example, "about 1:10" means 1.1:10.1 or 0.9:9.9, and about 5 hours means 4.5 hours or 5.5 hours, etc. The term "about" at the beginning of a string of values modifies each of the values by 10%.

All numerical values or numerical ranges include integers within such ranges and fractions of the values or the integers within ranges unless the context clearly indicates otherwise. Thus, to illustrate, reference to reduction of 95% or more includes 95%, 96%, 97%, 98%, 99%, 100% etc., as well as 95.1%, 95.2%, 95.3%, 95.4%, 95.5%, etc., 96.1%, 96.2%, 96.3%, 96.4%, 96.5%, etc., and so forth. Thus, to also illustrate, reference to a numerical range, such as "1-4" includes 2, 3, as well as 1.1, 1.2, 1.3, 1.4, etc., and so forth. For example, "1 to 4 weeks" includes 7, 8, 9, 10, 11, 12, 13, 14, 15, 16, 17, 18, 19, 20, 21, 22, 23, 24, 25, 26, 27, or 28 days.

Further, reference to a numerical range, such as "0.01 to 10" includes 0.011, 0.012, 0.013, etc., as well as 9.5, 9.6, 9.7, 9.8, 9.9, etc., and so forth. For example, a dosage of about "0.01 mg/kg to about 10 mg/kg" body weight of a subject includes 0.011 mg/kg, 0.012 mg/kg, 0.013 mg/kg, 0.014 mg/kg, 0.015 mg/kg etc., as well as 9.5 mg/kg, 9.6 mg/kg, 9.7 mg/kg, 9.8 mg/kg, 9.9 mg/kg etc., and so forth.

Reference to an integer with more (greater) or less than includes any number greater or less than the reference number, respectively. Thus, for example, reference to more than 2 includes 3, 4, 5, 6, 7, 8, 9, 10, 11, 12, 13, 14, 15, etc., and so forth. For example, administration of a recombinant viral vector, protease and/or glycosidase "two or more" times includes 3, 4, 5, 6, 7, 8, 9, 10, 11, 12, 13, 14, 15, or more times.

Further, reference to a numerical range, such as "1 to 90" includes 1.1, 1.2, 1.3, 1.4, 1.5, etc., as well as 81, 82, 83, 84, 85, etc., and so forth. For example, "between about 1 minute to about 90 days" includes 1.1 minutes, 1.2 minutes, 1.3 minutes, 1.4 minutes, 1.5 minutes, etc., as well as one day, 2 days, 3 days, 4 days, 5 days .... 81 days, 82 days, 83 days, 84 days, 85 days, etc., and so forth.

The instant invention is generally disclosed herein using affirmative language to describe the numerous embodiments of the instant invention. The instant invention also specifically includes embodiments in which particular subject matter is excluded, in full or in part, such as substances or materials, method steps and conditions, protocols, or procedures. For example, in certain embodiments of the instant invention, materials and/or method steps are excluded. Thus, even though the instant invention is generally not expressed herein in terms of what the instant invention does not include, aspects that are not expressly excluded in the instant invention are nevertheless disclosed herein.

## Claims

1. An immunoglobulin G-degrading enzyme polypeptide for use in the prevention or treatment of an humoral immune response caused by administration of a recombinant adeno-associated virus (AAV) vector, wherein the humoral immune response is against the recombinant AAV vector and/or a heterologous polynucleotide or a protein or peptide encoded by a heterologous polynucleotide encapsidated by the recombinant AAV vector.

2. The immunoglobulin G-degrading enzyme polypeptide for use according to claim 1, wherein the recombinant AAV vector is a gene therapy vector.

3. An immunoglobulin G-degrading enzyme polypeptide for use in combination with a recombinant gene therapy adeno-associated virus (AAV) vector for the treatment of a disease treated by said recombinant gene therapy AAV vector in a patient in need thereof.

4. The immunoglobulin G-degrading enzyme polypeptide for use according to any one of claims 1-3, wherein said recombinant AAV vector comprises capsid proteins selected from the group consisting of AAV1, AAV2, an AAV2 variant, AAV3, an AAV3 variant, AAV3B, an AAV3B variant, AAV4, AAV5, AAV6, an AAV6 variant, AAV7, AAV8, AAV9, AAV10, AAVcy10, AAVrh10, AAVrh74, AAVdj, AAV-Anc80, SEQ ID NO:1, SEQ ID NO:2 or AAV2i8.

5. The immunoglobulin G-degrading enzyme polypeptide for use according to any one of claims 1-4, wherein the immunoglobulin G-degrading enzyme polypeptide comprises the sequence of any one of SEQ ID NOs:3-18, 23, or 48.

6. The immunoglobulin G-degrading enzyme polypeptide for use according to claim 5, wherein said immunoglobulin G-degrading enzyme polypeptide further comprises a non-native signal sequence of any one of SEQ ID NOs:49-51.

7. The immunoglobulin G-degrading enzyme polypeptide for use according to any one of claims 2-5, wherein the disease treated by the recombinant gene therapy AAV vector is selected from the group consisting of proliferative diseases (cancers, tumors, dysplasias, etc.), Crigler-Najjar and metabolic diseases like metabolic diseases of the liver, Friedreich ataxia, infectious diseases, addiction (*e.g*., to tobacco, alcohol, or drugs), epilepsy, Canavan's disease, adrenoleukodystrophy, viral diseases (induced, *e.g*., by hepatitis B or C viruses, HIV, herpes, retroviruses, etc.), genetic diseases (cystic fibrosis, dystroglycanopathies, myopathies such as Duchenne muscular myopathy or dystrophy, myotubular myopathy, hemophilia A, hemophilia B, hemophilia A with inhibitors, hemophilia B with inhibitors, sickle-cell anemia, sickle cell disease, Fanconi's anemia, diabetes, amyotrophic lateral sclerosis (ALS), myotubularin myopathy, motor neuron diseases such as spinal muscular atrophy (SMA), spinobulbar muscular atrophy, or Charcot-Marie-Tooth disease, arthritis, severe combined immunodeficiencies (such as RS-SCID, ADA-SCID or X-SCID), Wiskott-Aldrich syndrome, X-linked thrombocytopenia, X-linked congenital neutropenia, chronic granulomatous disease, etc.), clotting factor deficiencies, cardiovascular disease (restenosis, ischemia, dyslipidemia, homozygous familial hypercholesterolemia, etc.), eye diseases such as retinitis pigmentosa, Leber congenital amaurosis, Leber hereditary optic neuropathy, and Stargardt disease; hereditary angioedema (HAE); lysosomal storage diseases such as San Filippo syndrome; hyperbilirubinemia such as CN type I or II or Gilbert's syndrome; Fabry disease, glycogen storage disease such as GSDI, GSDII (Pompe disease), GSDIII, GSDIV, GSDV, GSDVI, GSDVII, GSDVIII and lethal congenital glycogen storage disease of the heart.

8. The immunoglobulin G-degrading enzyme polypeptide for use according to any one of claims 2-6, wherein the recombinant gene therapy AAV vector comprises a therapeutic polynucleotide appropriate for treating the disease of claim 7.

9. The immunoglobulin G-degrading enzyme polypeptide for use according to any one of claims 3-8, wherein the recombinant gene therapy AAV vector and the immunoglobulin G-degrading enzyme polypeptide are administered simultaneously, separately, or sequentially.

10. The immunoglobulin G-degrading enzyme polypeptide for use according to any one of claims 3-9, wherein the immunoglobulin G-degrading enzyme polypeptide is administered before or simultaneously with said recombinant gene therapy AAV vector.

11. A nucleic acid sequence encoding an immunoglobulin G-degrading enzyme polypeptide for use in combination with a recombinant gene therapy adeno-associated virus (AAV) vector for the treatment of a disease treated by said recombinant gene therapy AAV vector in a patient in need thereof.

12. An expression vector comprising a nucleic acid sequence encoding an immunoglobulin G-degrading enzyme polypeptide for use in combination with a recombinant gene therapy adeno-associated virus (AAV) vector for the treatment of a disease treated by said recombinant gene therapy AAV vector in a patient in need thereof.

13. A package or kit comprising (a) a recombinant gene therapy adeno-associated virus (AAV) vector comprising a heterologous polynucleotide that encodes a heterologous protein, peptide or polynucleotide, (b) an immunoglobulin G-degrading enzyme polypeptide, and (c) a label with instructions for performing gene therapy, wherein (a) and (b) are in separate or the same container.

14. A package or kit according to claim 13, for use in the treatment of a disease by gene therapy.

15. The nucleic acid sequence for use according to claim 11, the expression vector for use according to claim 12, the package or kit according to claim 13, or the package or kit for use according to claim 14, wherein the immunoglobulin G-degrading enzyme polypeptide comprises the sequence of any of SEQ ID NOs:3-18, 23, or 48.
